(19) Europäisches Patentamt
European Patent Office
Office européen des brevets

(11) Veröffentlichungsnummer: **0 344 557 B1**

(12) # EUROPÄISCHE PATENTSCHRIFT

(45) Veröffentlichungstag der Patentschrift: **10.08.94**

(21) Anmeldenummer: **89109129.0**

(22) Anmeldetag: **20.05.89**

(51) Int. Cl.5: **C07D 239/26**, C09K 19/34, C09K 19/30, C09K 19/32, C07D 213/30, C07D 241/12, C07D 285/12, C07D 319/06, C07C 43/21, C07C 43/225, C07C 43/184

(54) **Flüssigkristallkomponenten mit einer Trimethylenoxygruppe.**

(30) Priorität: **01.06.88 CH 2093/88**
**10.03.89 CH 896/89**

(43) Veröffentlichungstag der Anmeldung:
**06.12.89 Patentblatt 89/49**

(45) Bekanntmachung des Hinweises auf die Patenterteilung:
**10.08.94 Patentblatt 94/32**

(84) Benannte Vertragsstaaten:
**CH DE FR GB IT LI NL**

(56) Entgegenhaltungen:
**EP-A- 0 122 389**

**PATENT ABSTRACTS OF JAPAN, Band 11, Nr. 115 (C-415)[2562], 10. April 1987, Seite 71 C 415; & JP-A-61 257 935 (ASAHI GLASS CO. LTD) 15-11-1986**

**KONTAKTE, Band 2, 1988, Seite 15**

**MOL. CRYST. LIQ. CRYST., Band 204, 1991, Seiten 27-35**

**LIQUID CRYSTALS, Band 10, Nr. 2, 1991, Seiten 243-260**

(73) Patentinhaber: **F. HOFFMANN-LA ROCHE AG**
**Postfach 3255**
**CH-4002 Basel (CH)**

(72) Erfinder: **Kelly, Stephen, Dr.**
**Salinenstrasse 3A**
**CH-4313 Möhlin (CH)**

(74) Vertreter: **Cottong, Norbert A. et al**
**Grenzacherstrasse 124**
**Postfach 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Verbindungen mit einer Trimethylenoxygruppe, flüssigkristalline Gemische, die solche Verbindungen enthalten sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen ("twisted nematic") und STN-Zellen ("super-twisted nematic") mit verdrillt nematischer Struktur, SBE-Zellen ("super-birefringence effect"). Phase-Change-Zellen mit einem cholesterisch-nematischen Phasenübergang und OMI-Zellen ("optical mode interference"). Die gebräuchlichsten Anzeigevorrichtungen beruhen auf dem Schadt-Helfrich-Effekt und besitzen eine verdrillt nematische Struktur.

In Appl. Phys. Lett. 36, 899 (1980) und in Recent Developments in Condensed Matter Physics 4, 309 (1981) werden ferner elektro-optische Vorrichtungen auf der Basis von chiral getilteten smektischen Flüssigkristallen vorgeschlagen. Hierbei werden die ferroelektrischen Eigenschaften dieser Materialien ausgenutzt. Als getiltete smektische Phasen eignen sich beispielsweise smektisch C, F, G, H, I und K Phasen. Bevorzugt sind im allgemeinen smektisch C Phasen, welche besonders grosse Ansprechgeschwindigkeiten ermöglichen. Die chiral getilteten Phasen werden üblicherweise mit $S_C^*$, $S_F^*$ etc. bezeichnet, wobei der Stern die Chiralität angibt.

Die Flüssigkristallmaterialien müssen eine gute chemische und thermische Stabilität und eine hohe Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Weiterhin sollen sie bei üblichen Betriebstemperaturen eine geeignete Mesophase besitzen, beispielsweise eine nematische, cholesterische oder chiral getiltete smektische Phase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. bei TFT-Anwendungen (Dünnfilmtransistor) in Fernsehgeräten. Anderseits sollten chiral getiltete smektische Flüssigkristalle eine ausreichend hohe spontane Polarisation besitzen.

Flüssigkristalle werden zwecks Optimierung der Eigenschaften in der Regel als Mischungen mehrerer Komponenten verwendet. Es ist daher wichtig, dass die Komponenten untereinander gut mischbar sind. Cholesterische Gemische können vorzugsweise aus einem oder mehreren optisch aktiven Dotierstoffen und einem nematischen Flüssigkristallmaterial bestehen, und ferroelektrische Flüssigkristalle können vorzugsweise aus einem oder mehreren optisch aktiven Dotierstoffen und einem Flüssigkristallmaterial mit getilteter smektischer Phase bestehen.

Gegenstand der vorliegenden Erfindung sind die Verbindungen der allgemeinen Formel

$$R^1\text{-}A^1\text{-}Z^1\text{-}A^2\text{-}(Z^2\text{-}A^3)_n\text{-}R^2 \qquad I$$

worin $Z^1$ die Gruppe $-CH_2CH_2CH_2O-$ oder $-OCH_2CH_2CH_2-$ bezeichnet; n für die Zahl 0 oder 1 steht; $R^1$ eine Gruppe $R^3$ oder $R^3\text{-}A^4\text{-}Z^3\text{-}$ darstellt; $R^2$ eine Gruppe $R^4$ oder $R^4\text{-}A^5\text{-}Z^4\text{-}$ darstellt; $Z^2$, $Z^3$ und $Z^4$ unabhängig voneinander eine einfache Kovalenzbindung, $-CH_2CH_2-$, $-CH_2O-$, $-OCH_2-$, -COO-, -OOC-, $-CH_2CH_2CH_2O-$ oder $-OCH_2CH_2CH_2-$ bedeuten; $A^1$, $A^2$, $A^3$, $A^4$ und $A^5$ unabhängig voneinander unsubstituiertes oder mit Methyl, Halogen und/oder Cyano substituiertes 1,4-Phenylen, in welchem gegebenenfalls 1-4 CH-Gruppen durch Stickstoff ersetzt sind, unsubstituiertes oder mit Methyl und/oder Cyano substituiertes trans-1,4-Cyclohexylen, in welchem gegebenenfalls 2 $CH_2$-Gruppen durch Sauerstoff und/oder Schwefel ersetzt sind, Bicyclo[2.2.2]octan-1,4-diyl, 1,3,4-Thiadiazol-2,5-diyl, Naphthalin-2,6-diyl, Tetralin-2,6-diyl oder Decalin-2,6-diyl bezeichnen; $R^3$ und $R^4$ unabhängig voneinander eine unsubstituierte oder mit Halogen und/oder Cyano substituierte Alkyl- oder Alkenylgruppe mit 1 bzw. 2 bis 18 Kohlenstoffatomen darstellen, in welcher gegebenenfalls 1 oder 2 nicht benachbarte $CH_2$-Gruppen durch -O-, -COO-und/oder -OOC- ersetzt sind, oder einer der Reste $R^3$ und $R^4$ auch Wasserstoff, Halogen, Cyano oder -NCS darstellt.

Die Verbindungen der Formel I besitzen trotz der hohen Flexibilität der Trimethylenoxygruppe $Z^1$ erstaunlicherweise eine hohe Tendenz zur Ausbildung flüssigkristalliner Phasen, insbesondere zur Ausbildung von nematischen, smektisch A oder getilteten smektischen (vor allem $S_C$) Phasen im Falle der

achiralen Verbindungen bzw. zur Ausbildung von cholesterischen oder chiral getilteten smektischen (vor allem $S_C^*$ ) Phasen im Falle der chiralen Verbindungen. Diese Mesophasentypen eignen sich insbesondere zur Erzielung nematischen, cholesterischen oder chiral getilteten smektischen Phasen in Gemischen.

Die Verbindungen der Formel I besitzen eine hohe Stabilität, sind vergleichsweise einfach herzustellen und sind sehr gut löslich untereinander und in bekannten Flüssigkristallmaterialien. Ferner besitzen sie tiefe Viskositäten und ergeben in Anzeigevorrichtungen kurze Ansprechzeiten.

Die erfindungsgemässen Verbindungen ermöglichen daher eine weitere Optimierung der Flüssigkristallgemische und eine Modifizierung der elektro-optischen Eigenschaften, z.B. der Viskositäten oder der elastischen Eigenschaften.

Die Eigenschaften der Verbindungen der Formel I können je nach Anzahl und Bedeutung der Ringe und je nach Wahl der Substituenten in breiten Bereichen variiert werden. Beispielsweise führen aromatische Ringe zu höheren und gesättigte Ringe zu tieferen Werten der optischen Anisotropie, und eine grössere Zahl von Ringen führt im allgemeinen zu höheren Klärpunkten. Polare Endgruppen, wie Cyano, Halogen oder -NCS, und Ringe wie Pyrimidin-2,5-diyl, trans-1,3-Dioxan-2,5-diyl etc. erhöhen die dielektrische Anisotropie und laterale Halogen- oder Cyanosubstituenten, Pyridazin-2,5-diyl etc. vermindern die dielektrische Anisotropie der Verbindungen der Formel I. Ferner können z.B. durch laterale Substitution der Ringe die Mesophasebereiche und die Löslichkeit und/oder durch eine C-C-Doppelbindung in der Seitenkette die elastischen Eigenschaften, die Schwellenspannunen, die Ansprechzeiten und die Mesophasen weiter modifiziert werden.

Der obige Ausdruck "unsubstituiertes oder mit Methyl, Halogen und/oder Cyano substituiertes 1,4-Phenylen, in welchem gegebenenfalls 1-4 CH-Gruppen durch Stickstoff ersetzt sind, "umfasst im Rahmen der vorliegenden Erfindung Gruppen wie 1,4-Phenylen, Pyridin-2,5-diyl, Pyrazin-2,5-diyl, Pyrimidin-2,5-diyl, Pyridazin-3,6-diyl und Tetrazin-3,6-diyl sowie mit Methyl, Halogen und/oder Cyano substituierte Ringe, insbesondere mit Methyl, Halogen und/oder Cyano substituiertes 1,4-Phenylen, wie Methyl-1,4-phenylen, Fluor-1,4-phenylen, 2,3-Difluor-1,4-phenylen, Chlor-1,4-phenylen, Cyano-1,4-phenylen oder 2,3-Dicyano-1,4-phenylen.

Der Ausdruck "unsubstituiertes oder mit Methyl und/oder Cyano substituiertes trans-1,4-Cyclohexylen, in welchem gegebenenfalls 2 $CH_2$-Gruppen durch Sauerstoff und/oder Schwefel ersetzt sind," umfasst Gruppen wie trans-1,4-Cyclohexylen, trans-1,3-Dioxan-2,5-diyl, trans-1,3-Dithian-2,5-diyl, 1-Cyano-trans-1,4-cyclohexylen, 2-Methyl-trans-1,4-cyclohexylen oder 1-Methyl-trans-1,4-cyclohexylen und dergleichen.

Der Ausdruck "Halogen" umfasst Fluor, Chlor, Brom und Jod.

Der Ausdruck "unsubstituierte oder mit Halogen und/oder Cyano substituierte Alkyl- und/oder Alkenylgruppe, mit 1 bzw. 2 bis 18 Kohlenstoffatomen in welcher gegebenenfalls 1 oder 2 nicht benachbarte $CH_2$-Gruppen durch -O-, -COO- und/oder -OOC- ersetzt sind," umfasst geradkettige und verzweigte (gegebenenfalls chirale) Reste wie Alkyl, 1-Alkenyl (insbesondere 1E-Alkenyl), 3-Alkenyl (insbesondere 3E-Alkenyl), 4-Alkenyl (insbesondere 4Z-Alkenyl), 5-Alkenyl, 6-Alkenyl, 7-Alkenyl und dergleichen, davon abgeleitete Reste mit Aether- und/oder Esterfunktionen, wie Alkoxy, Alkoxymethoxy, Alkenyloxy (z.B. 2E-Alkenyloxy, 3-Alkenyloxy, 4-Alkenyloxy, 5-Alkenyloxy etc.), Alkanoyloxy, Alkenoyloxy, Alkoxycarbonyl, Alkenyloxycarbonyl, 1-(Alkoxycarbonyl)äthoxy und dergleichen, und abgeleitete Reste mit Halogen- und/oder Cyano-Substituenten, wie 1-Fluoralkyl, 1-Chloralkyl, 1-Cyanoalkyl, 1-Fluoralkoxy, 2-Fluoralkoxy, 1-Chloralkoxy, 2-Chloralkoxy, 1-Cyanoalkoxy, 2-Cyanoalkoxy, 2-Fluoralkanoyloxy, 2-Chloralkanoyloxy, 1-Fluoralkoxycarbonyl, 2-Fluoralkoxycarbonyl, 1-Chloralkoxycarbonyl, 2-Chloralkoxycarbonyl, 2-Cyanoalkoxycarbonyl und dergleichen. Beispiele solcher Reste sind Methyl, Aethyl, Propyl, Butyl, Pentyl, Hexyl, Heptyl, Octyl, Nonyl, Decyl, Undecyl, Dodecyl, 1-Methylheptyl, 2-Methylbutyl, 3-Methylpentyl, 4-Methylhexyl, 5-Methylheptyl, 6-Methyloctyl, Methoxy, Aethoxy, Propyloxy Butyloxy, Pentyloxy, Hexyloxy, Heptyloxy, Octyloxy, Nonyloxy, Decyloxy, Undecyloxy, Dodecyloxy, 1-Methylheptyloxy, 2-Methylbutyloxy, 3-Methylpentyloxy, 4-Methylhexyloxy, 5-Methylheptyloxy, 6-Methyloctyloxy, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Acetoxy, Propanoyloxy, Butanoyloxy, Pentanoyloxy, Hexanoyloxy, Fluoracetoxy, 2-Fluorpropanoyloxy, 2-Fluorbutanoyloxy, 2-Fluorpentanoyloxy, 2-Fluorhexanoyloxy, Chloracetoxy, 2-Chlorpropanoyloxy, 2-Chlorbutanoyloxy, 2-Chlorpentanoyloxy, 2-Chlorhexanoyloxy, 2-Chlorheptanoyloxy, 1-Methylheptyloxycarbonyl, 2-Methylbutyloxycarbonyl, 2-Methylpentyloxycarbonyl, 2-Methylhexyloxycarbonyl, 2-Fluorpropyloxycarbonyl, 2-Fluorbutyloxycarbonyl, 2-Fluorpentyloxycarbonyl, 2-Fluorhexyloxycarbonyl, 2-Fluor-3-methylbutyloxycarbonyl, 2-Fluor--4-methylpentyloxycarbonyl, 2-Chlorpropyloxycarbonyl, 2-Chlorbutyloxycarbonyl, 2-Chlorpentyloxycarbonyl, 2-Chlorhexyloxycarbonyl, 2-Chlor-3-methylbutyloxycarbonyl, 2-Chlor-4-methylpentyloxycarbonyl, 2-Cyanopropyloxycarbonyl, 2-Cyanobutyloxycarbonyl, 2-Cyanopentyloxycarbonyl, 2-Cyanohexyloxycarbonyl, 2-Cyano-3-methylbutyloxycarbonyl oder 2-Cyano-4-methylpentyloxycarbonyl.

Der Ausdruck "Tetralin-2,6-diyl" bezeichnet 1,2,3,4-Tetrahydro-naphthalin-2,6-diyl. Der Ausdruck "Decalin-2,6-diyl" umfasst von Decahydronaphthalin abgeleitete, 2,6-disubstituierte Gruppen, insbesondee (4a$\alpha$H,8a$\beta$H)-Decahydronaphthalin-2$\alpha$,6$\beta$-yl.

Der Ausdruck "gesättigter Ring" umfasst unsubstituiertes oder mit Methyl und/oder Cyano-substituiertes trans-1,4-Cyclohexylen, in welchem gegebenenfalls 2 $CH_2$-Gruppen durch Sauerstoff und/oder Schwefel ersetzt sind, sowie Bicyclo[2,2,2]octan-1,4-diyl, Decalin-2,6-diyl und im Zusammenhang mit in 2-Stellung gebundenen Gruppen auch Tetralin-2,6-diyl.

Der Ausdruck "aromatischer Ring" umfasst unsubstituietes oder mit Methyl. Halogen und/oder Cyano substituiertes 1,4-Phenylen, in welchem gegebenenfalls 1-4 CH-Gruppen durch Stickstoff ersetzt sind, sowie 1,3,4-Thiadiazol-2,5-diyl, Naphthalin-2,6-diyl und im Zusammenhang mit in 6-Stellung gebundenen Gruppen auch Tetralin-2,6-diyl.

Im allgemeinen sind diejenigen Verbindungen der Formel I bevorzugt, worin $A^1$, $A^2$, $A^3$, $A^4$ und $A^5$ unabhängig voneinander 1,4-Phenylen oder trans-1,4-Cyclohexylen bezeichnen, oder eine der Gruppen $A^1$, $A^2$, $A^3$, $A^4$ und $A^5$ auch mit Methyl, Halogen und/oder Cyano substituiertes 1,4-Phenylen oder mit Methyl und/oder Cyano substituiertes trans-1,4-Cyclohexylen bezeichnet, und/oder eine der Gruppen $A^1$, $A^2$, $A^3$, $A^4$ und $A^5$ auch 1,4-Phenylen, in welchem 1-4 (vorzugsweise 1 oder 2) CH-Gruppen durch Stickstoff ersetzt sind, trans-1,4-Cyclohexylen, in welchem 2 $CH_2$-Gruppen durch Sauerstoff und/oder Schwefel ersetzt sind, Bicyclo[2,2,2]octan-1,4-diyl, 1,3,4-Thiadiazol-2,5-diyl, Naphthalin-2,6-diyl, Tetralin-2,6-diyl oder Decalin-2,6-diyl bezeichnet. Ferner sind diejenigen Verbindungen der Formel I bevorzugt, worin eine der Gruppen $Z^2$, $Z^3$ und $Z^4$ (insbesondere $Z^2$) eine einfache Kovalenzbindung, $-CH_2CH_2-$, $-CH_2O-$, $-OCH_2-$, -COO-, -OOC-, $-CH_2CH_2CH_2O-$ oder $-OCH_2CH_2CH_2-$ bedeutet und die beiden andern der Gruppen $Z^2$, $Z^3$ und $Z^4$ je eine einfache Kovalenzbindung, -COO- und/oder -OOC- bedeuten.

Vorzugsweise ist mindestens eine der Gruppen $A^1$, $A^2$, $A^3$, $A^4$ und $A^5$ (insbesondere mindestens eine der an $Z^1$ gebundenen Gruppen $A^1$ und $A^2$) ein gesättigter Ring, insbesondere trans-1,4-Cyclohexylen oder trans-1,3-Dioxan-2,5-diyl. Diese Verbindungen besitzen in der Regel eine höhere Tendenz zur Ausbildung flüssigkristalliner Phasen. Vorzugsweise ist die Methylengruppe der Trimethylengruppe $Z^1$ mit einem gesättigten Ring (insbesondere trans-1,4-Cyclohexylen oder trans-1,3-Dioxan-2,5-diyl) verknüpft; d.h. bevorzugte Verbindungen der Formel I sind diejenigen, worin $Z^1$ die Gruppe $-CH_2CH_2CH_2O-$ und $A^1$ einen gesättigten Ring bedeuten oder $Z^1$ die Gruppe $-OCH_2CH_2CH_2-$ und $A^2$ einen gesättigten Ring bedeuten, und insbesondere diejenigen, worin der gesättigte Ring $A^1$ bzw. $A^2$ trans-1,4-Cyclohexylen oder trans-1,3-Dioxan-2,5-diyl ist.

Eine bevorzugte Gruppe erfindungsgemässer Verbindungen sind die Verbindungen der allgemeinen Formel

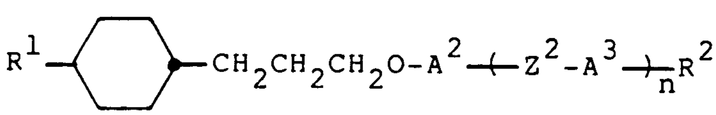

IA

insbesondere die Verbindungen der allgemeinen Formeln

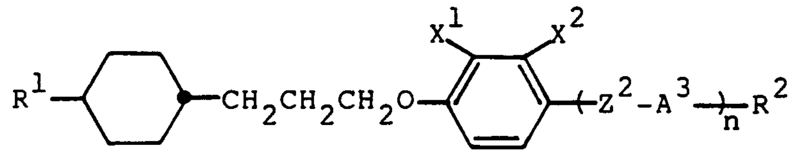

IB

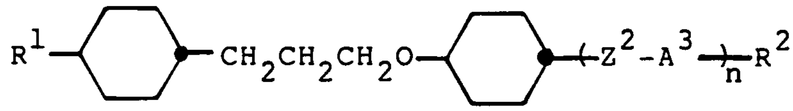

IC

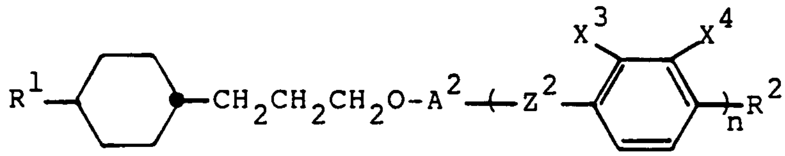

ID

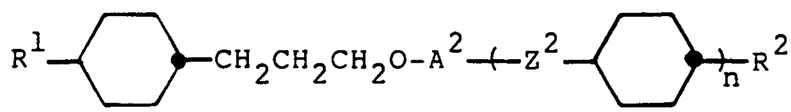

IE

worin jeweils $A^2$, $A^3$, $R^1$, $R^2$, $Z^2$ und n jeweils die obigen Bedeutungen haben und $X^1$, $X^2$, $X^3$ und $X^4$ unabhängig voneinander Wasserstoff, Methyl, Halogen oder Cyano bezeichnen.

Eine weitere bevorzugte Gruppe von erfindungsgemässen Verbindungen sind die Verbindungen der allgemeinen Formel

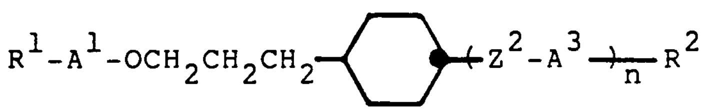

IF

insbesondere die Verbindungen der allgemeinen Formel

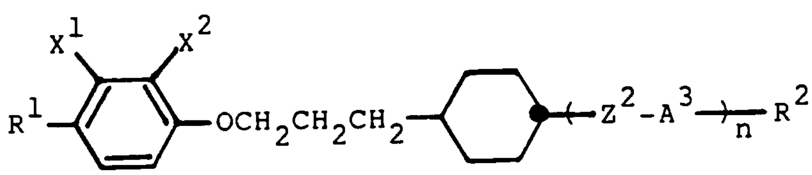

IG

worin $A^1$, $A^3$, $Z^2$, $R^1$, $R^2$ und n die obigen Bedeutungen haben und $X^1$ und $X^2$ unabhängig voneinander Wasserstoff, Methyl, Halogen oder Cyano bedeuten.

Die obigen Bemerkungen bezüglich $A^2$, $A^3$, $A^4$, $A^5$, $Z^2$, $Z^3$ und $Z^4$ in Formel I gelten sinngemäss auch für die Formeln IA-IG.

Beispiele besonders bevorzugter Untergruppen sind die Verbindungen der allgemeinen Formeln

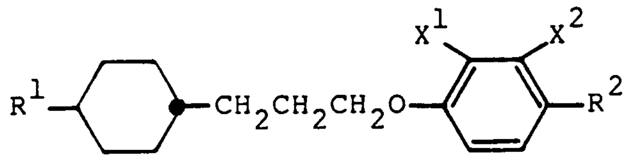

I-1

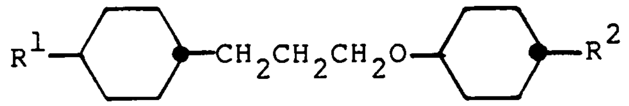

I-2

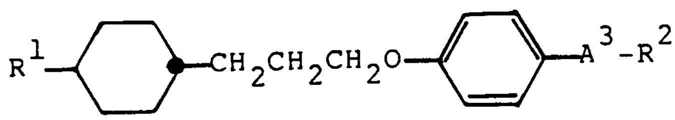

I-3

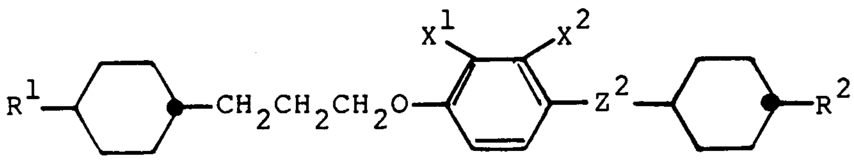

I-4

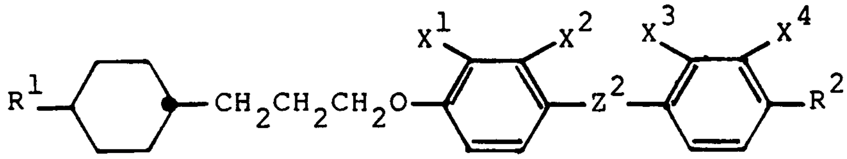

I-5

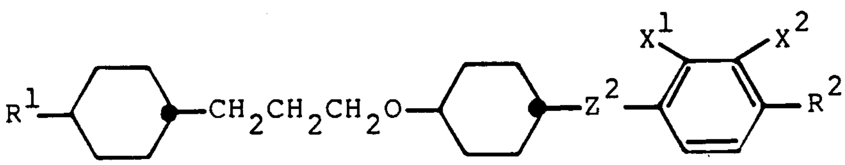

I-6

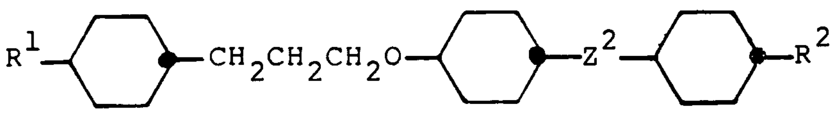

I-7

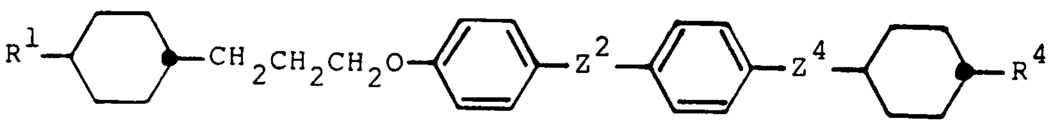

I-8

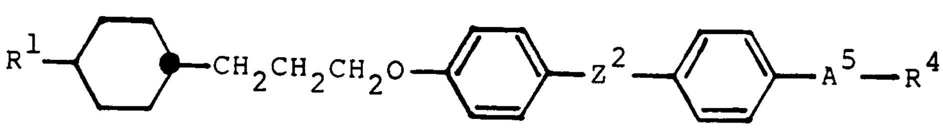

I-9

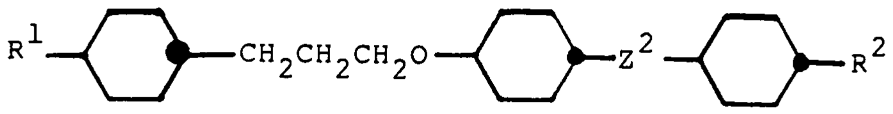

I-10

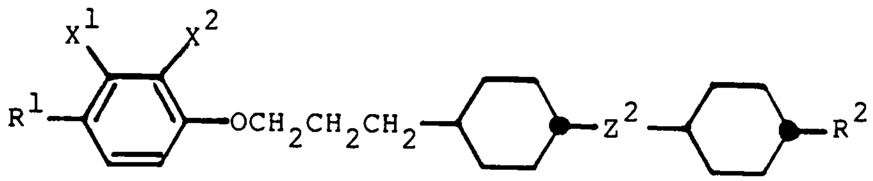

I-11

worin $A^3$, $A^5$, $R^1$, $R^2$, $R^4$, $X^1$, $X^2$, $X^3$, $X^4$, $Z^2$ und $Z^4$ jeweils die obigen Bedeutungen haben.

Die Verbindungen der Formeln I und IA bis IG, worin n für die Zahl 1 steht, sind meist Flüssigkristalle mit relativ hohen Klärpunkten. Diejenigen Verbindungen, worin n für die Zahl 0 steht, eignen sich hingegen vor allem als niederviskose Dotierstoffe, insbesondere wenn $R^1$ eine Gruppe $R^3$ und $R^2$ eine Gruppe $R^4$ bedeuten.

Die Gruppen $A^1$, $A^2$, $A^3$, $A^4$ und $A^5$ in den obigen Formeln I, IA bis IG und I-1 bis I-11 bedeuten jeweils vorzugsweise 1,4-Phenylen, Fluor-1,4-phenylen, 2,3-Difluor-1,4-phenylen, Chlor-1,4-phenylen, Cyano-1,4-phenylen, 2,3-Dicyano-1,4-phenylen, Methyl-1,4-phenylen, Pyridin-2,5-diyl, Pyazin-2,5-diyl, Pyrimidin-2,5-diyl, trans-1,4-Cyclohexylen, trans-1,3-Dioxan-2,5-diyl, Bicyclo[2,2,2]octan-1,4-diyl, 1,3,4-Thiadiazol-2,5-diyl, Naphthalin-2,6-diyl, Tetralin-2,6-diyl oder Decalin-2,6-diyl, wobei eine gegebenenfalls vorhandene Gruppe $A^4$ und/oder $A^5$ bevorzugt 1,4-Phenylen oder trans-1,4-Cyclohexylen bedeuet. Besonders bevorzugt sind jeweils diejenigen Verbindungen der obigen Formeln I, IA bis IG und I-1 bis I-11, worin eine der in der Formel vorkommenden Gruppen A (insbesondere $A^3$ oder $A^4$) 1,4-Phenylen, Fluor-1,4-phenylen, 2,3-Difluor-1,4-phenylen, Chlor-1,4-phenylen, Cyano-1,4-phenylen, 2,3-Dicyano-1,4-phenylen, Methyl-1,4-phenylen oder trans-1,4-Cylohexylen bedeutet, und/oder eine der in der Formel vorkommenden Gruppen A (insbesondere $A^2$ oder $A^3$) 1,4-Phenylen, Pyridin-2,5-diyl, Pyrazin-2,5-diyl, Pyrimidin-2,5-diyl, trans-1,4-Cyclohexylen, trans-1,3-Dioxan-2,5-diyl, Bicyclo[2,2,2]octan-1,4-diyl, 1,3,4-Thiadiazol-2,5-diyl, Naphthalin-2,6-diyl, Tetralin-2,6-diyl oder Decalin-2,6-diyl bedeuten, und weitere in der Formel gegebenenfalls vorkommende Gruppen A (insbesondere auch $A^4$ und $A^5$) unabhängig voneinander 1,4-Phenylen oder trans-1,4-Cyclohexylen bedeuten.

In den obigen Formeln I, IA bis IG and I-1 bis I-11 steht vorzugsweise eine der Gruppen $Z^2$, $Z^3$ und $Z^4$ für eine einfache Kovalenzbindung, $-CH_2CH_2-$, $-CH_2O-$, $-OCH_2-$, -COO-, -OOC-,$-CH_2CH_2CH_2O-$ oder $-OCH_2CH_2CH_2-$ und die beiden anderen der Gruppen $Z^2$, $Z^3$ und $Z^4$ (bzw. die andere der Gruppen $Z^3$ und $Z^4$ in den Formeln I-1, I-2 und I-3 bzw. die andere der Gruppen $Z^2$ und $Z^3$ in Formel I-9) stehen vorzugsweise für einfache Kovalenzbindungen.

$X^1$, $X^2$, $X^3$ und $X^4$ in den obigen Formeln IB, ID, IG, I-1, I-4, I-5, I-6 und I-11 bedeuten unabhängig voneinander vorzugsweise Wasserstoff, Methyl, Fluor, Chlor und/oder Cyano, besonders bevorzugt Wasserstoff und/oder Fluor. Vorzugsweise besitzen höchstens einer oder zwei der Substituenten $X^1$-$X^4$ im Molekül eine von Wasserstoff verschiedene Bedeutung und insbesondere können auch alle $X^1$-$X^4$ Wasserstoff bedeuten.

Besonders bevorzugt Bedeutungen von $A^3$ in Formel I-3 sind 1,4-Phenylen, Fluor-1,4-phenylen, 2,3-Difluor-1,4-phenylen, Pyridin-2,5-diyl, Pyrimidin-2,5-diyl, trans-1,4-Cyclohexylen, trans-1,3-Dioxan-2,5-diyl und Decalin-2,6-diyl.

In Formel I-5 steht $Z^2$ vorzugsweise für eine einfache Kovalenzbindung, -COO- oder -OOC-. Ferner sind im allgemeinen diejenigen Verbindungen der Formel I-5 bevorzugt, worin einer der Substituenten $X^1$-$X^4$ -(vorzugsweise $X^4$) Wasserstoff, Fluor, Chlor, Brom oder Cyano bedeutet, ein weiterer der Substituenten $X^1$-$X^4$ (vorzugsweise $X^3$) Wasserstoff oder Fluor bedeutet und die beiden andern der Substituenten $X^1$-$X^4$ Wasserstoff bedeuten.

In den Formeln I-8 und I-9 steht $Z^2$ vorzugsweise für eine einfache Kovalenzbindung, -COO- oder -OOC-. Ferner kann $A^5$ in Formel I-9 vorzugsweise 1,4-Phenylen, Pyrimidin-2,5-diyl, trans-1,4-Cyclohexylen oder Bicyclo[2.2.2.]octan-1,4-diyl bedeuten.

In den Formeln I-10 und I-11 steht $Z^2$ vorzugsweise für eine einfache Kovalenzbindung oder $-CH_2CH_2-$. Besonders bevorzugte Bedeutungen von $X^1$ und $X^2$ in Formel I-11 sind Wasserstoff und Fluor.

In den obigen Formeln I, IA bis IG und I-1 bis I-11 bedeuten vorzugsweise $R^1$ eine Gruppe $R^3$ und $R^2$ eine Gruppe $R^4$. Verbindungen, worin $R^1$ eine Gruppe $R^3$-$A^4$-$Z^3$- und/oder $R^2$ eine Gruppe $R^4$-$A^5$-$Z^4$- bedeuten, sind jedoch ebenfalls von Interesse, vor allem als Dotierstofe zur Erhöhung der Klärpunkte von Gemischen.

$R^3$ und $R^4$ besitzen vorzugsweise höchstens je etwa 18 Kohlenstoffatome, d.h. $R^3$ und $R^4$ in den obigen Formeln bedeuten unabhängig voneinander vorzugsweise eine unsubstituierte oder mit Halogen und/oder Cyano substituierte $C_1$-$C_{18}$-Alkyl- oder $C_2$-$C_{18}$-Alkenylgruppen, in welcher gegebenenfalls 1 oder 2 nicht benachbarte $CH_2$-Gruppen durch -O-, -COO- und/oder -OOC- ersetzt sind, oder einer der Reste $R^3$ und $R^4$ kann vorzugsweise auch Wasserstoff, Halogen, Cyano oder -NCS bedeuten. Für nematische und cholesterische Anwendungen sind im allgemeinen kurze Reste (z.B. Reste mit höchstens 12, vorzugsweise höchstens 7 Kohlenstoffatomen) bevorzugt und vorzugsweise kann auch einer der Reste Wasserstoff, Halogen, Cyano oder -NCS bedeuten. Für smektische Anwendung (insbesondere getiltete smektische Phasen) sind im allgemeinen diejenigen Verbindungen bevorzugt, worin $R^3$ und $R^4$ unabhängig voneinander eine unsubstituierte oder mit Halogen und/oder Cyano substituierte $C_1$-$C_{18}$-Alkyl- oder $C_2$-$C_{18}$-Alkenylgruppe bedeuten, in welcher gegebenenfalls 1 oder 2 nicht benachbarte $CH_2$-Gruppen durch -O-, -COO-und/oder -OOC- ersetzt sind, und die Summe der Kohlenstoffatome in $R^3$ und $R^4$ zusammen mindestens 10, vorzugsweise mindestens 12 beträgt.

Besonders bevorzugte Reste $R^3$ sind Alkyl, Alkenyl, Alkoxy, Alkenyloxy, Alkoxycarbonyl, Alkenyloxycarbonyl, Alkanoyloxy und Alkenoyloxy, insbesondee Alkyl, Alkenyl, Alkoxy und Alkenyloxy. Besonders bevorzugte Reste $R^4$ sind Alkyl, Alkenyl, Alkoxy, Alkenyloxy, Alkoxycarbonyl, Alkenyloxycarbonyl, Alkanoyloxy und Alkenoyloxy, insbesondere Alkyl, Alkenyl, Alkoxy und Alkenyloxy, sowie Halogen (insbesondere Fluor und Chlor), Cyano und -NCS. Geradkettige Reste $R^3$ bzw. $R^4$ sind im allgemeinen bevorzugt. Um beispielsweise chirale Dotierstoffe für cholesterische oder für chiral getiltete smektische Flüssigkristalle zu erhalten, können jedoch auch verzweigt-kettige und/oder mit Halogen und/oder Cyano substituierte Reste verwendet werden. Um bei smektischen Anwendungen eine hohe spontane Polarisation zu erhalten, sollte hierbei das Chiralitätszentrum (d.h. die Kettenverzweigung oder der Halogen- oder Cyanosubstituent) vorzugsweise nahe am Ringsystem, beispielsweise in 1- oder 2-Stellung des Restes $R^3$ bzw. $R^4$ sein. Ferner bleibt die Tendenz zur Bildung flüssigkristalliner Phasen grundsätzlich erhalten, wenn eine oder 2 nicht benachbarte $CH_2$-Gruppen in den Ketten durch -O-, -COO- und/oder -OOC- ersetzt werden.

Die Mesophasenbereiche, die Schwellenspannung, die Ansprechgeschwindigkeit, die Steilheit der Transmissionskennlinie etc. können ferner variiert werden durch Wahl der Stellung der C-C-Doppelbindung in ungesättigten Resten wie Alkenyl, Alkenyloxy und dergleichen. Der Effekt ist grundsätzlich bekannt z.B. aus Mol. Cryst. Liq. Cryst. 122, 241 (1985), 131, 109 (1985) und 148, 123 (1987). Bevorzugt sind Reste, welche die Doppelbindung in 1-Stellung (insbesondere E-Isomer), in 3-Stellung (insbesondere E-Isomer) oder in 4-Stellung (insbesondere Z-Isomer) der Kette unter Einschluss allfälliger Heteroatome aufweisen, wie 1E-Alkenyl, 3E-Alkenyl, 4Z-Alkenyl, 2E-Alkenyloxy, 3Z-Alkenyloxy und dergleichen. Ferner kann die Doppelbindung vorzugsweise auch in endständiger Position stehen, insbesondere im Falle von Verbindungen für smektische Anwendungen. Beispiele bevorzugter Reste mit endständiger Stellung der Doppelbindung sind 6-Heptenyl, 7-Octenyl, 8-Nonenyl, 9-Decenyl, 10-Undecenyl, 11-Dodecenyl, 5-Hexenyloxy, 6-Heptenyloxy, 7-Octenyloxy, 8-Nonenyloxy, 9-Decenyloxy, 10-Undecenyloxy oder 11-Dodecenyloxy.

Die Herstellung der erfindungsgemässen Verbindungen kann in an sich bekannter Weise aus bekannten oder Analogen bekannter Verbindungen erfolgen. In der Regel erfolgt die Herstellung am einfachsten durch Verätherung der betreffenden Hydroxyverbindung mit dem entsprechenden 3-substituierten 1-Propylhalogenid (vorzugsweise dem 1-Propylbromid). Wenn eine der Gruppen $Z^2$, $Z^3$ und $Z^4$ -COO- oder -OOC- bedeutet, kann die Herstellung vorzugsweise durch Veresterung der entspechenden Carbonsäure mit der entsprechenden Hydroxyverbindung oder durch Veresterung geeigneter Derivate hiervon erfolgen. Ferner können die Verbindungen, worin einer der Ringe 1,3-Dioxan oder 1,3-Dithian bedeutet, durch Umsetzung des entsprechenden Aldehyds mit dem ensprechenden 2-substituierten 1,3-Propandiol oder 1,3-Propandithiol erhalten werden.

Die erfindungsgemässen Verbindungen können in Form von Gemischen untereinander und/oder mit andern Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem in Handel erhältlich.

Die Erfindung betrifft daher ebenfalls ein flüssigkristallines Gemisch mit mindestens 2 Komponenten, welches dadurch gekennzeichnet ist, dass mindestens eine Komponente eine Verbindung der Formel I (insbesondere eine der als bevorzugt genannten Verbindungen) ist.

Aufgrund der guten Löslichkeit sowie anderseits der grossen Variationsbreite der Eigenschaften und Anwendungsbereiche, kann der Anteil an Verbindungen der Formel I in den erfindungsgemässen Gemischen in einem breiten Bereich variieren und etwa 0,1 bis 100 Gew.% betragen. Beispielsweise kann das Gemisch aus Verbindungen der Formel I bestehen. Anderseits werden z.B. chirale Dotierstoffe oft nur in relativ kleinen Mengen z.B. etwa 0,1 bis 10 Gew.% einsetzt. Im allgemeinen beträgt jedoch der Anteil an Verbindungen der Formel I in den erfindungsgemässen Gemischen etwa 1-60 Gew.%. Bevorzugt ist in der Regel ein Bereich von etwa 5-30 Gew.%.

Die erfindungsgemässen Gemische für nematische oder cholesterische Anwendungen enthalten neben einer oder mehreren Verbindungen der Formel I vorzugsweise eine oder mehrere Verbindungen aus der Gruppe der Verbindungen der allgemeinen Formeln

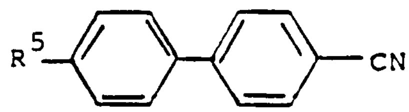

II

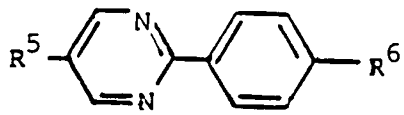

III

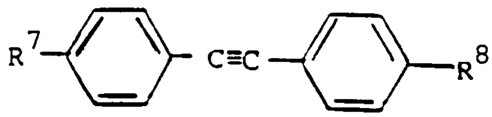

IV

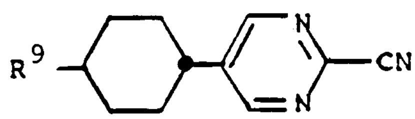

V

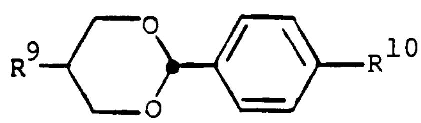

VI

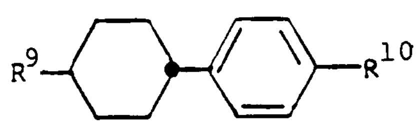

VII

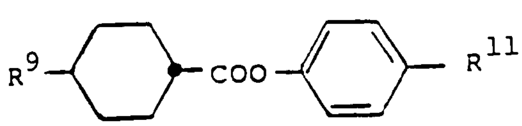

VIII

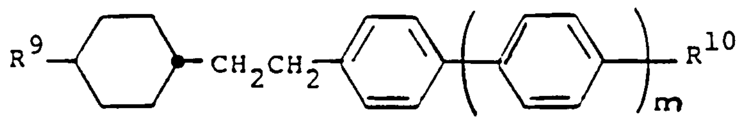
IX

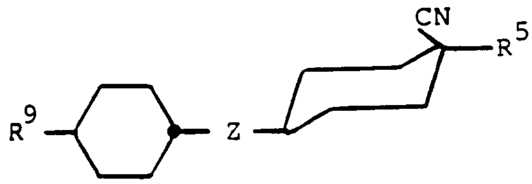
X

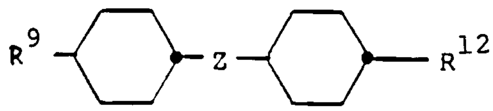
XI

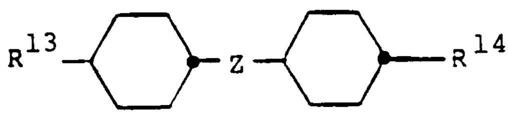
XII

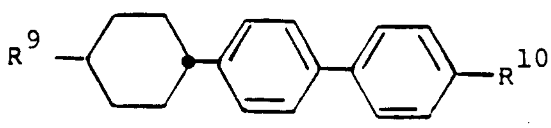
XIII

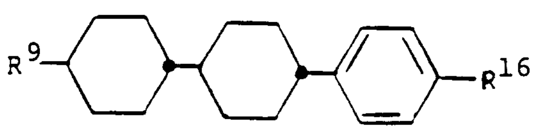
XIV

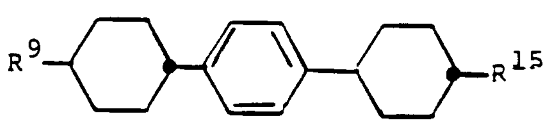
XV

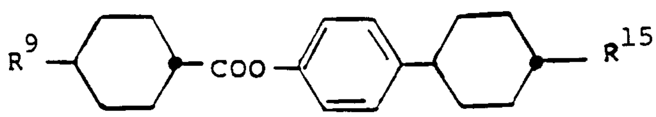
XVI

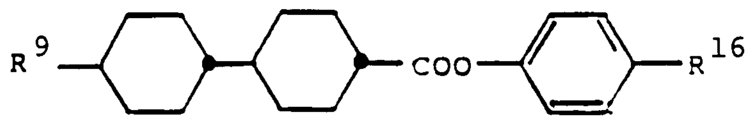

XVII

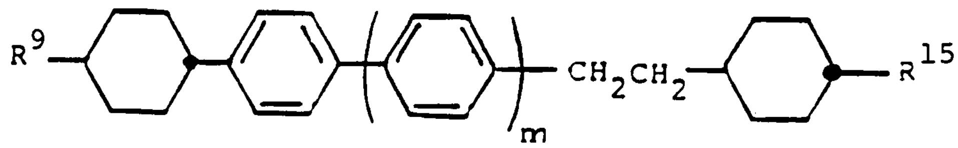

XVIII

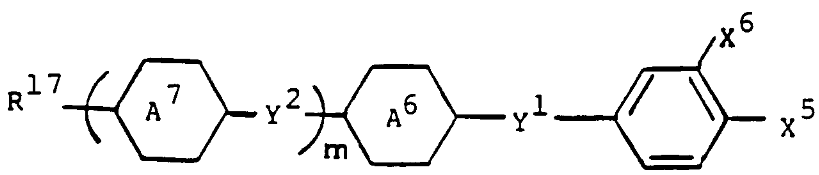

XIX

worin $R^5$ Alkyl, 3E-Alkenyl oder 4-Alkenyl bedeutet; $R^6$ Cyano oder Fluor darstellt; $R^7$ und $R^8$ Alkyl oder Alkoxy bezeichnen; $R^9$ und $R^{15}$ unabhängig voneinander Alkyl, 1E-Alkenyl, 3E-Alkenyl oder 4-Alkenyl bedeuten; $R^{10}$ Cyano, -NCS, Alkyl, 1E-Alkenyl, 3E-Alkenyl, 4-Alkenyl, Alkoxy, 2E-Alkenyloxy oder 3-Alkenyloxy bezeichnet; $R^{11}$ Alkoxy, 2E-Alkenyloxy oder 3-Alkenyloxy bedeutet: m für die Zahl 0 oder 1 steht: Z eine einfache Kovalenzbindung oder $-CH_2CH_2-$ darstellt; $R^{12}$ Cyano, Alkyl, 1E-Alkenyl, 3E-Alkenyl oder 4-Alkenyl bedeutet; $R^{13}$ Alkyl, 1E-Alkenyl oder 4-Alkenyl bezeichnet; $R^{14}$ Alkoxy, 2E-Alkenyloxy oder 3-Alkenyloxy darstellt; $R^{16}$ Cyano, Alkyl, 1E-Alkenyl, 3E-Alkenyl, 4-Alkenyl, Alkoxy, 2E-Alkenyloxy oder 3-Alkenyloxy bezeichnet; $X^5$ Fluor oder Chlor und $X^5$ Wasserstoff, Fluor oder Chlor bezeichnen; $R^{17}$ Alkyl, 3E-Alkenyl, 4-Alkenyl, Alkoxy, 2E-Alkenyloxy oder 3-Alkenyloxy bedeutet; eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; und die Ringe $A^6$ und $A^7$ unabhängig voneinander trans-1,4-Cyclohexylen, in welchem gegebenenfalls 2 nicht benachbarte $CH_2$-Gruppen durch Sauerstoff ersetzt sind, oder 1,4-Phenylen, in welchem gegebenenfalls 1 oder 2 $CH_2$-Gruppen durch Stickstoff ersetzt sind, darstellen.

Vorzugsweise besitzen die Reste $R^5$ und $R^7$-$R^{17}$ höchstens je 12 Kohlenstoffatome, insbesondere höchstens je 7 Kohlenstoffatome.

Die erfindungsgemässen Gemische für smektische Anwendungen (insbesondere für getiltete smektische oder chiral getiltete smektische Phasen) enthalten neben einer oder mehreren Verbindungen der Formel I vorzugsweise eine oder mehrere Verbindungen aus der Gruppe der Verbindungen der allgemeinen Formeln

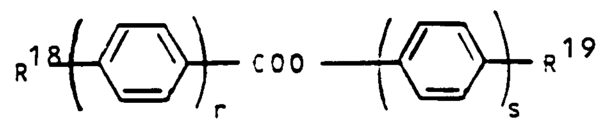

XX

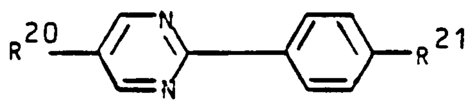

XXI

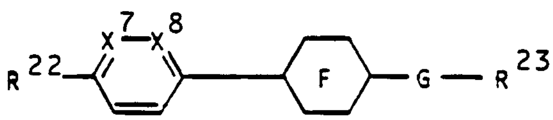

XXII

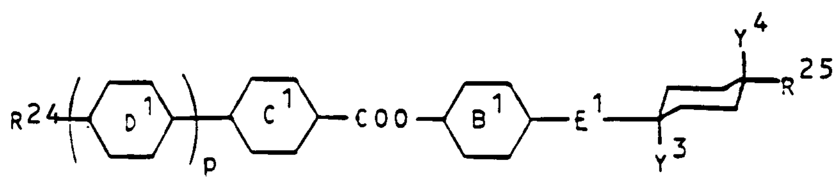

XXIII

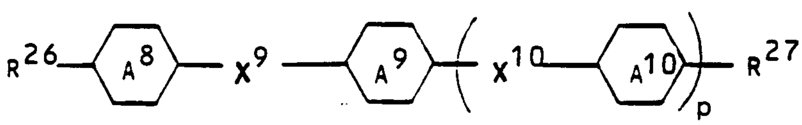

XXIV

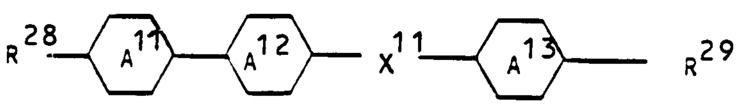

XXV

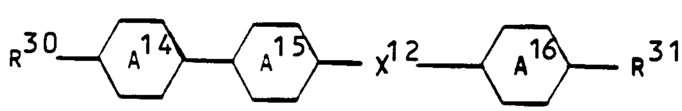

XXVI

worin $R^{18}$ und $R^{19}$ Alkyl, Alkoxy, Alkanoyl, Alkanoyloxy, Alkoxycarbonyl oder Alkoxycarbonyloxy mit bis zu 18 Kohlenstoffatomen bedeuten; r und s unabhängig voneinander 1 oder 2 bedeuten; $R^{20}$ und $R^{21}$ Alkyl oder Alkoxy mit 1-18 Kohlenstoffatomen darstellen; $X^7$ für CH und $X^8$ für N steht oder $X^7$ für N und $X^8$ für CH steht; G eine einfache Kovalenzbindung, trans-1,4-Cyclohexylen, cis-4-Cyano-trans-1,4-cyclohexylen oder gegebenenfalls mit Halogen oder Methyl substituiertes 1,4-Phenylen bedeutet; Ring F trans-1,4-Cyclohexylen, gegebenenfalls mit Halogen oder Methyl substituiertes 1,4-Phenylen oder, wenn G eine einfache Kovalenzbindung bedeutet, auch cis-4-Cyano-trans-1,4-cyclohexylen darstellt; $R^{22}$ und $R^{23}$ je eine gegebenenfalls halogensubstituierte Alkyl- oder Alkenylgruppe bezeichnen, in welcher gegebenenfalls eine oder zwei nicht benachbarte $CH_2$-Gruppen durch -O-, -COO- und/oder -OOC- ersetzt sind; p für die Zahl 0 oder 1 steht; $E^1$ eine einfache Kovalenzbindung, $-CH_2-CH_2-$, $-OCH_2-$, -COO- oder -OOC- bedeutet; die

Ringe $B^1$, $C^1$ und $D^1$ gegebenenfalls mit Cyano, Halogen oder Niederalkyl substituiertes 1,4-Phenylen bezeichnen; $Y^3$ und $Y^4$ Wasserstoff bedeuten oder einer der Substituenten $Y^3$ und $Y^4$ auch Cyano bedeutet; $R^{24}$ und $R^{25}$ unabhängig voneinander gegebenenfalls halogensubstituiertes $C_1$-$C_{18}$-Alkyl oder gegebenenfalls halogensubstituiertes $C_2$-$C_{18}$-Alkenyl darstellen, in welchen gegebenenfalls eine oder zwei nicht benachbarte $CH_2$-Gruppen durch Sauerstoff ersetzt sind; $X^9$ eine einfache Kovalenzbindung, -COO- oder -OOC- und $X^{10}$ eine einfache Kovalenzbindung, -COO-, -OOC-, $-CH_2CH_2-$, $-OCH_2$-oder $-CH_2O-$ darstellen; die Ringe $A^8$, $A^9$ und $A^{10}$ unabhängig voneinander unsubstituiertes oder mit Cyano, Halogen oder Niederalkyl substituiertes 1,4-Phenylen oder einer der Ringe nach Pyrimidin-2,5-diyl oder Pyrazin-2,5-diyl bedeutet und/oder, wenn p für die Zahl 1 steht, einer der Ringe auch trans-1,4-Cyclohexylen oder trans-m-Dioxan-2,5-diyl bedeutet; $R^{26}$ eine gegebenenfalls halogensubstituierte Alkenylgruppe mit bis zu 18 Kohlenstoffatomen bedeutet, worin gegebenenfalls 1 oder 2 nicht benachbarte $CH_2$-Gruppen durch -O-, -CO-, -COO- oder -OOC- ersetzt sind und/oder gegebenenfalls eine C-C-Einfachbindung durch eine C-C-Doppelbindung ersetzt ist; $R^{27}$ eine gegebenenfalls halogensubstituierte Alkylgruppe mit bis zu 18 Kohlenstoffatomen bedeutet, worin gegebenenfalls 1 oder 2 nicht benachbarte $CH_2$-Gruppen durch -O-, -CO-, -COO- oder -OOC- ersetzt sind und/oder gegebenenfalls eine C-C-Einfachbindung durch eine C-C-Doppelbindung ersetzt ist; $X^{11}$ eine einfache Kovalenzbindung, -COO-, -OOC-, $-CH_2CH_2-$, $-OCH_2-$ oder $-CH_2O-$ bezeichnet; einer der Ringe $A^{11}$, $A^{12}$ und $A^{13}$ Pyrimidin-2,5-diyl darstellt, einer der Ringe $A^{11}$, $A^{12}$ und $A^{13}$ unsubstituiertes oder mit Cyano, Halogen oder Niederalkyl substituiertes 1,4-Phenylen darstellt und einer der Ringe $A^{11}$, $A^{12}$ und $A^{13}$ trans-1,4-Cyclohexylen oder unsubstituiertes oder mit Cyano, Halogen oder Niederalkyl substituiertes 1,4-Phenylen darstellt; und $R^{28}$ und $R^{29}$ unabhängig voneinander eine gegebenenfalls halogensubstituierte Alkylgruppe mit bis zu 18 Kohlenstoffatomen bedeuten, worin gegebenenfalls 1 oder 2 nicht benachbarte $CH_2$-Gruppen durch -O-, -CO-, -COO- und/oder -OOC-ersetzt sind; $X^{12}$ eine einfache Kovalenzbindung, -COO-, -OOC-, $-CH_2CH_2-$, $-OCH_2-$ oder $-CH_2O-$ bezeichnet; einer der Ringe $A^{14}$, $A^{15}$ und $A^{16}$ trans-m-Dioxan-2,5-diyl darstellt, und die beiden anderen der Ringe $A^{14}$ , $A^{15}$ und $A^{16}$ unabhängig voneinander unsubstituiertes oder mit Cyano, Halogen oder Niederalkyl substituiertes 1,4-Phenylen darstellen; $R^{30}$ und $R^{31}$ unabhängig voneinander eine gegebenenfalls halogensubstituierte Alkylgruppe mit bis zu 18 Kohlenstoffatomen bedeuten, worin gegebenenfalls 1 oder 2 nicht benachbarte $CH_2$-Gruppen durch -O-, -CO-, -COO- und/oder -OOC-ersetzt sind.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Die optischen Antipoden von chiralen Verbindungen besitzen jeweils die gleichen Phasenumwandlungstemperaturen und absolut gleiche Werte der Verdrillung aber mit entgegengesetzten Vorzeichen. Die zur Charakterisierung der Phasenumwandlungen verwendeten Abkürzungen stehen für folgende Bedeutungen:

| | |
|---|---|
| C | für kristallin |
| S | für smektisch |
| $S_A$, $S_B$, $S_C$ etc. | für smektisch A, B, C etc. |
| $S_C^*$ , $S_F^*$ | für chiral smektische C, F etc. |
| Ch | für cholesterisch |
| N | für nematisch |
| I | für isotrop. |

Beispiel 1

Ein Gemisch von 0,5 g 4-(5-Nonyl-2-pyrimidinyl)phenol, 0,55 g 3-(trans-4-Pentylcyclohexyl)-1-propylbromid, 0.91 g Kaliumcarbonat und 50 ml absolutem Butanon wurde über Nacht unter Rückfluss erhitzt. Anschliessend wurde das abgekühlte Reaktionsgemisch auf Wasser gegossen und dreimal mit je 50 ml Diäthyläther extrahiert. Die vereinigten organischen Phasen wurden mit 500 ml Wasser gewaschen, über Magnesiumsulfat getrocknet, filtriert und eingeengt. Chromatographie des Rückstandes an Keiselgel mit Toluol und Umkristallisation aus Aethanol ergab reines 2-(4-[3-(trans-4-Pentylcyclohexyl)-1-propyloxy]phenyl -5-nonylpyridimin mit Smp. (C-$S_C$) 83 ° C, Umwandlung $S_C$-N 103 ° C, Klp. (N-I) 133 ° C.

In analoger Weise können folgende Verbindungen hergestellt werden:

2-(4-[3-(trans-4-Methylcyclohexyl)-1-propyloxy]phenyl)-5-propylpyrimidin;
2-(4-[3-(trans-4-Aethylcyclohexyl)-1-propyloxy]phenyl)-5-propylpyrimidin;
2-(4-[3-(trans-4-Porpylcyclohexyl)-1-propyloxy]phenyl)-5-propylpyrimidin;
2-(4-[3-(trans-4-Butylcyclohexyl)-1-propyloxy]phenyl)-5-propylpyrimidin;
2-(4-[3-(trans-4-Pentylcyclohexyl)-1-propyloxy]phenyl)-5-propylpyrimidin;
2-(4-[3-(trans-4-Hexylcyclohexyl)-1-propyloxy]phenyl)-5-propylpyrimidin;

2-(4-[3-(trans-4-Heptylcyclohexyl)-1-propyloxy]phenyl)-5-propylpyrimidin;
2-(4-[3-(trans-4-Octylcyclohexyl)-1-propyloxy]phenyl)-5-propylpyrimidin;
2-(4-[3-(trans-4-Nonylcyclohexyl)-1-propyloxy]phenyl)-5-propylpyrimidin;
2-(4-[3-(trans-4-Decylcyclohexyl)-1-propyloxy]phenyl)-5-propylpyrimidin;
2-(4-[3-(trans-4-Methylcyclohexyl)-1-propyloxy]phenyl)-5-butylpyrimidin;
2-(4-[3-(trans-4-Aethylcyclohexyl)-1-propyloxy]phenyl)-5-butylpyrimidin;
2-(4-[3-(trans-4-Propylcyclohexyl)-1-propyloxy]phenyl)-5-butylpyrimidin;
2-(4-[3-(trans-4-Butylcyclohexyl)-1-propyloxy]phenyl)-5-butylpyrimidin;
2-(4-[3-(trans-4-Pentylcyclohexyl)-1-propyloxy]phenyl)-5-butylpyrimidin;
2-(4-[3-(trans-4-Hexylcyclohexyl)-1-propyloxy]phenyl)-5-butylpyrimidin;
2-(4-[3-(trans-4-Heptylcyclohexyl)-1-propyloxy]phenyl)-5-butylpyrimidin;
2-(4-[3-(trans-4-Octylcyclohexyl)-1-propyloxy]phenyl)-5-butylpyrimidin;
2-(4-[3-(trans-4-Nonylcyclohexyl)-1-propyloxy]phenyl)-5-butylpyrimidin;
2-(4-[3-(trans-4-Decylcyclohexyl)-1-propyloxy]phenyl)-5-butylpyrimidin;
2-(4-[3-(trans-4-Methylcyclohexyl)-1-propyloxy]phenyl)-5-pentylpyrimidin;
2-(4-[3-(trans-4-Aethylcyclohexyl)-1-propyloxy]phenyl)-5-pentylpyrimidin;
2-(4-[3-(trans-4-Propylcyclohexyl)-1-propyloxy]phenyl)-5-pentylpyrimidin;
2-(4-[3-(trans-4-Butylcyclohexyl)-1-propyloxy]phenyl)-5-pentylpyrimidin;
2-(4-[3-(trans-4-Pentylcyclohexyl)-1-propyloxy]phenyl)-5-pentylpyrimidin, Smp. (C-N) 82 °C; Klp. (N-I) 139 °C;
2-(4-[3-(trans-4-Hexylcyclohexyl)-1-propyloxy]phenyl)-5-pentylpyrimidin;
2-(4-[3-(trans-4-Heptylcyclohexyl)-1-propyloxy]phenyl)-5-pentylpyrimidin;
2-(4-[3-(trans-4-Octylcyclohexyl)-1-propyloxy]phenyl)-5-pentylpyrimidin;
2-(4-[3-(trans-4-Nonylcyclohexyl)-1-propyloxy]phenyl)-5-pentylpyrimidin;
2-(4-[3-(trans-4-Decylcyclohexyl)-1-propyloxy]phenyl)-5-pentylpyrimidin;
2-(4-[3-(trans-4-Propylcyclohexyl)-1-propyloxy]phenyl)-5-hexylpyrimidin;
2-(4-[3-(trans-4-Butylcyclohexyl)-1-propyloxy]phenyl)-5-hexylpyrimidin;
2-(4-[3-(trans-4-Pentylcyclohexyl)-1-propyloxy]phenyl)-5-hexylpyrimidin, Smp. (C-N) 74 °C, Klp. (N-I) 135 °C;
2-(4-[3-(trans-4-Hexylcyclohexyl)-1-propyloxy]phenyl)-5-hexylpyrimidin;
2-(4-[3-(trans-4-Heptylcyclohexyl)-1-propyloxy]phenyl)-5-hexylpyrimidin;
2-(4-[3-(trans-4-Octylcyclohexyl)-1-propyloxy]phenyl)-5-hexylpyrimidin;
2-(4-[3-(trans-4-Nonylcyclohexyl)-1-propyloxy]phenyl)-5-hexylpyrimidin;
2-(4-[3-trans-4-Decylcyclohexyl)-1-propyloxy]phenyl)-5-hexylpyrimidin;
2-(4-[3-(trans-4-Methylcyclohexyl)-1-propyloxy]phenyl)-5-heptylpyrimidin;
2-(4-[3-(trans-4-Aethylcyclohexyl)-1-propyloxy]phenyl)-5-heptylpyrimidin;
2-(4-[3-(trans-4-Propylcyclohexyl)-1-propyloxy]phenyl)-5-heptylpyrimidin;
2-(4-[3-(trans-4-Butylcyclohexyl)-1-propyloxy]phenyl)-5-heptylpyrimidin;
2-(4-[3-(trans-4-Pentylcyclohexyl)-1-propyloxy]phenyl)-5-heptylpyrimidin, Smp. (C-N) 84 °C, Klp. (N-I) 137 °C;
2-(4-[3-(trans-4-Hexylcyclohexyl)-1-propyloxy]phenyl)-5-heptylpyrimidin;
2-(4-[3-(trans-4-Heptylcyclohexyl)-1-propyloxy]phenyl)-5-heptylpyrimidin;
2-(4-[3-(trans-4-Octylcyclohexyl)-1-propyloxy]phenyl)-5-heptylpyrimidin;
2-(4-[3-(trans-4-Nonylcyclohexyl)-1-propyloxy]phenyl)-5-heptylpyrimidin;
2-(4-[3-(trans-4-Decylcyclohexyl)-1-propyloxy]phenyl)-5-heptylpyrimidin;
2-(4-[3-(trans-4-Methylcyclohexyl)-1-propyloxy]phenyl)-5-octylpyrimidin;
2-(4-[3-(trans-4-Aethylcyclohexyl)-1-propyloxy]phenyl)-5-octylpyrimidin;
2-(4-[3-(trans-4-Porpylcyclohexyl)-1-propyloxy]phenyl)-5-octylpyrimidin;
2-(4-[3-(trans-4-Butylcyclohexyl)-1-propyloxy]phenyl)-5-octylpyrimidin;
2-(4-[3-(trans-4-Pentylcyclohexyl)-1-propyloxy]phenyl)-5-octylpyrimidin, Smp. (C-$S_C$) 79 °C. Umwandlung $S_C$-N 88 °C, Klp. (N-I) 133 °C;
2-(4-[3-(trans-4-Hexylcyclohexyl)-1-propyloxy]phenyl)-5-octylpyrimidin;
2-(4-[3-(trans-4-Heptylcyclohexyl)-1-propyloxy]phenyl)-5-octylpyrimidin;
2-(4-[3-(trans-4-Octylcyclohexyl)-1-propyloxy]phenyl)-5-octylpyrimidin;
2-(4-[3-(trans-4-Nonylcyclohexyl)-1-propyloxy]phenyl)-5-octylpyrimidin;
2-(4-[3-(trans-4-Decylocyclohexyl)-1-propyloxy]phenyl)-5-octylpyrimidin;
2-(4-[3-(trans-4-Methylcyclohexyl)-1-propyloxy]phenyl)-5-nonylpyrimidin;
2-(4-[3-(trans-4-Aethylcyclohexyl)-1-propyloxy]phenyl)-5-nonylpyrimidin;

2-(4-[3-(trans-4-Propylcyclohexyl)-1-propyloxy]phenyl)-5-nonylpyrimidin;
2-(4-[3-(trans-4-Butylcyclohexyl)-1-propyloxy]phenyl)-5-nonylpyrimidin;
2-(4-[3-(trans-4-Hexylcyclohexyl)-1-propyloxy]phenyl)-5-nonylpyrimidin;
2-(4-[3-(trans-4-Heptylcyclohexyl)-1-propyloxy]phenyl)-5-nonylpyrimidin;
2-(4-[3-(trans-4-Octylcyclohexyl)-1-propyloxy]phenyl)-5-nonylpyrimidin;
2-(4-[3-(trans-4-Nonylcyclohexyl)-1-propyloxy]phenyl)-5-nonylpyrimidin;
2-(4-[3-(trans-4-Decylcyclohexyl)-1-propyloxy]phenyl)-5-nonylpyrimidin;
2-(4-[3-(trans-4-Methylcyclohexyl)-1-propyloxy]phenyl)-5-decylpyrimidin;
2-(4-[3-(trans-4-Aethylcyclohexyl)-1-propyloxy]phenyl)-5-decylpyrimidin;
2-(4-[3-(trans-4-Propylcyclohexyl)-1-propyloxy]phenyl)-5-decylpyrimidin;
2-(4-[3-(trans-4-Butylcyclohexyl)-1-propyloxy]phenyl)-5-decylpyrimidin;
2-(4-[3-(trans-4-Pentylcyclohexyl)-1-propyloxy]phenyl)-5-decylpyrimidin, Smp. (C-$S_C$) 69 °C, Umwandlung $S_C$-N110 °C, Klp. (N-I) 130 °C;
2-(4-[3-(trans-4-Hexylcyclohexyl)-1-propyloxy])phenyl)-5-decylpyrimidin;
2-(4-[3-(trans-4-Heptylcyclohexyl)-1-propyloxy])phenyl)-5-decylpyrimidin;
2-(4-[3-(trans-4-Octylcyclohexyl)-1-propyloxy])phenyl)-5-decylpyrimidin;
2-(4-[3-(trans-4-Nonylcyclohexyl)-1-propyloxy])phenyl)-5-decylpyrimidin;
2-(4-[3-(trans-4-Decylcyclohexyl)-1-propyloxy])phenyl)-5-decylpyrimidin;
2-(4-[3-(trans-4-Pentylcyclohexyl)-1-propyloxy]phenyl)-5-[(S)-2-methylbutyl]pyrimidin;
2-(4-[3-(trans-4-Pentylcyclohexyl)-1-propyloxy]phenyl)-5-[(S)-3-methylpentyl]pyrimidin;
2-(4-[3-(trans-4-Pentylcyclohexyl)-1-propyloxy]phenyl)-5-[(S)-4-methylhexyl]pyrimidin;
2-(4-[3-(trans-4-Pentylcyclohexyl)-1-propyloxy]phenyl)-5-[(S)-5-methylheptyl]pyrimidin;
2-(4-[3-(trans-4-Propylcyclohexyl)-1-propyloxy]phenyl)-5-methylpyridin;
2-(4-[3-(trans-4-Propylcyclohexyl)-1-propyloxy]phenyl)-5-äthylpyridin;
2-(4-[3-(trans-4-Propylcyclohexyl)-1-propyloxy]phenyl)-5-propylpyridin;
2-(4-[3-(trans-4-Propylcyclohexyl)-1-propyloxy]phenyl)-5-butylpyridin;
2-(4-[3-(trans-4-Propylcyclohexyl)-1-propyloxy]phenyl)-5-pentylpyridin;
2-(4-[3-(trans-4-Propylcyclohexyl)-1-propyloxy]phenyl)-5-hexylpyridin;
2-(4-[3-(trans-4-Propylcyclohexyl)-1-propyloxy]phenyl)-5-heptylpyridin;
2-(4-[3-(trans-4-Propylcyclohexyl)-1-propyloxy]phenyl)-5-octylpyridin;
2-(4-[3-(trans-4-Propylcyclohexyl)-1-propyloxy]phenyl)-5-nonylpyridin;
2-(4-[3-(trans-4-Propylcyclohexyl)-1-propyloxy]phenyl)-5-decylpyridin;
2-(4-[3-(trans-4-Pentylcyclohexyl)-1-propyloxy]phenyl)-5-methylpyridin;
2-(4-[3-(trans-4-Pentylcyclohexyl)-1-propyloxy]phenyl)-5-äthylpyridin;
2-(4-[3-(trans-4-Pentylcyclohexyl)-1-propyloxy]phenyl)-5-propylpyridin;
2-(4-[3-(trans-4-Pentylcyclohexyl)-1-propyloxy]phenyl)-5-butylpyridin;
2-(4-[3-(trans-4-Pentylcyclohexyl)-1-propyloxy]phenyl)-5-pentylpyridin;
2-(4-[3-(trans-4-Pentylcyclohexyl)-1-propyloxy]phenyl)-5-hexylpyridin;
2-(4-[3-(trans-4-Pentylcyclohexyl)-1-propyloxy]phenyl)-5-heptylpyridin;
2-(4-[3-(trans-4-Pentylcyclohexyl)-1-propyloxy]phenyl)-5-octylpyridin;
2-(4-[3-(trans-4-Pentylcyclohexyl)-1-propyloxy]phenyl)-5-nonylpyridin;
2-(4-[3-(trans-4-Pentylcyclohexyl)-1-propyloxy]phenyl)-5-decylpyridin, Smp. (C-S) 57 °C, Umwandlung S-S 71 °C, Umwandlung S-$S_C$ 122 °C, Umwandlung $S_C$-$S_A$ 136 °C, Klp. ($S_A$-I) 139 °C;
2-(4-[3-(trans-4-Heptylcyclohexyl)-1-propyloxy]phenyl)-5-methylpyridin;
2-(4-[3-(trans-4-Heptylcyclohexyl)-1-propyloxy]phenyl)-5-äthylpyridin;
2-(4-[3-(trans-4-Heptylcyclohexyl)-1-propyloxy]phenyl)-5-propylpyridin;
2-(4-[3-(trans-4-Heptylcyclohexyl)-1-propyloxy]phenyl)-5-butylpyridin;
2-(4-[3-(trans-4-Heptylcyclohexyl)-1-propyloxy]phenyl)-5-pentylpyridin;
2-(4-[3-(trans-4-Heptylcyclohexyl)-1-propyloxy]phenyl)-5-hexylpyridin;
2-(4-[3-(trans-4-Heptylcyclohexyl)-1-propyloxy]phenyl)-5-heptylpyridin;
2-(4-[3-(trans-4-Heptylcyclohexyl)-1-propyloxy]phenyl)-5-octylpyridin;
2-(4-[3-(trans-4-Heptylcyclohexyl)-1-propyloxy]phenyl)-5-nonylpyridin;
2-(4-[3-(trans-4-Heptylcyclohexyl)-1-propyloxy]phenyl)-5-decylpyridin;
2-(4-[3-(trans-4-Pentylcyclohexyl)-1-propyloxy]phenyl)-5-[(S)-2-methylbutyl]pyridin;
2-(4-[3-(trans-4-Pentylcyclohexyl)-1-propyloxy]phenyl)-5-[(S)-3-methylpentyl]pyridin;
2-(4-[3-(trans-4-Pentylcyclohexyl)-1-propyloxy]phenyl)-5-[(S)-4-methylhexyl]pyridin;
2-(4-[3-(trans-4-Pentylcyclohexyl)-1-propyloxy]phenyl)5-[(S)-5-methylheptyl]pyridin;
2-(4-[3-(trans-4-Pentylcyclohexyl)-1-propyloxy]phenyl)-5-[(S)-6-methyloctyl]pyridin;

2-(4-[3-(trans-4-Pentylcyclohexyl)-1-propyloxy]phenyl)-5-[(S)-1-methylheptyloxy]pyridin;
2-(4-[3-(trans-4-Propylcyclohexyl)-1-propyloxy]phenyl)-5-methylpyrazin;
2-(4-[3-(trans-4-Propylcyclohexyl)-1-propyloxy]phenyl)-5-äthylpyrazin;
2-(4-[3-(trans-4-Propylcyclohexyl)-1-propyloxy]phenyl)-5-propylpyrazin;
2-(4-[3-(trans-4-Propylcyclohexyl)-1-propyloxy]phenyl)-5-butylpyrazin;
2-(4-[3-(trans-4-Propylcyclohexyl)-1-propyloxy]phenyl)-5-pentylpyrazin;
2-(4-[3-(trans-4-Propylcyclohexyl)-1-propyloxy]phenyl)-5-hexylpyrazin;
2-(4-[3-(trans-4-Propylcyclohexyl)-1-propyloxy]phenyl)-5-heptylpyrazin;
2-(4-[3-(trans-4-Pentylcyclohexyl)-1-propyloxy]phenyl)-5-methylpyrazin;
2-(4-[3-(trans-4-Pentylcyclohexyl)-1-propyloxy]phenyl)-5-äthylpyrazin;
2-(4-[3-(trans-4-Pentylcyclohexyl)-1-propyloxy]phenyl)-5-propylpyrazin;
2-(4-[3-(trans-4-Pentylcyclohexyl)-1-propyloxy]phenyl)-5-butylpyrazin;
2-(4-[3-(trans-4-Pentylcyclohexyl)-1-propyloxy]phenyl)-5-pentylpyrazin;
2-(4-[3-(trans-4-Pentylcyclohexyl)-1-propyloxy]phenyl)-5-hexylpyrazin;
2-(4-[3-(trans-4-Pentylcyclohexyl)-1-propyloxy]phenyl)-5-heptylpyrazin;
2-(4-[3-(trans-4-Heptylcyclohexyl)-1-propyloxy]phenyl)-5-methylpyrazin;
2-(4-[3-(trans-4-Hetpylcyclohexyl)-1-propyloxy]phenyl)-5-äthylpyrazin;
2-(4-[3-(trans-4-Heptylcyclohexyl)-1-propyloxy]phenyl)-5-propylpyrazin;
2-(4-[3-(trans-4-Heptylcyclohexyl)-1-propyloxy]phenyl)-5-butylpyrazin;
2-(4-[3-(trans-4-Heptylcyclohexyl)-1-propyloxy]phenyl)-5-pentylpyrazin;
2-(4-[3-(trans-4-Heptylcyclohexyl)-1-propyloxy]phenyl)-5-hexylpyrazin;
2-(4-[3-(trans-4-Heptylcyclohexyl)-1-propyloxy]phenyl)-5-heptylpyrazin;
2-Methyl-5-(4-[3-(trans-4-Pentylcyclohexyl)-1-propyloxy]phenyl)-1,3,4-thiadiazol;
2-Aethyl-5-(4-[3-(trans-4-Pentylcyclohexyl)-1-propyloxy]phenyl)-1,3,4-thiadiazol;
2-Propyl-5-(4-[3-(trans-4-Pentylcyclohexyl)-1-propyloxy]phenyl)-1,3,4-thiadiazol;
2-Butyl-5-(4-[3-(trans-4-Pentylcyclohexyl)-1-propyloxy]phenyl)-1,3,4-thiadiazol;
2-Pentyl-5-(4-[3-(trans-4-Pentylcyclohexyl)-1-propyloxy]phenyl)-1,3,4-thiadiazol;
2-Hexyl-5-(4-[3-(trans-4-Pentylcyclohexyl)-1-propyloxy]phenyl)-1,3,4-thiadiazol;
2-Heptyl-5-(4-[3-(trans-4-Pentylcyclohexyl)-1-propyloxy]phenyl)-1,3,4-thiadiazol;
2-Octyl-5-(4-[3-(trans-4-Pentylcyclohexyl)-1-propyloxy]phenyl)-1,3,4-thiadiazol;
2-Nonyl-5-(4-[3-(trans-4-Pentylcyclohexyl)-1-propyloxy]phenyl)-1,3,4-thiadiazol;
2-Decyl-5-(4-[3-(trans-4-Pentylcyclohexyl)-1-propyloxy]phenyl)-1,3,4-thiadiazol;
2-(4-Methoxyphenyl)-5-[3-(trans-4-pentylcyclohexyl)-1propyloxy]-1,3,4-thiadiazol;
2-(4-Aethoxyphenyl)-5-[3-(trans-4-pentylcyclohexyl)-1propyloxy]-1,3,4-thiadiazol;
2-(4-Propyloxyphenyl)-5-[3-(trans-4-pentylcyclohexyl)-1propyloxy]-1,3,4-thiadiazol;
2-(4-Butoxyphenyl)-5-[3-(trans-4-pentylcyclohexyl)-1propyloxy]-1,3,4-thiadiazol;
2-(4-Pentyloxyphenyl)-5-[3-(trans-4-pentylcyclohexyl)-1propyloxy]-1,3,4-thiadiazol;
2-(4-Hexyloxyphenyl)-5-[3-(trans-4-pentylcyclohexyl)-1propyloxy]-1,3,4-thiadiazol;
2-(4-Heptyloxyphenyl)-5-[3-(trans-4-pentylcyclohexyl)-1propyloxy]-1,3,4-thiadiazol;
2-(4-Octyloxyphenyl)-5-[3-(trans-4-pentylcyclohexyl)-1propyloxy]-1,3,4-thiadiazol;
2-(4-Nonyloxyphenyl)-5-[3-(trans-4-pentylcyclohexyl)-1propyloxy]-1,3,4-thiadiazol;
2-(4-Decyloxyphenyl)-5-[3-(trans-4-pentylcyclohexyl)-1propyloxy]-1,3,4-thiadiazol;
1-Methoxy-4-[3-(trans-4-propylcyclohexyl)-1-propyloxy]benzol;
1-Aethoxy-4-[3-(trans-4-propylcyclohexyl)-1-propyloxy]benzol;
1-Propyloxy-4-[3-(trans-4-propylcyclohexyl)-1-propyloxy]benzol;
1-Butyloxy-4-[3-(trans-4-propylcyclohexyl)-1-propyloxy]benzol;
1-Pentyloxy-4-[3-(trans-4-propylcyclohexyl)-1-propyloxy]benzol;
1-Hexyloxy-4-[3-(trans-4-propylcyclohexyl)-1-propyloxy]benzol;
1-Heptyloxy-4-[3-(trans-4-propylcyclohexyl)-1-propyloxy]benzol;
1-Octyloxy-4-[3-(trans-4-propylcyclohexyl)-1-propyloxy]benzol;
1-Nonyloxy-4-[3-(trans-4-propylcyclohexyl)-1-propyloxy]benzol;
1-Decyloxy-4-[3-(trans-4-propylcyclohexyl)-1-propyloxy]benzol;
1-Methoxy-4-[3-(trans-4-pentylcyclohexyl)-1-propyloxy]benzol,Smp. (C-I) 47 °C, Klp. (N-I) 46 °C;
1-Aethoxy-4-[3-(trans-4-pentylcyclohexyl-1-propyloxy]benzol, Smp. (C-N) 50 °C, Klp. (N-I) 57 °C;
1-Propyloxy-4-[3-(trans-4-pentylcyclohexyl)-1-propyloxy]benzol, Smp. (C-I) 51 °C, Klp. (N-I) 48 °C;
1-Butyloxy-4-[3-(trans-4-pentylcyclohexyl)-1-propyloxy]benzol, Smp. (C-I) 59 °C, Umwandlung $S_A$-N 45 °C, Klp. (N-I) 55 °C;
1-Pentyloxy-4-[3-(trans-4-pentylcyclohexyl)-1-propyloxy]benzol, Smp. (C-$S_A$) 42 °C, Umwandlung $S_A$-N

48 °C, Klp. (N-I) 53 °C;

1-Hexyloxy-4-[3-(trans-4-pentylcyclohexyl)-1-propyloxy]benzol, Smp. (C-$S_A$) 44 °C, Umwandlung $S_A$-N 54 °C, Klp. (N-I) 57 °C;

1-Heptyloxy-4-[3-(trans-4-pentylcyclohexyl)-1-propyloxy]benzol, Smp. (C-$S_A$) 51 °C, Klp. ($S_A$-I) 57 °C;

1-Octyloxy-4-[3-(trans-4-pentylcyclohexyl)-1-propyloxy]benzol;

1-Nonyloxy-4-[3-(trans-4-pentylcyclohexyl)-1-propyloxy]benzol;

1-Decyloxy-4-[3-(trans-4-pentylcyclohexyl)-1-propyloxy]benzol;

1-Methoxy-4-[3-(trans-4-heptylcyclohexyl-1-propyloxy]benzol;

1-Aethoxy-4-[3-(trans-4-heptylcyclohexyl)-1-propyloxy]benzol;

1-Propyloxy-4-[3-(trans-4-heptylcyclohexyl-1-propyloxy]benzol;

1-Butyloxy-4-[3-(trans-4-heptylcyclohexyl)-1-propyloxy]benzol;

1-Pentyloxy-4-[3-(trans-4-heptylcyclohexyl)-1-propyloxy]benzol;

1-Hexyloxy-4-[3-(trans-4-heptylcyclohexyl)-1-propyloxy]benzol;

1-Heptyloxy-4-[3-(trans-4-heptylcyclohexyl)-1-propyloxy]benzol;

1-Octyloxy-4-[3-(trans-4-heptylcyclohexyl)-1-propyloxy]benzol;

1-Nonyloxy-4-[3-(trans-4-heptylcyclohexyl)-1-propyloxy]benzol;

1-Decyloxy-4-[3-(trans-4-heptylcyclohexyl)-1-propyloxy]benzol;

1-[(R)-1-Methylheptyloxy]-4-[3-(trans-4-[(S)-3-methylpentyl]cyclohexyl)-1-propyloxy]benzol;

1-Methyl-4-[3-(trans-4-pentylcyclohexyl)-1-propyloxy]benzol;

1-Aethyl-4-[3-(trans-4-pentylcyclohexyl)-1-propyloxy]benzol;

1-Propyl-4-[3-(trans-4-pentylcyclohexyl)-1-propyloxy]benzol;

1-Butyl-4-[3-(trans-4-pentylcyclohexyl)-1-propyloxy]benzol;

1-Pentyl-4-[3-(trans-4-pentylcyclohexyl)-1-propyloxy]benzol;

1-Hexyl-4-[3-(trans-4-pentylcyclohexyl)-1-propyloxy]benzol;

1-Heptyl-4-[3-(trans-4-pentylcyclohexyl)-1-propyloxy]benzol;

1-Octyl-4-[3-trans-4-pentylcyclohexyl)-1-propyloxy]benzol;

1-Nonyl-4-[3-(trans-4-pentylcyclohexyl)-1-propyloxy]benzol;

1-Decyl-4-[3-(trans-4-pentylcyclohexyl)-1-propyloxy]benzol;

4-[3-(trans-4-Methylcyclohexyl)-1-propyloxy]benzonitril;

4-[3-(trans-4-Aethylcyclohexyl)-1-propyloxy]benzonitril;

4-[3-(trans-4-Propylcyclohexyl)-1-propyloxy]benzonitril;

4-[3-(trans-4-Butylcyclohexyl)-1-propyloxy]benzonitril;

4-[3-(trans-4-Pentylcyclohexyl)-1-propyloxy]benzonitril, Smp. (C-I) 62 °C, Klp. (N-I) 54 °C;

4-[3-(trans-4-Hexylcyclohexyl)-propyloxy]benzonitril;

4-[3-(trans-4-Heptylcyclohexyl)-1-propyloxy]benzonitril;

4-[3-(trans-4-Octylcyclohexyl)-1-propyloxy]benzonitril;

4-[3-(trans-4-Nonylcyclohexyl)-1-propyloxy]benzonitril;

4-[3-(trans-4-Decylcyclohexyl)-1-propyloxy]benzonitril;

4-[3-(trans-4-Methylcyclohexyl)-1-propyloxy]-2-fluorbenzonitril;

4-[3-(trans-4-Aethylcyclohexyl)-1-propyloxy]-2-fluorbenzonitril;

4-[3-(trans-4-Propylcyclohexyl)-1-propyloxy]-2-fluorbenzonitril;

4-[3-(trans-4-Butylcyclohexyl)-1-propyloxy]-2-fluorbenzonitril;

4-[3-(trans-4-Pentylcyclohexyl)-1-propyloxy]-2-fluorbenzonitril, Smp. (C-I) 59 °C, Klp. (N-I) 29 °C;

4-[3-(trans-4-Hexylcyclohexyl)-1-propyloxy]-2-fluorbenzonitril;

4-[3-(trans-4-Heptylcyclohexyl)-1-propyloxy]-2-fluorbenzonitril;

4-[3-(trans-4-Octylcyclohexyl)-1-propyloxy]-2-fluorbenzonitril;

4-[3-(trans-4-Nonylcyclohexyl)-1-propyloxy]-2-fluorbenzonitril;

4-[3-(trans-4-Decylcyclohexyl)-1-propyloxy]-2-fluorbenzonitril;

4-[3-(trans-4-Methylcyclohexyl)-1-propyloxy]-3-fluorbenzonitril;

4-[3-(trans-4-Aethylcyclohexyl)-1-propyloxy]-3-fluorbenzonitril;

4-[3-(trans-4-Propylcyclohexyl)-1-propyloxy]-3-fluorbenzonitril;

4-[3-(trans-4-Butylcyclohexyl)-1-propyloxy]-3-fluorbenzonitril;

4-[3-(trans-4-Pentylcyclohexyl)-1-propyloxy]-3-fluorbenzonitril, Smp. (C-N) 45 °C, Klp. (N-I) 46 °C;

4-[3-(trans-4-Hexylcyclohexyl)-1-propyloxy]-3-fluorbenzonitril;

4-[3-(trans-4-Heptylcyclohexyl)-1-propyloxy]-3-fluorbenzonitril;

4-[3-(trans-4-Octylcyclohexyl)-1-propyloxy]-3-fluorbenzonitril;

4-[3-(trans-4-Nonylcyclohexyl)-1-propyloxy]-3-fluorbenzonitril;

4-[3-(trans-4-Decylcyclohexyl)-1-propyloxy]-3-fluorbenzonitril;

1-[3-(trans-4-Methylcyclohexyl)-1-propyloxy]-4-fluorbenzol;
1-[3-(trans-4-Aethylcyclohexyl)-1-propyloxy]-4-fluorbenzol;
1-[3-(trans-4-Propylcyclohexyl)-1-propyloxy]-4-fluorbenzol;
1-[3-(trans-4-Butylcyclohexyl)-1-propyloxy]-4-fluorbenzol;
1-[3-(trans-4-Pentylcyclohexyl)-1-propyloxy]-4-fluorbenzol; Smp. (C-I) 17 °C, Klp. (N-I) 11 °C;
1-[3-(trans-4-Hexylcyclohexyl)-1-propyloxy]-4-fluorbenzol;
1-[3-(trans-4-Heptylcyclohexyl)-1-propyloxy]-4-fluorbenzol;
1-[3-(trans-4-Pentylcyclohexyl)-1-propyloxy]-4-chlorbenzol, Smp. (C-I) 37 °C, Klp. (N-I) 33 °C;
1-[3-(trans-4-Pentylcyclohexyl)-1-propyloxy]-4-brombenzol, Smp. (C-I) 48 °C, Klp. (N-I) 37 °C;
1-[3-(trans-4-Pentylcyclohexyl)-1-propyloxy]-4-jodbenzol, Smp. (C-I) 54 °C, Klp. (N-I) 34 °C;
1-[3-(trans-4-Methylcyclohexyl)-1-propyloxy]-3,4-difluorbenzol;
1-[3-(trans-4-Aethylcyclohexyl)-1-propyloxy]-3,4-difluorbenzol;
1-[3-(trans-4-Propylcyclohexyl)-1-propyloxy]-3,4-difluorbenzol;
1-[3-(trans-4-Butylcyclohexyl)-1-propyloxy]-3,4-difluorbenzol;
1-[3-(trans-4-Pentylcyclohexyl)-1-propyloxy]-3,4-dilfuorbenzol,Smp. (C-I) 4 °C;
1-[3-(trans-4-Hexylcylohexyl)-1-propyloxy]-3,4-difluorbenzol;
1-[3-(trans-4-Heptylcyclohexyl)-1-propyloxy]-3,4-difluorbenzol;
1-[3-(trans-4-Octylcyclohexyl)-1-propyloxy]-3,4-difluorbenzol;
1-[3-(trans-4-[trans-4-Propylcyclohexyl]cyclohexyl)-1-propyloxy]-4-fluorbenzol;
1-[3-(trans-4-[trans-4-Pentylcyclohexyl]cyclohexyl)-1-propyloxy]-4-fluorbenzol, Smp. (C-$S_B$) 69 °C, Umwandlung $S_B$-N 95 °C, Klp. (N-I) 127 °C;
1-[3-(trans-4-[trans-4-Propylcyclohexyl]cyclohexyl)-1-propyloxy]-3,4-difluorbenzol; Smp. (C-N) 79 °C, Klp. (N-I) 107 °C;
1-[3-(trans-4-[trans-4-Pentylcyclohexyl]cyclohexyl)-1-propyloxy]-3,4-difluorbenzol, Smp. (C-$S_B$) 57 °C. Umwandlung $S_B$-N 81 °C, Klp. (N-I) 112 °C;
4-[3-(trans-4-[trans-4-Propylcyclohexyl]cyclohexyl)-1-propyloxy]-3-fluorbenzonitril;
4-[3-(trans-4-trans-4-Pentylcyclohexyl]cyclohexyl)-1-propyloxy]-3-fluorbenzonitril, Smp. (C-N) 98 °C, Klp. (N-I) 143 °C;
4-[3-(trans-4-[trans-4-Propylcyclohexyl]cyclohexyl)-1-propyloxy]benzonitril;
4-[3-(trans-4-[trans-4-Pentylcyclohexyl]cyclohexyl)-1-propyloxy]benzonitril, Smp. (C-N) 93 °C, Klp. (N-I) 159 °C;
1-[3-(trans-4-[trans-4-Propylcyclohexyl]cyclohexyl)-1-propyloxy]-4-äthoxy-2,3-difluorbenzol, Smp. (C-N) 59 °C, Klp. (N-I) 136 °C;
1-[3-(trans-4-[trans-4-Pentylcyclohexyl]cyclohexyl)-1-propyloxy]-4-äthoxy-2,3-difluorbenzol, Smp. (C-$S_A$) 49 °C, Umwandlung $S_A$-N 100 °C, Klp. (N-I) 137 °C;
4-[3-(trans-4-Methylcyclohexyl)-1-propyloxy]-phenylisothiocyanat;
4-[3-(trans-4-Aethylcyclohexyl)-1-propyloxy]-phenylisothiocyanat;
4-[3-(trans-4-Propylcyclohexyl)-1-propyloxy]-phenylisothiocyanat;
4-[3-(trans-4-Butylcyclohexyl)-1-propyloxy]-phenylisothiocyanat;
4-[3-(trans-4-Pentylcyclohexyl)-1-propyloxy]-phenylisothiocyanat;
4-[3-(trans-4-Hexylcyclohexyl)-1-propyloxy]-phenylisothiocyanat;
4-[3-(trans-4-Heptylcyclohexyl)-1-propyloxy]-phenylisothiocyanat;
4-[3-(trans-4-Octylcyclohexyl)-1-propyloxy]-phenylisothiocyanat;
2,3-Dicyano-1-propyl-4-[3-(trans-4-propylcyclohexyl)-1-propyloxy]benzol;
2,3-Dicyano-1-pentyl-4-[3-(trans-4-propylcyclohexyl)-1-propyloxy]benzol;
2,3-Dicyano-1-heptyl-4-[3-(trans-4-propylcyclohexyl)-1-propyloxy]benzol;
2,3-Dicyano-1-propyl-4-[3-(trans-4-pentylcyclohexyl)-1-propyloxy]benzol;
2,3-Dicyano-1-pentyl-4-[3-(trans-4-pentylcyclohexyl)-1-propyloxy]benzol, Smp. (C-I) 123 °C;
2,3-Dicyano-1-heptyl-4-[3-(trans-4-pentylcyclohexyl)-1-propyloxy]benzol;
2,3-Dicyano-1-propyl-4-[3-(trans-4-heptylcyclohexyl)-1-propyloxy]benzol;
2,3-Dicyano-1-pentyl-4-[3-(trans-4-heptylcyclohexyl)-1-propyloxy]benzol;
2,3-Dicyano-1-heptyl-4-[3-(trans-4-heptylcyclohexyl)-1-propyloxy]benzol;
2,3-Difluor-1-äthoxy-4-[3-(trans-4-propylcyclohexyl)-1-propyloxy]benzol;
2,3-Difluor-1-äthoxy-4-[3-(trans-4-pentylcyclohexyl)-1-propyloxy]benzol;
2,3-Dicyano-1-[3-(trans-4-propylcyclohexyl)-1-propyloxy]-4-[2-(trans-4-pentylcyclohexyl)äthyl]benzol;
2,3-Dicyano-1-[3-(trans-4-pentylcyclohexyl)-1-propyloxy]-4-[2-(trans-4-pentylcyclohexyl)äthyl]benzol, Smp. (C-I) 198 °C;
2,3-Dicyano-1-[3-(trans-4-heptylcyclohexyl)-1-propyloxy]-4-[2-(trans-4-pentylcyclohexyl)äthyl]benzol;

4-[3-(trans-4-Methylcyclohexyl)-1-propyloxy]-4′-cyanobiphenyl;
4-[3-(trans-4-Aethylcyclohexyl)-1-propyloxy]-4′-cyanobiphenyl;
4-[3-(trans-4-Propylcyclohexyl)-1-propyloxy]-4′-cyanobiphenyl;
4-[3-(trans-4-Butylcyclohexyl)-1-propyloxy]-4′-cyanobiphenyl;
4-[3-(trans-4-Pentylcyclohexyl)-1-propyloxy]-4′-cyanobiphenyl, Smp. (C-$S_A$) 83 °C, Umwandlung $S_A$-N 149 °C, Klp. (N-I) 167 °C;
4-[3-(trans-4-Hexylcyclohexyl)-1-propyloxy]-4′-cyanobiphenyl;
4-[3-(trans-4-Heptylcyclohexyl)-1-propyloxy]-4′-cyanobiphenyl;
4-[3-(trans-4-Octylcyclohexyl)-1-propyloxy]-4′-cyanobiphenyl;
4-[3-(trans-4-Methylcyclohexyl)-1-propyloxy]-4′-fluorbiphenyl;
4-[3-(trans-4-Aethylcyclohexyl)-1-propyloxy]-4′-fluorbiphenyl;
4-[3-(trans-4-Propylcyclohexyl)-1-propyloxy]-4′-fluorbiphenyl;
4-[3-(trans-4-Butylcyclohexyl)-1-propyloxy]-4′-fluorbiphenyl;
4-[3-(trans-4-Pentylcyclohexyl)-1-propyloxy]-4′-fluorbiphenyl;
4-[3-(trans-4-Hexylcyclohexyl)-1-propyloxy]-4′-fluorbiphenyl;
4-[3-(trans-4-Heptylcyclohexyl)-1-propyloxy]-4′-fluorbiphenyl;
4-[3-(trans-4-Octylcyclohexyl)-1-propyloxy]-4′-fluorbiphenyl;
(R)-α-[(4′-[3-(trans-4-Pentylcyclohexyl)-1-propyloxy]-4-biphenylyl)oxy]propionsäure-äthylester; Smp. (C-$S_A$) 79 °C, Umwandlung $S_A$-Ch 80 °C, Klp. (Ch-I) 81 °C;
1,4-Di-[3-(trans-4-methylcyclohexyl)-1-propyloxy]benzol;
1,4-Di-[3-(trans-4-äthylcylohexyl)-1-propyloxy]benzol;
1,4-Di-[3-(trans-4-propylcyclohexyl)-1-propyloxy]benzol;
1,4-Di-[3-(trans-4-butylcyclohexyl)-1-propyloxy]benzol;
1,4-Di-[3-(trans-4-pentylcyclohexyl)-1-propyloxy]benzol, Smp. (C-$S_A$) 108 °C, Umwandlung $S_A$-N 112 °C, N-I 115 °C;
1,4-Di-[3-(trans-4-hexylcyclohexyl)-1-propyloxy]benzol;
1,4-Di-[3-(trans-4-heptylcyclohexyl)-1-propyloxy]benzol;
1,4-Di-[3-(trans-4-octylcyclohexyl)-1-propyloxy]benzol;
1,4-Di-[3-(trans-4-nonylcyclohexyl)-1-propyloxy]benzol;
1,4-Di-[3-(trans-4-decylcyclohexyl)-1-propyloxy]benzol;
1-[3-(trans-4-Pentylcyclohexyl)-1-propyloxy]-4-[3-(trans-4-propylcyclohexyl)-1-propyloxy]benzol;
4,4′-Di-[3-(trans-4-methylcyclohexyl)-1-propyloxy]biphenyl;
4,4′-Di-[3-(trans-4-äthylcyclohexyl)-1-propyloxy]biphenyl;
4,4′-Di-[3-(trans-4-propylcyclohexyl)-1-propyloxy]biphenyl;
4,4′-Di-[3-(trans-4-butylcyclohexyl)-1-propyloxy]biphenyl;
4,4′-Di-[3-(trans-4-pentylcyclohexyl)-1-propyloxy]biphenyl, Smp. (C-S) 67 °C, Umwandlung S-$S_A$ 182 °C, Klp. ($S_A$-I) 202 °C;
4,4′-Di-[3-(trans-4-hexylcyclohexyl)-1-propyloxy]biphenyl;
4,4′-Di-[3-(trans-4-heptylcyclohexyl)-1-propyloxy]biphenyl;
4,4′-Di-[3-(trans-4-octylcyclohexyl)-1-propyloxy]biphenyl;
4,4′-Di-[3-(trans-4-nonylcyclohexyl)-1-propyloxy]biphenyl;
4,4′-Di-[3-(trans-4-Decylcyclohexyl)-1-propyloxy]biphenyl;
4-[3-(trans-4-Pentylcyclohexyl)-1-propyloxy]-4′-[3-(trans-4-propylcyclohexyl)-1-propyloxy]biphenyl;
1-[3-(trans-4-Methylcyclohexyl)-1-propyloxy]-4-[2-(trans-4-methylcyclohexyl)äthyl]benzol;
1-[3-(trans-4-Aethylcyclohexyl)-1-propyloxy]-4-[2-(trans-4-methylcyclohexyl)äthyl]benzol;
1-[3-(trans-4-Propylcyclohexyl)-1-propyloxy]-4-[2-(trans-4-methylcyclohexyl)äthyl]benzol;
1-[3-(trans-4-Butylcyclohexyl)-1-propyloxy]-4-[2-(trans-4-methylcyclohexyl)äthyl]benzol;
1-[3-(trans-4-Pentylcyclohexyl)-1-propyloxy]-4-[2-(trans-4-methylcyclohexyl)äthyl]benzol;
1-[3-(trans-4-Hexylcyclohexyl)-1-propyloxy]-4-[2-(trans-4-methylcyclohexyl)äthyl]benzol;
1-[3-(trans-4-Heptylcyclohexyl)-1-propyloxy]-4-[2-(trans-4-methylcyclohexyl)äthyl]benzol;
1-[3-(trans-4-Methylcyclohexyl)-1-propyloxy]-4-[2-(trans-4-äthylcyclohexyl)äthyl]benzol;
1-[3-(trans-4-Aethylcyclohexyl)-1-propyloxy]-4-[2-(trans-4-äthylcyclohexyl)äthyl]benzol;
1-[3-(trans-4-Propylcyclohexyl)-1-propyloxy]-4-[2-(trans-4-äthylcyclohexyl)äthyl]benzol;
1-[3-(trans-4-Butylcyclohexyl)-1-propyloxy]-4-[2-(trans-4-äthylcyclohexyl)äthyl]benzol;
1-[3-(trans-4-Pentylcyclohexyl)-1-propyloxy]-4-[2-(trans-4-äthylcyclohexyl)äthyl]benzol;
1-[3-(trans-4-Hexylcyclohexyl)-1-propyloxy]-4-[2-(trans-4-äthylcyclohexyl)äthyl]benzol;
1-[3-(trans-4-Heptylcyclohexyl)-1-propyloxy]-4-[2-(trans-4-äthylcyclohexyl)äthyl]benzol;
1-[3-(trans-4-Methylcyclohexyl)-1-propyloxy]-4-[2-(trans-4-propylcyclohexyl)äthyl]benzol;

1-[3-(trans-4-Aethylcyclohexyl)-1-propyloxy]-4-[2-(trans-4-propylcyclohexyl)äthyl]benzol;
1-[3-(trans-4-Propylcyclohexyl)-1-propyloxy]-4-[2-(trans-4-propylcyclohexyl)äthyl]benzol;
1-[3-(trans-4-Butylcyclohexyl)-1-propyloxy]-4-[2-(trans-4-propylcyclohexyl)äthyl]benzol;
1-[3-(trans-4-Pentylcyclohexyl)-1-propyloxy]-4-[2-(trans-4-propylcyclohexyl)äthyl]benzol;
1-[3-(trans-4-Hexylcyclohexyl)-1-propyloxy]-4-[2-(trans-4-propylcyclohexyl)äthyl]benzol;
1-[3-(trans-4-Heptylcyclohexyl)-1-propyloxy]-4-[2-(trans-4-propylcyclohexyl)äthyl]benzol;
1-[3-(trans-4-Methylcyclohexyl)-1-propyloxy]-4-[2-(trans-4-butylcyclohexyl)äthyl]benzol;
1-[3-(trans-4-Aethylcyclohexyl)-1-propyloxy]-4-[2-(trans-4-butylcyclohexyl)äthyl]benzol;
1-[3-(trans-4-Propylcyclohexyl)-1-propyloxy]-4-[2-(trans-4-butylcyclohexyl)äthyl]benzol;
1-[3-(trans-4-Butylcyclohexyl)-1-propyloxy]-4-[2-(trans-4-butylcyclohexyl)äthyl]benzol;
1-[3-(trans-4-Pentylcyclohexyl)-1-propyloxy]-4-[2-trans-4-butylcyclohexyl)äthyl]benzol;
1-[3-(trans-4-Hexylcyclohexyl)-1-propyloxy]-4-[2-(trans-4-butylcyclohexyl)äthyl]benzol;
1-[3-(trans-4-Heptylcyclohexyl)-1-propyloxy]-4-[2-(trans-4-butylcyclohexyl)äthyl]benzol;
1-[3-(trans-4-Methylcyclohexyl)-1-propyloxy]-4-[2-(trans-4-pentylcyclohexyl)äthyl]benzol;
1-[3-(trans-4-Aethylcyclohexyl)-1-propyloxy]-4-[2-(trans-4-pentylcyclohexyl)äthyl]benzol;
1-[3-(trans-4-Propylcyclohexyl)-1-propyloxy]-4-[2-(trans-4-pentylcyclohexyl)äthyl]benzol;
1-[3-(trans-4-Butylcyclohexyl)-1-propyloxy]-4-[2-(trans-4-pentylcyclohexyl)äthyl]benzol;
1-[3-(trans-4-Pentylcyclohexyl)-1-propyloxy]-4-[2-(trans-4-pentylcyclohexyl)äthyl]benzol, Smp. (C-$S_B$) 86 °C, Umwandlung $S_B$-N 109 °C, Klp. (N-I) 117 °C;
1-[3-(trans-4-Hexylcyclohexyl)-1-propyloxy]-4-[2-(trans-4-pentylcyclohexyl)äthyl]benzol;
1-[3-(trans-4-Heptylcyclohexyl)-1-propyloxy]-4-[2-(trans-4-pentylcyclohexyl)äthyl]benzol;
1-[3-(trans-4-Methylcyclohexyl)-1-propyloxy]-4-[2-(trans-4-hexylcyclohexyl)äthyl]benzol;
1-[3-(trans-4-Aethylcyclohexyl)-1-propyloxy]-4-[2-(trans-4-hexylcyclohexyl)äthyl]benzol;
1-[3-(trans-4-Propylcyclohexyl)-1-propyloxy]-4-[2-(trans-4-hexylcyclohexyl)äthyl]benzol;
1-[3-(trans-4-Butylcyclohexyl)-1-propyloxy]-4-[2-(trans-4-hexylcyclohexyl)äthyl]benzol;
1-[3-(trans-4-Pentylcyclohexyl)-1-propyloxy]-4-[2-(trans-4-hexylcyclohexyl)äthyl]benzol;
1-[3-(trans-4-Hexylcyclohexyl)-1-propyloxy]-4-[2-(trans-4-hexylcyclohexyl)äthyl]benzol;
1-[3-(trans-4-Heptylcyclohexyl)-1-propyloxy]-4-[2-(trans-4-hexylcyclohexyl)äthyl]benzol;
1-[3-(trans-4-Methylcyclohexyl)-1-propyloxy]-4-[2-(trans-4-heptylcyclohexyl)äthyl]benzol;
1-[3-(trans-4-Aethylcyclohexyl)-1-propyloxy]-4-[2-(trans-4-heptylcyclohexyl)äthyl]benzol;
1-[3-(trans-4-Propylcyclohexyl)-1-propyloxy]-4-[2-(trans-4-heptylcyclohexyl)äthyl]benzol;
1-[3-(trans-4-Butylcyclohexyl)-1-propyloxy]-4-[2-(trans-4-heptylcyclohexyl)äthyl]benzol;
1-[3-(trans-4-Pentylcyclohexyl)-1-propyloxy]-4-[2-(trans-4-heptylcyclohexyl)äthyl]benzol;
1-[3-(trans-4-Hexylcyclohexyl)-1-propyloxy]-4-[2-(trans-4-heptylcyclohexyl)äthyl]benzol;
1-[3-(trans-4-Heptylcyclohexyl)-1-propyloxy]-4-[2-(trans-4-heptylcyclohexyl)äthyl]benzol;
4-[3-(trans-4-Propylcyclohexyl)-1-propyloxy]-4′-[2-(trans-4-propylcyclohexyl)äthyl]biphenyl;
4-[3-(trans-4-Pentylcyclohexyl)-1-propyloxy]-4′-[2-(trans-4-propylcyclohexyl)äthyl]biphenyl;
4-[3-(trans-4-Pentylcyclohexyl)-1-propyloxy]-4′-[2-(trans-4-pentylcyclohexyl)äthyl]biphenyl;
1-[3-(trans-4-Methylcyclohexyl)-1-propyloxy]-4-[(trans-4-methylcyclohexyl)methoxy]benzol;
1-[3-(trans-4-Aethylcyclohexyl)-1-propyloxy]-4-[(trans-4-methylcyclohexyl)methoxy]benzol;
1-[3-(trans-4-Propylcyclohexyl)-1-propyloxy]-4-[(trans-4-methylcyclohexyl)methoxy]benzol;
1-[3-(trans-4-Butylcyclohexyl)-1-propyloxy]-4-[(trans-4-methylcyclohexyl)methoxy]benzol;
1-[3-(trans-4-Pentylcyclohexyl)-1-propyloxy]-4-[(trans-4-methylcyclohexyl)methoxy]benzol;
1-[3-(trans-4-Hexylcyclohexyl)-1-propyloxy]-4-[(trans-4-methylcyclohexyl)methoxy]benzol;
1-[3-(trans-4-Heptylcyclohexyl)-1-propyloxy]-4-[(trans-4-methylcyclohexyl)methoxy]benzol;
1-[3-(trans-4-Methylcyclohexyl)-1-propyloxy]-4-[(trans-4-äthylcyclohexyl)methoxy]benzol;
1-[3-(trans-4-Aethylcyclohexyl)-1-propyloxy]-4-[(trans-4-äthylcyclohexyl)methoxy]benzol;
1-[3-(trans-4-Propylcyclohexyl)-1-propyloxy]-4-[(trans-4-äthylcyclohexyl)methoxy]benzol;
1-[3-(trans-4-Butylcyclohexyl)-1-propyloxy]-4-[(trans-4-äthylcyclohexyl)methoxy]benzol;
1-[3-(trans-4-Methylcyclohexyl)-1-propyloxy]-4-[(trans-4-propylcyclohexyl)methoxy]benzol;
1-[3-(trans-4-Aethylcyclohexyl)-1-propyloxy]-4-[(trans-4-propylcyclohexyl)methoxy]benzol;
1-[3-(trans-4-Propylcyclohexyl)-1-propyloxy]-4-[(trans-4-propylcyclohexyl)methoxy]benzol;
1-[3-(trans-4-Butylcyclohexyl)-1-propyloxy]-4-[(trans-4-propylcyclohexyl)methoxy]benzol;
1-[3-(trans-4-Pentylcyclohexyl)-1-propyloxy]-4-[(trans-4-propylcyclohexyl)methoxy]benzol;
1-[3-(trans-4-Hexylcyclohexyl)-1-propyloxy]-4-[(trans-4-propylcyclohexyl)methoxy]benzol;
1-[3-(trans-4-Heptylcyclohexyl)-1-propyloxy]-4-[(trans-4-propylcyclohexyl)methoxy]benzol;
1-[3-(trans-4-Methylcyclohexyl)-1-propyloxy]-4-[(trans-4-butylcyclohexyl)methoxy]benzol;
1-[3-(trans-4-Aethylcyclohexyl)-1-propyloxy]-4-[(trans-4-butylcyclohexyl)methoxy]benzol;

1-[3-(trans-4-Propylcyclohexyl)-1-propyloxy]-4-[(trans-4-butylcyclohexyl)methoxy]benzol;
1-[3-(trans-4-Butylcyclohexyl)-1-propyloxy]-4-[(trans-4-butylcyclohexyl)methoxy]benzol;
1-[3-(trans-4-Methylcyclohexyl)-1-propyloxy]-4-[(trans-4-pentylcyclohexyl)methoxy]benzol;
1-[3-(trans-4-Aethylcyclohexyl)-1-propyloxy]-4-[(trans-4-pentylcyclohexyl)methoxy]benzol;
1-[3-(trans-4-Propylcyclohexyl)-1-propyloxy]-4-[(trans-4-pentylcyclohexyl)methoxy]benzol;
1-[3-(trans-4-Butylcyclohexyl)-1-propyloxy]-4-[(trans-4-pentylcyclohexyl)methoxy]benzol;
1-[3-(trans-4-Pentylcyclohexyl)-1-propyloxy]-4-[(trans-4-pentylcyclohexyl)methoxy]benzol, Smp. (C-$S_A$) 104 °C, Umwandlung $S_A$-N 116 °C, Klp. (N-I) 124 °C;
1-[3-(trans-4-Hexylcyclohexyl)-1-propyloxy]-4-[(trans-4-pentylcyclohexyl)methoxy]benzol;
1-[3-(trans-4-Heptylcyclohexyl)-1-propyloxy]-4-[(trans-4-pentylcyclohexyl)methoxy]benzol;
1-[3-(trans-4-Methylcyclohexyl)-1-propyloxy]-4-[(trans-4-hexylcyclohexyl)methoxy]benzol;
1-[3-(trans-4-Aethylcyclohexyl)-1-propyloxy]-4-[(trans-4-hexylcyclohexyl)methoxy]benzol;
1-[3-(trans-4-Propylcyclohexyl)-1-propyloxy]-4-[(trans-4-hexylcyclohexyl)methoxy]benzol;
1-[3-(trans-4-Butylcyclohexyl)-1-propyloxy]-4-[(trans-4-hexylcyclohexyl)methoxy]benzol;
1-[3-(trans-4-Pentylcyclohexyl)-1-propyloxy]-4-[(trans-4-hexylcyclohexyl)methoxy]benzol;
1-[3-(trans-4-Hexylcyclohexyl)-1-propyloxy]-4-[(trans-4-hexylcyclohexyl)methoxy]benzol;
1-[3-(trans-4-Heptylcyclohexyl)-1-propyloxy]-4-[(trans-4-hexylcyclohexyl)methoxy]benzol;
1-[3-(trans-4-Methylcyclohexyl)-1-propyloxy]-4-[(trans-4-heptylcyclohexyl)methoxy]benzol;
1-[3-(trans-4-Aethylcyclohexyl)-1-propyloxy]-4-[(trans-4-heptylcyclohexyl)methoxy]benzol;
1-[3-(trans-4-Propylcyclohexyl)-1-propyloxy]-4-[(trans-4-heptylcyclohexyl)methoxy]benzol;
1-[3-(trans-4-Butylcyclohexyl)-1-propyloxy]-4-[(trans-4-heptylcyclohexyl)methoxy]benzol;
1-[3-(trans-4-Pentylcyclohexyl)-1-propyloxy]-4-[(trans-4-heptylcyclohexyl)methoxy]benzol;
1-[3-(trans-4-Hexylcyclohexyl)-1-propyloxy]-4-[(trans-4-heptylcyclohexyl)methoxy]benzol;
1-[3-(trans-4-Heptylcyclohexyl)-1-propyloxy]-4-[(trans-4-heptylcyclohexyl)methoxy]benzol;
1-[3-(trans-4-Methylcyclohexyl)-1-propyloxy]-4-(trans-4-methylcyclohexyl)benzol;
1-[3-(trans-4-Aethylcyclohexyl)-1-propyloxy]-4-(trans-4-methylcyclohexyl)benzol;
1-[3-(trans-4-Propylcyclohexyl)-1-propyloxy]-4-(trans-4-methylcyclohexyl)benzol;
1-[3-(trans-4-Butylcyclohexyl)-1-propyloxy]-4-(trans-4-methylcyclohexyl)benzol;
1-[3-(trans-4-Pentylcyclohexyl)-1-propyloxy]-4-(trans-4-methylcyclohexyl)benzol;
1-[3-(trans-4-Hexylcyclohexyl)-1-propyloxy]-4-(trans-4-methylcyclohexyl)benzol;
1-[3-(trans-4-Heptylcyclohexyl)-1-propyloxy]-4-(trans-4-methylcyclohexyl)benzol;
1-[3-(trans-4-Methylcyclohexyl)-1-propyloxy]-4-(trans-4-äthylcylohexyl)benzol;
1-[3-(trans-4-Aethylcyclohexyl)-1-propyloxy]-4-(trans-4-äthylcyclohexyl)benzol;
1-[3-(trans-4-Propylcyclohexyl)-1-propyloxy]-4-(trans-4-äthylcyclohexyl)benzol;
1-[3-(trans-4-Butylcyclohexyl)-1-propyloxy]-4-(trans-4-äthylcyclohexyl)benzol;
1-[3-(trans-4-Pentylcyclohexyl)-1-propyloxy]-4-(trans-4-äthylcyclohexyl)benzol;
1-[3-(trans-4-Hexylcyclohexyl)-1-propyloxy]-4-(trans-4-äthylcyclohexyl)benzol;
1-[3-(trans-4-Heptylcyclohexyl)-1-propyloxy]-4-(trans-4-äthylcyclohexyl)benzol;
1-[3-(trans-4-Methylcyclohexyl)-1-propyloxy]-4-(trans-4-propylcyclohexyl)benzol;
1-[3-(trans-4-Aethylcyclohexyl)-1-propyloxy]-4-(trans-4-propylcyclohexyl)benzol;
1-[3-(trans-4-Propylcyclohexyl)-1-propyloxy]-4-(trans-4-propylcyclohexyl)benzol;
1-[3-(trans-4-Butylcyclohexyl)-1-propyloxy]-4-(trans-4-propylcyclohexyl)benzol;
1-[3-(trans-4-Pentylcyclohexyl)-1-propyloxy]-4-(trans-4-propylcyclohexyl)benzol;
1-[3-(trans-4-Hexylcyclohexyl)-1-propyloxy]-4-(trans-4-propylcyclohexyl)benzol;
1-[3-(trans-4-Heptylcyclohexyl)-1-propyloxy]-4-(trans-4-propylcyclohexyl)benzol;
1-[3-(trans-4-Methylcyclohexyl)-1-propyloxy]-4-(trans-4-butylcyclohexyl)benzol;
1-[3-(trans-4-Aethylcyclohexyl)-1-propyloxy]-4-(trans-4-butylcyclohexyl)benzol;
1-[3-(trans-4-Propylcyclohexyl)-1-propyloxy]-4-(trans-4-butylcyclohexyl)benzol;
1-[3-(trans-4-Butylcyclohexyl)-1-propyloxy]-4-(trans-4-butylcyclohexyl)benzol;
1-[3-(trans-4-Pentylcyclohexyl)-1-propyloxy]-4-(trans-4-butylcyclohexyl)benzol;
1-[3-(trans-4-Hexylcyclohexyl)-1-propyloxy]-4-(trans-4-butylcyclohexyl)benzol;
1-[3-(trans-4-Heptylcyclohexyl)-1-propyloxy]-4-(trans-4-butylcyclohexyl)benzol;
1-[3-(trans-4-Methylcyclohexyl)-1-propyloxy]-4-(trans-4-pentylcyclohexyl)benzol;
1-[3-(trans-4-Aethylcyclohexyl)-1-propyloxy]-4-(trans-4-pentylcyclohexyl)benzol;
1-[3-(trans-4-Propylcyclohexyl)-1-propyloxy]-4-(trans-4-pentylcyclohexyl)benzol;
1-[3-(trans-4-Butylcyclohexyl)-1-propyloxy]-4-(trans-4-pentylcyclohexyl)benzol;
1-[3-(trans-4-Pentylcyclohexyl)-1-propyloxy]-4-(trans-4-pentylcyclohexyl)benzol, Smp. (C-$S_B$) 67 °C, Umwandlung $S_B$-N 113 °C, Klp. (N-I) 127 °C;

1-[3-(trans-4-Methylcyclohexyl)-1-propyloxy]-4-(trans-4-hexylcyclohexyl)benzol;
1-[3-(trans-4-Aethylcyclohexyl)-1-propyloxy]-4-(trans-4-hexylcyclohexyl)benzol;
1-[3-(trans-4-Propylcyclohexyl)-1-propyloxy]-4-(trans-4-hexylcyclohexyl)benzol;
1-[3-(trans-4-Butylcyclohexyl)-1-propyloxy]-4-(trans-4-hexylcyclohexyl)benzol;
1-[3-(trans-4-Pentylcyclohexyl)-1-propyloxy]-4-(trans-4-hexylcyclohexyl)benzol;
1-[3-(trans-4-Hexylcyclohexyl)-1-propyloxy]-4-(trans-4-hexylcyclohexyl)benzol;
1-[3-(trans-4-Heptylcyclohexyl)-1-propyloxy]-4-(trans-4-hexylcyclohexyl)benzol;
1-[3-(trans-4-Methylcyclohexyl)-1-propyloxy]-4-(trans-4-heptylcyclohexyl)benzol;
1-[3-(trans-4-Aethylcyclohexyl)-1-propyloxy]-4-(trans-4-heptylcyclohexyl)benzol;
1-[3-(trans-4-Propylcyclohexyl)-1-propyloxy]-4-(trans-4-heptylcyclohexyl)benzol;
1-[3-(trans-4-Butylcyclohexyl)-1-propyloxy]-4-(trans-4-heptylcyclohexyl)benzol;
1-[3-(trans-4-Pentylcyclohexyl)-1-propyloxy]-4-(trans-4-heptylcyclohexyl)benzol;
1-[3-(trans-4-Hexylcyclohexyl)-1-propyloxy]-4-(trans-4-heptylcyclohexyl)benzol;
1-[3-(trans-4-Heptylcyclohexyl)-1-propyloxy]-4-(trans-4-heptylcyclohexyl)benzol;
4-[3-(trans-4-Propylcyclohexyl)-1-propyloxy]-4′-(trans-4-propylcyclohexyl)biphenyl;
4-[3-(trans-4-Pentylcyclohexyl)-1-propyloxy]-4′-(trans-4-propylcyclohexyl)biphenyl;
4-[3-(trans-4-Pentylcyclohexyl)-1-propyloxy]-4′-(trans-4-pentylcyclohexyl)biphenyl;
(4aαH, 8aβH)-Decahydro-2α-(4-[3-(trans-4-methylcyclohexyl)-1-propyloxy]phenyl)-6β-pentylnaphthalin;
(4aαH, 8aβH)-Decahydro-2α-(4-[3-(trans-4-äthylcyclohexyl)-1-propyloxy]phenyl)-6β-pentylnaphthalin;
(4aαH, 8aβH)-Decahydro-2α-(4-[3-(trans-4-propylcyclohexyl)-1-propyloxy]phenyl)-6β-pentylnaphthalin;
(4aαH, 8aβH)-Decahydro-2α-(4-[3-(trans-4-butylcyclohexyl)-1-propyloxy]phenyl)-6β-pentylnaphthalin;
(4aαH, 8aβH)-Decahydro-2α-(4-[3-(trans-4-pentylcyclohexyl)-1-propyloxy]phenyl)-6β-pentylnaphtalin, Smp. (C-$S_A$) 95 °C, Umwandlung $S_A$-N 121 °C, Klp. (N-I) 159 °C;
(4aαH, 8aβH)-Decahydro-2α-(4-[3-(trans-4-hexylcyclohexyl)-1-propyloxy]phenyl)-6β-pentylnaphthalin;
(4aαH, 8aβH)-Decahydro-2α(4-[3-(trans-4-heptylcyclohexyl)-1-propyloxy]phenyl)-6β-pentylnaphthalin;
(4aαH, 8aβH)-Decahydro-2α-(4-[3-(trans-4-octylcyclohexyl)-1-propyloxy]phenyl)-6β-pentylnaphthalin;
1-Methyl-4-(4-[3-(trans-4-Pentylcyclohexyl)-1-propyloxy]phenyl)bicyclo[2,2,2]octan;
1-Aethyl-4-(4-[3-(trans-4-Pentylcyclohexyl)-1-propyloxy]phenyl)bicyclo[2,2,2]octan;
1-Propyl-4-(4-[3-(trans-4-Pentylcyclohexyl)-1-propyloxy]phenyl)bicyclo[2,2,2]octan;
1-Butyl-4-(4-[3-(trans-4-Pentylcyclohexyl)-1-propyloxy]phenyl)bicyclo[2,2,2]octan;
1-Pentyl-4-(4[3-(trans-4-Pentylcyclohexyl)-1-propyloxy]phenyl)bicyclo[2,2,2]octan;
1-Hexyl-4-(4-[3-(trans-4-Pentylcyclohexyl)-1-propyloxy]phenyl)bicyclo[2,2,2]octan;
1-Heptyl-4-(4-[3-(trans-4-Pentylcyclohexyl)-1-propyloxy]phenyl)bicyclo[2,2,2,]octan;
1-Octyl-4-(4-[3-(trans-4-Pentylcyclohexyl)-1-propyloxy]phenyl)bicyclo[2,2,2]octan;
1-Nonyl-4-(4-[3-(trans-4-Pentylcyclohexyl)-1-propyloxy]phenyl)bicyclo[2,2,2,]octan;
1-Decyl-4-(4-[3-(trans-4-Pentylcyclohexyl)-1-propyloxy]phenyl)bicyclo[2,2,2]octan;
2-(4′-[3-(trans-4-Pentylcyclohexyl)-1-propyloxy]-4-biphenylyl)-5-methylpyrimidin;
2-(4′-[3-(trans-4-Pentylcyclohexyl)-1-propyloxy]-4-biphenylyl)-5-äthylpyrimidin;
2-(4′-[3-(trans-4-Pentylcyclohexyl)-1-propyloxy]-4-biphenylyl)-5-propylpyrimidin;
2-(4′-[3-(trans-4-Pentylcyclohexyl)-1-propyloxy]-4-biphenylyl)-5-butylpyrimidin;
2-(4′-[3-(trans-4-Pentylcyclohexyl)-1-propyloxy]-4-biphenylyl)-5-pentylpyrimidin;
2-(4′-[3-(trans-4-Pentylcyclohexyl)-1-propyloxy]-4-biphenylyl)-5-hexylpyrimidin;
2-(4′-[3-(trans-4-Pentylcyclohexyl)-1-propyloxy]-4-biphenylyl)-5-heptylpyrimidin;
2-(4′-[3-(trans-4-Pentylcyclohexyl)-1-propyloxy]-4-biphenylyl)-5-octylpyrimidin;
2-(4′-[3-(trans-4-Pentylcyclohexyl)-1-propyloxy]-4-biphenylyl)-5-nonylpyrimidin;
2-(4′-[3-(trans-4-Pentylcyclohexyl)-1-propyloxy]-4-biphenylyl)-5-decylpyrimidin, Smp. (C-S) 115 °C, Umwandlung S-$S_C$ 142 °C, Umwandlung $S_C$-N 205 °C, Klp. (N-I) 215 °C.

Beispiel 2

1,9 g 4-Heptyloxybenzoesäure, 2,5 g 4-[3-(trans-4-Pentylcyclohexyl)-1-propyloxy]phenol, 0.1 g 4-(Dimethylamino)pyridin wurden in 50 ml Dichlormethan gelöst und die Lösung innert 10 Minuten unter Rühren protionenweise mit 2,0 g N,N′-Dicyclohexylcarbodiimid versetzt. Das Gemisch wurde über Nacht bie Raumtemperatur gerührt und dann filtriert. Das Filtrat wurde mit Dichlormethan verdünnt, zweimal mit je 50 ml gesättigter Natriumcarbonat-Lösung und dann mit Wasser gewaschen, über Magnesiumsulfat getrocknet, filtriert und eingeengt. Das erhaltene Rohprodukt wurde an Keiselgel mit Toluol chromatographisch gereinigt. Der erhaltene 4-Heptyloxybenzoesäure-4-[3-(trans-4-pentylcyclohexyl)-1-propyloxy]phenylester wurde aus Aethanol umkristallisiert; Smp. (C-$S_C$) 74 °C, Umwandlung S-$S_C$ 67 °C (monotrop), Umwandlung

$S_C$-N 86 ° C, Klp. (N-I) 148 ° C;

Das als Ausgangsmaterial verwendete 4-[3-(trans-4-Pentylcyclohexyl)-1-propyloxy]phenol wurde wie folgt hergestellt;

Ein Gemisch von 5,0 g 3-(trans-4-Pentylcyclohexyl)-1-propylbromid, 10,0 g Hydrochinon, 10,0 g wasserfreiem Kaliumcarbonat und 250 ml absolutem Butanon wurde über Nacht unter Rückfluss erhitzt. Anschliessend wurde das abgekühlte Reaktionsgemisch auf Wasser gegossen und dreimal mit je 100 ml Dichlormethan extrahiert. Die vereinigten organischen Phasen wurden mit 500 ml Wasser gewaschen, über Magnesiumsulfat getrocknet, filtriert und eingeengt. Chromatographie des Rückstandes an Kieselgel mit Toluol/Aethylacetat (Vol. 4:1) ergab 2,9 g 4-[3-(trans-4-Pentylcyclohexyl)-1-propyloxy]phenol; Smp. 100-101 ° C.

In analoger Weise können folgende Verbindungen hergestellt werden:

4-Methoxybenzoesäure-4-[3-(trans-4-propylcyclohexyl)-1-propyloxy]phenylester;
4-Aethoxybenzoesäure-4-[3-(trans-4-propylcyclohexyl)-1-propyloxy]phenylester;
4-Propyloxybenzoesäure-4-[3-(trans-4-propylcyclohexyl)-1-propyloxy]phenylester;
4-Butyloxybenzoesäure-4-[3-(trans-4-propylcyclohexyl)-1-propyloxy]phenylester;
4-Pentyloxybenzoesäure-4-[3-(trans-4-propylcyclohexyl)-1-propyloxy]phenylester;
4-Hexyloxybenzoesäure-4-[3-(trans-4-propylcyclohexyl)-1-propyloxy]phenylester;
4-Heptyloxybenzoesäure-4-[3-(trans-4-propylcyclohexyl)-1-propyloxy]phenylester;
4-Octyloxybenzoesäure-4-[3-(trans-4-propylcyclohexyl)-1-propyloxy]phenylester;
4-Nonyloxybenzoesäure-4-[3-(trans-4-propylcyclohexyl)-1-propyloxy]phenylester;
4-Decyloxybenzoesäure-4-[3-(trans-4-propylcyclohexyl)-1-propyloxy]phenylester;
4-Undecycloxybenzoesäure-4-[3-(trans-4-propylcyclohexyl)-1-propyloxy]phenylester;
4-Dodecyloxybenzoesäure-4-[3-(trans-4-propylcyclohexyl)-1-propyloxy]phenylester;
4-Methoxybenzoesäure-4-[3-(trans-4-pentylcyclohexyl)-1-propyloxy]phenylester;
4-Aethoxybenzoesäure-4-[3-(trans-4-pentylcyclohexyl)-1-propyloxy]phenylester;
4-Propyloxybenzoesäure-4-[3-(trans-4-pentylcyclohexyl)-1-propyloxy]phenylester;
4-Butyloxybenzoesäure-4-[3-(trans-4-pentylcyclohexyl)-1-propyloxy]phenylester;
4-Pentyloxybenzoesäure-4-[3-(trans-4-pentylcyclohexyl)-1-propyloxy]phenylester;
4-Hexyloxybenzoesäure-4-[3-(trans-4-pentylcyclohexyl)-1-propyloxy]phenylester;

4-Octyloxybenzoesäure-4-[3-(trans-4-pentylcyclohexyl)-1-propyloxy]phenylester, smp. (C-$S_C$) 68 ° C, Umwandlung S-$S_C$ 67 ° C (monotrop), Umwandlung $S_C$-N 99 ° C, Klp. (N-I) 147 ° C.

4-Nonyloxybenzoesäure-4-[3-(trans-4-pentylcyclohexyl)-1-propyloxy]phenylester, Smp. (C-$S_C$) 77 ° C, Umwandlung S-$S_C$ 68 ° C (monotrop), Umwandlung $S_C$-N 109 ° C, Klp. (N-I) 144 ° C;

4-Decyloxybenzoesäure-4-[3-(trans-4-pentylcyclohexyl)-1-propyloxy]phenylester, Smp. (C-S) 64 ° C, Umwandlung S-$S_C$ 70 ° C, Umwandlung $S_C$-N 116 ° C, Klp. (N-I) 143 ° C;

4-Undecyloxybenzoesäure-4-[3-(trans-4-pentylcyclohexyl)-1-propyloxy]phenylester, Smp. (C-$S_C$) 75 ° C, Umwandlung S-$S_C$ 71 ° C (monotrop), Umwandlung $S_C$-N 121 ° C, Klp. (N-I) 141 ° C;

4-Dodecyloxybenzoesäure-4-[3-(trans-4-pentylcyclohexyl)-1-propyloxy]phenylester, Smp. (C-$S_C$) 58 ° C, Umwandlung S-$S_C$ 75 ° C (monotrop), Umwandlung $S_C$-N 125 ° C, Klp. (N-I) 140 ° C;

4-Methoxybenzoesäure-4-[3-(trans-4-heptylcyclohexyl)-1-propyloxy]phenylester;
4-Aethoxybenzoesäure-4-[3-(trans-4-heptylcyclohexyl)-1-propyloxy]phenylester;
4-Propyloxybenzoesäure-4-[3-(trans-4-heptylcyclohexyl)-1-propyloxy]phenylester;
4-Butyloxybenzoesäure-4-[3-(trans-4-heptylcyclohexyl)-1-propyloxy]phenylester;
4-Pentyloxybenzoesäure-4-[3-(trans-4-heptylcyclohexyl)-1-propyloxy]phenylester;
4-Hexyloxybenzoesäure-4-[3-(trans-4-heptylcyclohexyl)-1-propyloxy]phenylester;
4-Heptyloxybenzoesäure-4-[3-(trans-4-heptylcyclohexyl)-1-propyloxy]phenylester;
4-Octyloxybenzoesäure-4-[3-(trans-4-heptylcyclohexyl)-1-propyloxy]phenylester;
4-Nonyloxybenzoesäure-4-[3-(trans-4-heptylcyclohexyl)-1-propyloxy]phenylester;
4-Decyloxybenzoesäure-4-[3-(trans-4-heptylcyclohexyl)-1-propyloxy]phenylester;
4-Undecyloxybenzoesäure-4-[3-(trans-4-heptylcyclohexyl)-1-propyloxy]phenylester;
4-Dodecyloxybenzoesäure-4-[3-(trans-4-heptylcyclohexyl)-1-propyloxy]phenylester;
4-(Allyloxy)benzoesäure-4-[3-trans-4-propylcyclohexyl)-1-propyloxy]phenylester;
4-(3-Butenyl)oxybenzoesäure-4-[3-(trans-4-propylcyclohexyl)-1-propyloxy]phenylester;
4-(4-Pentenyl)oxybenzoesäure-4-[3-(trans-4-propylcyclohexyl)-1-propyloxy]phenylester;
4-(5-Hexenyl)oxybenzoesäure-4-[3-(trans-4-propylcyclohexyl)-1-propyloxy]phenylester;
4-(6-Heptenyl)oxybenzoesäure-4-[3-(trans-4-propylcyclohexyl)-1-propyloxy]phenylester;
4-(7-Octenyl)oxybenzoesäure-4-[3-(trans-4-propylcyclohexyl)-1-propyloxy]phenylester;
4-(8-Nonenyl)oxybenzoesäure-4-[3-(trans-4-propylcyclohexyl)-1-propyloxy]phenylester;
4-(9-Decenyl)oxybenzoesäure-4-[3-(trans-4-propylcyclohexyl)-1-propyloxy]phenylester;

4-(10-Undecyl)oxybenzoesäure-4-[3-(trans-4-propylcyclohexyl)-1-propyloxy]phenylester;
4-(11-Dodecyl)oxybenzoesäure-4-[3-(trans-4-propylcyclohexyl)-1-propyloxy]phenylester;
4-Allyloxybenzoesäure-4-[3-(trans-4-pentylcyclohexyl)-1-propyloxy]phenylester;
4-(3-Pentenyl)oxybenzoesäure-4-[3-(trans-4-pentylcyclohexyl)-1-propyloxy]phenylester;
4-(4-Butenyl)oxybenzoesäure-4-[3-(trans-4-pentylcyclohexyl)-1-propyloxy]phenylester;
4-(5-Hexenyl)oxybenzoesäure-4-[3-(trans-4-pentylcyclohexyl)-1-propyloxy]phenylester;
4-(6-Heptenyl)oxybenzoesäure-4-[3-(trans-4-pentylcyclohexyl)-1-propyloxy]phenylester, Smp. (C-$S_C$) 64 °C, Umwandlung S-$S_C$ 52 °C (monotrop), Umwandlung $S_C$-N 73 °C, Klp. (N-I) 147 °C;
4-(7-Octenyl)oxybenzoesäure-4-[3-(trans-4-pentylcyclohexyl)-1-propyloxy]phenylester, Smp. (C-$S_C$) 75 °C, Umwandlung S-$S_C$ 57 °C (monotrop), Umwandlung $S_C$-N 86 °C, Klp. (N-I) 142 °C;
4-(8-Nonenyl)oxybenzoesäure-4-[3-(trans-4-pentylcyclohexyl)-1-propyloxy]phenylester, Smp. (C-$S_C$) 61 °C, Umwandlung S-$S_C$ 56 °C (monotrop), Umwandlung $S_C$-N 99 °C, Klp. (N-I) 144 °C;
4-(9-Decenyl)oxybenzoesäure-4-[3-(trans-4-pentylcyclohexyl)-1-propyloxy]phenylester, Smp. (C-$S_C$) 64 °C, Umwandlung S-$S_C$ 58 °C (monotrop), Umwandlung $S_C$-N 105 °C, Klp. (N-I) 138 °C;
4-(10-Undecenyl)oxybenzoesäure-4-[3-(trans-4-pentylcyclohexyl)-1-propyloxy]phenylester, Smp. (C-$S_C$) 71 °C, Umwandlung S-$S_C$ 61 °C (monotrop), Umwandlung $S_C$-N 114 °C, Klp. (N-I) 139 °C;
4-(11-Dodecenyl)oxybenzoesäure-4-[3-(trans-4-pentylcyclohexyl)-1-propyloxy]phenylester, Smp. (C-S) 55 °C, Umwandlung S-$S_C$ 65 °C, Umwandlung $S_C$-N 117 °C , Klp. (N-I) 136 °C;
4-Allyloxybenzoesäure-4-[3-(trans-4-heptylcyclohexyl)-1-propyloxy]phenylester;
4-(3-Butenyl)oxybenzoesäure-4-[3-(trans-4-heptylcyclohexyl)-1-propyloxy]phenylester;
4-(4-Pentenyl)oxybenzoesäure-4-[3-(trans-4-heptylcyclohexyl)-1-propyloxy]phenylester;
4-(5-Hexenyl)oxybenzoesäure-4-[3-(trans-4-heptylcyclohexyl)-1-propyloxy]phenylester;
4-(6-Heptenyl)oxybenzoesäure-4-[3-(trans-4-heptylcyclohexyl)-1-propyloxy]phenylester;
4-(7-Octenyl)oxybenzoesäure-4-[3-(trans-4-heptylcyclohexyl)-1-propyloxy]phenylester;
4-(8-Nonenyl)oxybenzoesäure-4-[3-(trans-4-heptylcyclohexyl)-1-propyloxy]phenylester;
4-(9-Decenyl)oxybenzoesäure-4-[3-(trans-4-heptylcyclohexyl)-1-propyloxy]phenylester;
4-Methoxy-3-fluorbenzoesäure-4-[3-(trans-4-propylcyclohexyl)-1-propyloxy]phenylester;
4-Aethoxy-3-fluorbenzoesäure-4-[3-(trans-4-propylcyclohexyl)-1-propyloxy]phenylester;
4-Propyloxy-3-fluorbenzoesäure-4-[3-(trans-4-propylcyclohexyl)-1-propyloxy]phenylester;
4-Butyloxy-3-fluorbenzoesäure-4-[3-(trans-4-propylcyclohexyl)-1-propyloxy]phenylester;
4-Pentyloxy-3-fluorbenzoesäure-4-[3-(trans-4-propylcyclohexyl)-1-propyloxy]phenylester;
4-Hexyloxy-3-fluorbenzoesäure-4-[3-(trans-4-propylcyclohexyl)-1-propyloxy]phenylester;
4-Heptyloxy-3-fluorbenzoesäure-4-[3-(trans-4-propylcyclohexyl)-1-propyloxy]phenylester;
4-Octyloxy-3-fluorbenzoesäure-4-[3-(trans-4-propylcyclohexyl)-1-propyloxy]phenylester;
4-Nonyloxy-3-fluorbenzoesäure-4-[3-(trans-4-propylcyclohexyl)-1-propyloxy]phenylester;
4-Decyloxy-3-fluorbenzoesäure-4-[3-(trans-4-propylcyclohexyl)-1-propyloxy]phenylester;
4-Undecyloxy-3-fluorbenzoesäure-4-[3-(trans-4-propylcyclohexyl)-1-propyloxy]phenylester;
4-Dodecyloxy-3-fluorbenzoesäure-4-[3-(trans-4-propylcyclohexyl)-1-propyloxy]phenylester;
4-Methoxy-3-fluorbenzoesäure-4-[3-(trans-4-pentylcyclohexyl)-1-propyloxy]phenylester;
4-Aethoxy-3-fluorbenzoesäure-4-[3-(trans-4-pentylcyclohexyl)-1-propyloxy]phenylester;
4-Propyloxy-3-fluorbenzoesäure-4-[3-(trans-4-pentylcyclohexyl)-1-propyloxy]phenylester;
4-Butyloxy-3-fluorbenzoesäure-4-[3-(trans-4-pentylcyclohexyl)-1-propyloxy]phenylester;
4-Pentyloxy-3-fluorbenzoesäure-4-[3-(trans-4-pentylcyclohexyl)-1-propyloxy]phenylester;
4-Hexyloxy-3-fluorbenzoesäure-4-[3-(trans-4-pentylcyclohexyl)-1-propyloxy]phenylester;
4-Octyloxy-3-fluorbenzoesäure-4-[3-(trans-4-pentylcyclohexyl)-1-propyloxy]phenylester;
4-Nonyloxy-3-fluorbenzoesäure-4-[3-(trans-4-pentylcyclohexyl)-1-propyloxy]phenylester;
4-Decyloxy-3-fluorbenzoesäure-4-[3-(trans-4-pentylcyclohexyl)-1-propyloxy]phenylester;
4-Undecyloxy-3-fluorbenzoesäure-4-[3-(trans-4-pentylcyclohexyl)-1-propyloxy]phenylester;
4-Dodecyloxy-3-fluorbenzoesäure-4-[3-(trans-4-pentylcyclohexyl)-1-propyloxy]phenylester, Smp. (C-$S_C$) 69 °C, Umwandlung S-$S_C$ <40 °C, Umwandlung $S_C$-N 120 °C, Klp. (N-I) 129 °C;
4-Methoxy-3-fluorbenzoesäure-4-[3-(trans-4-heptylcyclohexyl)-1-propyloxy]phenylester;
4-Aethoxy-3-fluorbenzoesäure-4-[3-(trans-4-heptylcyclohexyl)-1-propyloxy]phenylester;
4-Propyloxy-3-fluorbenzoesäure-4-[3-(trans-4-heptylcyclohexyl)-1-propyloxy]phenylester;
4-Butyloxy-3-fluorbenzoesäure-4-[3-(trans-4-heptylcyclohexyl)-1-propyloxy]phenylester;
4-Pentyloxy-3-fluorbenzoesäure-4-[3-(trans-4-heptylcyclohexyl)-1-propyloxy]phenylester;
4-Hexyloxy-3-fluorbenzoesäure-4-[3-(trans-4-heptylcyclohexyl)-1-propyloxy]phenylester;
4-Heptyloxy-3-fluorbenzoesäure-4-[3-(trans-4-heptylcyclohexyl)-1-propyloxy]phenylester;
4-Octyloxy-3-fluorbenzoesäure-4-[3-(trans-4-heptylcyclohexyl)-1-propyloxy]phenylester;

4-Nonyloxy-3-fluorbenzoesäure-4-[3-(trans-4-heptylcyclohexyl)-1-propyloxy]phenylester;
4-Decyloxy-3-fluorbenzoesäure-4-[3-(trans-4-heptylcyclohexyl)-1-propyloxy]phenylester;
4-Undecyloxy-3-fluorbenzoesäure-4-[3-(trans-4-heptylcyclohexyl)-1-propyloxy]phenylester;
4-Dodecyloxy-3-fluorbenzoesäure-4-[3-(trans-4-heptylcyclohexyl)-1-propyloxy]phenylester;
4-(11-Dodecenyl)oxy-3-fluorbenzoesäure-4-[3-(trans-4-pentylcyclohexyl)-1-propyloxy]phenylester;
4-Heptyloxy-2,3-difluorbenzoesäure-4-[3-(trans-4-pentylcyclohexyl)-1-propyloxy]phenylester, Smp. (C-$S_C$) 79 °C, Umwandlung $S_C$-N 94 °C, Klp. (N-I) 139 °C;
4-Octyloxy-2,3-difluorbenzoesäure-4-[3-(trans-4-pentylcyclohexyl)-1-propyloxy]phenylester, Smp. (C-$S_C$) 70 °C, Umwandlung $S_C$-N 103 °C, Klp. (N-I) 138 °C;
4-Nonyloxy-2,3-difluorbenzoesäure-4-[3-(trans-4-pentylcyclohexyl)-1-propyloxy]phenylester, Smp. (C-$S_C$) 73 °C, Umwandlung $S_C$-N 110 °C, Klp. (N-I) 136 °C;
4-Decyloxy-2,3-difluorbenzoesäure-4-[3-(trans-4-pentylcyclohexyl)-1-propyloxy]phenylester, Smp. (C-$S_C$) 68 °C, Umwandlung $S_C$-N 115 °C, Klp. (N-I) 135 °C;
4-Undecyloxy-2,3-difluorbenzoesäure-4-[3-(trans-4-pentylcyclohexyl)-1-propyloxy]phenylester, Smp. (C-$S_C$) 68 °C, Umwandlung $S_C$-N 118 °C, Klp. (N-I) 133 °C;
4-Dodecyloxy-2,3-difluorbenzosäure-4-[3-(trans-4-pentylcyclohexyl)-1-propyloxy]phenylester, Smp. (C-$S_C$) 66 °C, Umwandlung $S_C$-N 121 °C, Klp. (N-I) 133 °C;
4-Dodecyloxy-2-chlorbenzoesäure-4-[3-(trans-4-pentylcyclohexyl)-1-propyloxy]phenylester;
4-Dodecyloxy-2-brombenzoesäure-4-[3-(trans-4-pentylcyclohexyl)-1-propyloxy]phenylester;
4-Dodecyloxy-2-cyanobenzoesäure-4-[3-(trans-4-pentylcyclohexyl)-1-propyloxy]phenylester;
4-Dodecyloxy-2-chlorbenzoesäure-4-[3-(trans-4-pentylcyclohexyl)-1-propyloxy]phenylester, Smp (C-$S_C$) 60 °C, Umwandlung $S_C$-N 105 °C, Klp. (N-I) 122 °C;
4-Dodecyloxy-3-brombenzoesäure-4-[3-(trans-4-pentylcyclohexyl)-1-propyloxy]phenylester, Smp (C-$S_C$) 70 °C, Umwandlung $S_C$-N 92 °C, Klp. (N-I) 112 °C;
4-Dodecyloxy-3-cyanobenzoesäure-4-[3-(trans-4-pentylcyclohexyl)-1-propyloxy]phenylester, Smp (C-I) 103 °C, Umwandlung $S_C$-N 97 °C, Klp. (N-I) 100 °C;
(S)-4-(1-Methylheptyl)oxybenzoesäure-4-[3-(trans-4-pentylcyclohexyl)-1-propyloxy]phenylester;
(S)-4-(1-Methylheptyl)oxy-3-fluorbenzoesäure-4-[3-(trans-4-pentylcyclohexyl)-1-propyloxy]phenylester;
(S)-4-(1-Methylheptyl)oxy-3-chlorbenzoesäure-4-[3-(trans-4-pentylcyclohexyl)-1-propyloxy]phenylester;
(S)-4-(1-Methylheptyl)oxy-3-brombenzoesäure-4-[3-(trans-4-pentylcyclohexyl)-1-propyloxy]phenylester;
(S)-4-(1-Methylheptyl)oxy-3-cyanobenzoesäure-4-[3-(trans-4-pentylcyclohexyl)-1-propyloxy]phenylester;
trans-4-Methylcyclohexancarbonsäure-4-[3-(trans-4-propylcyclohexyl)-1-propyloxy]phenylester;
trans-4-Aethylcyclohexancarbonsäure-4-[3-(trans-4-propylcyclohexyl)-1-propyloxy]phenylester;
trans-4-Propylcyclohexancarbonsäure-4-[3-(trans-4-propylcyclohexyl)-1-propyloxy]phenylester;
trans-4-Butylcyclohexancarbonsäure-4-[3-(trans-4-propylcyclohexyl)-1-propyloxy]phenylester;
trans-4-Pentylcyclohexancarbonsäure-4-[3-(trans-4-propylcyclohexyl)-1-propyloxy]phenylester;
trans-4-Hexylcyclohexancarbonsäure-4-[3-(trans-4-propylcyclohexyl)-1-propyloxy]phenylester;
trans-4-Heptylcyclohexancarbonsäure-4-[3-(trans-4-propylcyclohexyl)-1-propyloxy]phenylester;
trans-4-Methylcyclohexancarbonsäure-4-[3-(trans-4-pentylcyclohexyl)-1-propyloxy]phenylester;
trans-4-Aethylcyclohexancarbonsäure-4-[3-(trans-4-pentylcyclohexyl)-1-propyloxy]phenylester;
trans-4-Propylcyclohexancarbonsäure-4-[3-(trans-4-pentylcyclohexyl)-1-propyloxy]phenylester;
trans-4-Butylcyclohexancarbonsäure-4-[3-(trans-4-pentylcyclohexyl)-1-propyloxy]phenylester;
trans-4-Pentylcyclohexancarbonsäure-4-[3-(trans-4-pentylcyclohexyl)-1-propyloxy]phenylester, Smp. (C-S) 88 °C, Umwandlung S-$S_B$ 102 °C, Umwandlung $S_B$-$S_A$ 114 °C, Umwandlung $S_A$-N 127 °C, Klp. (N-I) 150 °C;
trans-4-Hexylcyclohexancarbonsäure-4-[3-(trans-4-pentylcyclohexyl)-1-propyloxy]phenylester;
trans-4-Heptylcyclohexancarbonsäure-4-[3-(trans-4-pentylcyclohexyl)-1-propyloxy]phenylester;
trans-4-Methylcyclohexancarbonsäure-4-[3-(trans-4-heptylcyclohexyl)-1-propyloxy]phenylester;
trans-4-Aethylcyclohexancarbonsäure-4-[3-(trans-4-heptylcyclohexyl)-1-propyloxy]phenylester;
trans-4-Propylcyclohexancarbonsäure-4-[3-(trans-4-heptylcyclohexyl)-1-propyloxy]phenylester;
trans-4-Butylcyclohexancarbonsäure-4-[3-(trans-4-heptylcyclohexyl)-1-propyloxy]phenylester;
trans-4-Pentylcyclohexancarbonsäure-4-[3-(trans-4-heptylcyclohexyl)-1-propyloxy]phenylester;
trans-4-Hexylcyclohexancarbonsäure-4-[3-(trans-4-heptylcyclohexyl)-1-propyloxy]phenylester;
trans-4-Heptylcyclohexancarbonsäure-4-[3-(trans-4-heptylcyclohexyl)-1-propyloxy]phenylester;
trans-4-Vinylcyclohexancarbonsäure-4-[3-(trans-4-pentylcyclohexyl)-1-propyloxy]phenylester;
trans-4-(2-Propenyl)cyclohexancarbonsäure-4-[3-(trans-4-pentylcyclohexyl)-1-propyloxy]phenylester;
trans-4-(3-Butenyl)cyclohexancarbonsäure-4-[3-(trans-4-pentylcyclohexyl)-1-propyloxy]phenylester;
trans-4-(4-Pentenyl)cyclohexancarbonsäure-4-[3-(trans-4-pentylcyclohexyl)-1-propyloxy]phenylester;

4-(trans-4-Pentylcyclohexyl)benzoesäure-4-[3-(trans-4-pentylcyclohexyl)-1-propyloxy]phenylester, Smp. (C-$S_C$) 132 °C, Umwandlung $S_C$-$S_A$ 142 °C, Umwandlung $S_A$-N 161 °C, Klp. (N-I) 232 °C;

4-(4-Pentylbicyclo[2,2,2]oct-1-yl)benzoesäure-4-[3-(trans-4-pentylcyclohexyl)-1-propyloxy]phenylester, Smp. (C-$S_A$) 171 °C, Umwandlung $S_A$-N 194 °C, Klp. (N-I) 260 °C;

4-(5-Pentyl-2-pyrimidinyl)benzoesäure-4-[3-(trans-4-pentylcyclohexyl)-1-propyloxy]phenylester, Smp. (C-$S_C$) 97 °C, Umwandlung $S_C$-N 80 °C, Klp. (N-I) 229 °C;

4-(5-Hexyl-2-pyrimidinyl)benzoesäure-4-[3-(trans-4-pentylcyclohexyl)-1-propyloxy]phenylester;

4-(5-Heptyl-2-pyrimidinyl)benzoesäure-4-[3-(trans-4-pentylcyclohexyl)-1-propyloxy]phenylester, Smp. (C-N) 118 °C, Klp. (N-I) 219 °C;

4-(5-Octyl-2-pyrimidinyl)benzoesäure-4-[3-(trans-4-pentylcyclohexyl)-1-propyloxy]phenylester;

4-(5-Nonyl-2-pyrimidinyl)benzoesäure-4-[3-(trans-4-pentylcyclohexyl)-1-propyloxy]phenylester;

4-(5-Decyl-2-pyrimidinyl)benzoesäure-4-[3-(trans-4-pentylcyclohexyl)-1-propyloxy]phenylester, Smp. (C-$S_C$) 108 °C, Umwandlung $S_C$-N 126 °C, Klp. (N-I) 205 °C;

trans-4-(trans-4-Pentylcyclohexyl)cyclohexancarbonsäure-4-[3-(trans-4-pentylcyclohexyl)-1-propyloxy]-phenylester, Smp. (C-$S_C$) 77 °C, Umwandlung $S_B$-$S_A$ 189 °C, Umwandlung $S_A$-N 220 °C, Klp. (N-I) 239 °C;

4-[2-(trans-4-Pentylcyclohexyl)äthyl]benzoesäure-4-[3-(trans-4-pentylcyclohexyl)-1-propyloxy]-phenylester, Smp. (C-$S_B$) 85 °C, Umwandlung $S_B$-$S_C$ 117 °C, Umwandlung $S_C$-$S_A$ 134 °C, Umwandlung $S_A$-N 182 °C, Klp. (N-I) 206 °C;

4-[(trans-4-Pentylcyclohexyl)methoxy]benzoesäure-4-[3-(trans-4-pentylcyclohexyl)-1-propyloxy]-phenylester, Smp. (C-$S_B$) 95 °C, Umwandlung $S_B$-$S_C$ 103 °C, Umwandlung $S_C$-$S_A$ 154 °C, Umwandlung $S_A$-N 178 °C, Klp. (N-I) 212 °C;

4-[trans-4-Pentylcyclohexylcarbonyloxy]benzoesäure-4-[3-(trans-4-pentylcyclohexyl)-1-propyloxy]-phenylester, Smp. (C-S) 95 °C, Umwandlung S-$S_C$ 98 °C, Umwandlung $S_C$-N 113 °C, Klp. (N-I) 228 °C;

4-[3-(trans-4-Pentylcyclohexyl)-1-propyloxy]benzoesäure-4-[3-(trans-4-pentylcyclohexyl)-1-propyloxy]-phenylester, Smp. (C-S) 88 °C, Umwandlung S-$S_C$ 112 °C, Umwandlung $S_C$-$S_A$ 157 °C, Umwandlung $S_A$-N 183 °C, Klp. (N-I) 196 °C;

4-[3-(trans-4-Pentylcyclohexyl)-1-propyloxy]benzoesäure-4-[2-(trans-4-pentylcyclohexyl)äthyl]-phenylester, Smp. (C-S) 77 °C, Umwandlung S-$S_C$ 116 °C, Umwandlung $S_C$-$S_A$ 132 °C, Umwandlung $S_A$-N 190 °C, Klp. (N-I) 210 °C;

4-[3-(trans-4-Pentylcyclohexyl)-1-propyloxy]benzoesäure-4-[(trans-4-pentylcyclohexyl)methoxy]-phenylester;

4-[3-(trans-4-Pentylcyclohexyl)-1-propyloxy]benzoesäure-4-(trans-4-pentylcyclohexyl)phenylester;

4-[3-(trans-4-Pentylcyclohexyl)-1-propyloxy]benzoesäure-4-(trans-4-pentylcyclohexylcarbonyloxy)-phenylester;

## Beispiel 3

Eine Lösung von 2,1 g 4-[3-(trans-4-Pentylcyclohexyl)-1-propyloxy]benzaldehyd und 1,2 g 2-Pentyl-1,3-propandiol in 50 ml Toluol wurde mit 2 Tropfen 10%iger (Vol.) Schwefelsäure versetzt. Das Gemisch wurde 2,5 Stunden zum Sieden erhitzt, wobei das entstandene Wasser gleichzeitig abdestilliert wurde. Dann wurden 4 Tropfen Triäthylamin zum Reaktionsgemisch zugegeben. Nach dem Erkalten wurde das Gemisch mit 20 ml 1N Natriumhydrogencarbonat-Lösung und zweimal mit je 20 ml Wasser gewaschen, über Natriumsulfat getrocknet, filtriert und eingeengt. Chromatographie des Rückstandes an Kieselgel mit Toluol ergab 0,6 g trans-5-Pentyl-2-(4-[3-(trans-4-pentylcyclohexyl) -1-propyloxy]phenyl)-1,3-dioxan. Smp. (C-$S_B$) 65 °C, Umwandlung $S_B$-N 73 °C, Klp. (N-I) 128 °C.

Der als Ausgansmaterial verwendete 4-[3-(trans-4-Pentylcyclohexyl)-1-propyloxy]benzaldehyd wurde wie folgt hergestellt:

1,9 g 4-Hydroxybenzaldehyd, 5,0 g 3-(trans-4-Pentylcyclohexyl)-1-propylbromid, 8,3 g Kaliumcarbonat und 50 ml Butanon wurden in analoger Weise zu Beispiel 1 zu 6,0 g 4-[3-(trans-4-Pentylcyclohexyl)-1-propyloxy]benzaldehyd umgesetzt.

In analoger Weise können folgende Verbindungen hergestellt werden:

trans-5-Methyl-2-(4-[3-(trans-4-propylcyclohexyl)-1-propyloxy]phenyl]-1,3-dioxan;

trans-5-Aethyl-2-(4-[3-(trans-4-propylcyclohexyl)-1-propyloxy]phenyl]-1,3-dioxan;

trans-5-Propyl-2-(4-[3-(trans-4-propylcyclohexyl)-1-propyloxy]phenyl]-1,3-dioxan;

trans-5-Butyl-2-(4-[3-(trans-4-propylcyclohexyl)-1-propyloxy]phenyl]-1,3-dioxan;

trans-5-Pentyl-2-(4-[3-(trans-4-propylcyclohexyl)-1-propyloxy]phenyl]-1,3-dioxan;

trans-5-Hexyl-2-(4-[3-(trans-4-propylcyclohexyl)-1-propyloxy]phenyl]-1,3-dioxan;

trans-5-Heptyl-2-(4-[3-(trans-4-propylcyclohexyl)-1-propyloxy]phenyl]-1,3-dioxan;

trans-5-Methyl-2-(4-[3-(trans-4-pentylcyclohexyl)-1-propyloxy]phenyl]-1,3-dioxan;
trans-5-Aethyl-2-(4-[3-(trans-4-pentylcyclohexyl)-1-propyloxy]phenyl]-1,3-dioxan;
trans-5-Propyl-2-(4-[3-(trans-4-pentylcyclohexyl)-1-propyloxy]phenyl]-1,3-dioxan;
trans-5-Butyl-2-(4-[3-(trans-4-pentylcyclohexyl)-1-propyloxy]phenyl]-1,3-dioxan;
trans-5-Hexyl-2-(4-[3-(trans-4-pentylcyclohexyl)-1-propyloxy]phenyl]-1,3-dioxan;
trans-5-Heptyl-2-(4-[3-(trans-4-pentylcyclohexyl)-1-propyloxy]phenyl]-1,3-dioxan;
trans-5-Methyl-2-(4-[3-(trans-4-heptylcyclohexyl)-1-propyloxy]phenyl)-1,3-dioxan;
trans-5-Aethyl-2-(4-[3-(trans-4-heptylcyclohexyl)-1-propyloxy]phenyl)-1,3-dioxan;
trans-5-Propyl-2-(4-[3-(trans-4-heptylcyclohexyl)-1-propyloxy]phenyl)-1,3-dioxan;
trans-5-Butyl-2-(4-[3-(trans-4-heptylcyclohexyl)-1-propyloxy]phenyl)-1,3-dioxan;
trans-5-Pentyl-2-(4-[3-(trans-4-heptylcyclohexyl)-1-propyloxy]phenyl)-1,3-dioxan;
trans-5-Hexyl-2-(4-[3-(trans-4-heptylcyclohexyl)-1-propyloxy]phenyl)-1,3-dioxan;
trans-5-Heptyl-2-(4-[3-(trans-4-heptylcyclohexyl)-1-propyloxy]phenyl)-1,3-dioxan.

Beispiel 4

0,14 g 4-Hydroxy-2-fluorbenzonitril, 0,35 g 4-[3-(trans-4-Pentylcyclohexyl)-1-propyloxy]benzoesäure, 0,24 g N,N′-Dicyclohexylcarbodiimid, 0,04 g 4-(Dimethylamino)pyridin und 25 ml Dichlormethan wurden in analoger Weise zu Beispiel 2 umgesetzt. Dies ergab 0,32 g 4-[3-(trans-4-Pentylcyclohexyl)-1-propyloxy]-benzoesäure -4-cyano-3-fluorphenylester mit Smp. (C-N) 75 °C, Klp. (N-I) 146 °C.

Die als Ausgangsmaterial verwendete 4-[3-(trans-4-Pentylcyclohexyl)-1-propyloxy]benzoesäure wurde die folgt hergestellt:

Eine Lösung von 5 g 4-[3-(trans-4-Pentylcyclohexyl)-1-propyloxy]benzaldehyd in 100 ml Aceton wurde tropfenweise mit 10 ml Jones'Reagenz versetzt. Das Gemisch wurde 1 Stunde bei Raumtemperatur gerührt und dann auf 100 ml Wasser gegossen. Der dabei entstandene Niederschlag wurde abfiltriert, portionenweise mit Wasser gewaschen, und am Vakuum getrocknet. Das Rohprodukt wurde aus Aethanol umkristallisiert und ergab 2,2 g reine 4-[3-(trans-4-Pentylcyclohexyl)-1-propyloxy]benzoesäure mit Smp. (C-N) 204 °C, Klp. (N-I) 215 °C.

In analoger Weise können folgende Verbindungen hergestellt werden:

4-[3-(trans-4-Methylcyclohexyl)-1-propyloxy]benzoesäure-4-cyano-3-fluorphenylester;
4-[3-(trans-4-Aethylcyclohexyl)-1-propyloxy]benzoesäure-4-cyano-3-fluorphenylester;
4-[3-(trans-4-Propylcyclohexyl)-1-propyloxy]benzoesäure-4-cyano-3-fluorphenylester;
4-[3-(trans-4-Butylcyclohexyl)-1-propyloxy]benzoesäure-4-cyano-3-fluorphenylester;
4-[3-(trans-4-Hexylcyclohexyl)-1-propyloxy]benzoesäure-4-cyano-3-fluorphenylester;
4-[3-(trans-4-Heptylcyclohexyl)-1-propyloxy]benzoesäure-4-cyano-3-fluorphenylester;
4-[3-(trans-4-Octylcyclohexyl)-1-propyloxy]benzoesäure-4-cyano-3-fluorphenylester;
4-[3-(trans-4-Nonylcyclohexyl)-1-propyloxy]benzoesäure-4-cyano-3-fluorphenylester;
4-[3-(trans-4-Decylcyclohexyl)-1-propyloxy]benzoesäure-4-cyano-3-fluorphenylester;
4-[3-(trans-4-Methylcyclohexyl)-1-propyloxy]benzoesäure-4-cyano-2-fluorphenylester;
4-[3-(trans-4-Aethylcyclohexyl)-1-propyloxy]benzoesäure-4-cyano-2-fluorphenylester;
4-[3-(trans-4-Propylcyclohexyl)-1-propyloxy]benzoesäure-4-cyano-2-fluorphenylester;
4-[3-(trans-4-Butylcyclohexyl)-1-propyloxy]benzoesäure-4-cyano-2-fluorphenylester;
4-[3-(trans-4-Pentylcyclohexyl)-1-propyloxy]benzoesäure-4-cyano-2-fluorphenylester;
4-[3-(trans-4-Hexylcyclohexyl)-1-propyloxy]benzoesäure-4-cyano-2-fluorphenylester;
4-[3-(trans-4-Heptylcyclohexyl)-1-propyloxy]benzoesäure-4-cyano-2-fluorphenylester;
4-[3-(trans-4-Octylcyclohexyl)-1-propyloxy]benzoesäure-4-cyano-2-fluorphenylester;
4-[3-(trans-4-Nonylcyclohexyl)-1-propyloxy]benzoesäure-4-cyano-2-fluorphenylester;
4-[3-(trans-4-Decylcyclohexyl)-1-propyloxy]benzoesäure-4-cyano-2-fluorphenylester;
4-[3-(trans-4-Methylcyclohexyl)-1-propyloxy]benzoesäure-4-cyanophenylester;
4-[3-(trans-4-Aethylcyclohexyl)-1-propyloxy]benzoesäure-4-cyanophenylester;
4-[3-(trans-4-Propylcyclohexyl)-1-propyloxy]benzoesäure-4-cyanophenylester;
4-[3-(trans-4-Butylcyclohexyl)-1-propyloxy]benzoesäure-4-cyanophenylester;

4-[3-(trans-4-Pentylcyclohexyl)-1-propyloxy]benzoesäure-4-cyanophenylester, Smp. (C-$S_A$) 82 °C, Umwandlung $S_A$-N117 °C, Klp. (N-I) 168 °C;

4-[3-(trans-4-Pentylcyclohexyl)-1-propyloxy]benzoesäure-3,4-difluorphenylester, Smp. (C-N) 75 °C, Umwandlung $S_A$-N 65 °C, Klp. (N-I) 110 °C;

4-[3-(trans-4-Pentylcyclohexyl)-1-propyloxy]benzoesäure-4-fluorphenylester, Smp. (C-N) 90 °C, Umwandlung $S_A$-N 73 °C, Klp. (N-I) 127 °C;

4-[3-(trans-4-Pentylcyclohexyl)-1-propyloxy]benzoesäure-4-chlorphenylester, Smp. (C-$S_A$) 96 °C, Umwandlung $S_A$-N123 °C, Klp. (N-I) 150 °C;

4-[3-(trans-4-Pentylcyclohexyl)-1-propyloxy]benzoesäure-4-bromphenylester, Smp. (C-$S_A$) 108 °C, Umwandlung $S_A$-N 131 °C, Klp. (N-I) 152 °C;

4-[3-(trans-4-Pentylcyclohexyl)-1-propyloxy]benzoesäure-4-jodphenylester, Smp. (C-$S_A$) 114 °C, Umwandlung $S_A$-N 133 °C, Klp. (N-I) 149 °C;

4-[3-(trans-4-Propylcyclohexyl)-1-propyloxy]benzoesäure-4-methylphenylester;

4-[3-(trans-4-Propylcyclohexyl)-1-propyloxy]benzoesäure-4-äthylphenylester;

4-[3-(trans-4-Propylcyclohexyl)-1-propyloxy]benzoesäure-4-propylphenylester;

4-[3-(trans-4-Propylcyclohexyl)-1-propyloxy]benzoesäure-4-butylphenylester;

4-[3-(trans-4-Propylcyclohexyl)-1-propyloxy]benzoesäure-4-pentylphenylester;

4-[3-(trans-4-Propylcyclohexyl)-1-propyloxy]benzoesäure-4-hexylphenylester;

4-[3-(trans-4-Propylcyclohexyl)-1-propyloxy]benzoesäure-4-heptylphenylester;

4-[3-(trans-4-Propylcyclohexyl)-1-propyloxy]benzoesäure-4-octylphenylester;

4-[3-(trans-4-Pentylcyclohexyl)-1-propyloxy]benzoesäure-4-methylphenylester;

4-[3-(trans-4-Pentylcyclohexyl)-1-propyloxy]benzoesäure-4-äthylphenylester;

4-[3-(trans-4-Pentylcyclohexyl)-1-propyloxy]benzoesäure-4-propylphenylester;

4-[3-(trans-4-Pentylcyclohexyl)-1-propyloxy]benzoesäure-4-butylphenylester;

4-[3-(trans-4-Pentylcyclohexyl)-1-propyloxy]benzoesäure-4-pentylphenylester;

4-[3-(trans-4-Pentylcyclohexyl)-1-propyloxy]benzoesäure-4-hexylphenylester;

4-[3-(trans-4-Pentylcyclohexyl)-1-propyloxy]benzoesäure-4-heptylphenylester;

4-[3-(trans-4-Pentylcyclohexyl)-1-propyloxy]benzoesäure-4-octylphenylester;

4-[3-(trans-4-Heptylcyclohexyl)-1-propyloxy]benzoesäure-4-methylphenylester;

4-[3-(trans-4-Heptylcyclohexyl)-1-propyloxy]benzoesäure-4-äthylphenylester;

4-[3-(trans-4-Heptylcyclohexyl)-1-propyloxy]benzoesäure-4-propylphenylester;

4-[3-(trans-4-Heptylcyclohexyl)-1-propyloxy]benzoesäure-4-butylphenylester;

4-[3-(trans-4-Heptylcyclohexyl)-1-propyloxy]benzoesäure-4-pentylphenylester;

4-[3-(trans-4-Heptylcyclohexyl)-1-propyloxy]benzoesäure-4-hexylphenylester;

4-[3-(trans-4-Heptylcyclohexyl)-1-propyloxy]benzoesäure-4-heptylphenylester;

4-[3-(trans-4-Heptylcyclohexyl)-1-propyloxy]benzoesäure-4-octylphenylester;

4-[3-(trans-4-Pentylcyclohexyl)-1-propyloxy]benzoesäure-4-methyl-2-fluorphenylester;

4-[3-(trans-4-Pentylcyclohexyl)-1-propyloxy]benzoesäure-4-äthyl-2-fluorphenylester;

4-[3-(trans-4-Pentylcyclohexyl)-1-propyloxy]benzoesäure-4-propyl-2-fluorphenylester;

4-[3-(trans-4-Pentylcyclohexyl)-1-propyloxy]benzoesäure-4-butyl-2-fluorphenylester;

4-[3-(trans-4-Pentylcyclohexyl)-1-propyloxy]benzoesäure-4-pentyl-2-fluorphenylester;

4-[3-(trans-4-Pentylcyclohexyl)-1-propyloxy]benzoesäure-4-hexyl-2-fluorphenylester;

4-[3-(trans-4-Pentylcyclohexyl)-1-propyloxy]benzoesäure-4-heptyl-2-fluorphenylester;

4-[3-(trans-4-Pentylcyclohexyl)-1-propyloxy]benzoesäure-4-octyl-2-fluorphenylester;

4-[3-(trans-4-Pentylcyclohexyl)-1-propyloxy]benzoesäure-4-nonyl-2-fluorphenylester;

4-[3-(trans-4-Pentylcyclohexyl)-1-propyloxy]benzoesäure-4-decyl-2-fluorphenylester;

4-[3-(trans-4-Pentylcyclohexyl)-1-propyloxy]benzoesäure-4-methyl-3-fluorphenylester;

4-[3-(trans-4-Pentylcyclohexyl)-1-propyloxy]benzoesäure-4-äthyl-3-fluorphenylester;

4-[3-(trans-4-Pentylcyclohexyl)-1-propyloxy]benzoesäure-4-propyl-3-fluorphenylester;

4-[3-(trans-4-Pentylcyclohexyl)-1-propyloxy]benzoesäure-4-butyl-3-fluorphenylester;

4-[3-(trans-4-Pentylcyclohexyl)-1-propyloxy]benzoesäure-4-pentyl-3-fluorphenylester;

4-[3-(trans-4-Pentylcyclohexyl)-1-propyloxy]benzoesäure-4-hexyl-3-fluorphenylester;

4-[3-(trans-4-Pentylcyclohexyl)-1-propyloxy]benzoesäure-4-heptyl-3-fluorphenylester;

4-[3-(trans-4-Pentylcyclohexyl)-1-propyloxy]benzoesäure-4-octyl-3-fluorphenylester;

4-[3-(trans-4-Pentylcyclohexyl)-1-propyloxy]benzoesäure-4-nonyl-3-fluorphenylester;

4-[3-(trans-4-Pentylcyclohexyl)-1-propyloxy]benzoesäure-4-decyl-3-fluorphenylester;

4-[3-(trans-4-Pentylcyclohexyl)-1-propyloxy]benzoesäure-trans-4-methylcyclohexylester;

4-[3-(trans-4-Pentylcyclohexyl)-1-propyloxy]benzoesäure-trans-4-äthylcyclohexylester;

4-[3-(trans-4-Pentylcyclohexyl)-1-propyloxy]benzoesäure-trans-4-propylcyclohexylester;

4-[3-(trans-4-Pentylcyclohexyl)-1-propyloxy]benzoesäure-trans-4-butylcyclohexylester;

4-[3-(trans-4-Pentylcyclohexyl)-1-propyloxy]benzoesäure-trans-4-pentylcyclohexylester, Smp. (C-$S_A$) 64 °C, Umwandlung $S_A$-N 108 °C, Klp. (N-I) 132 °C;

4-[3-(trans-4-Pentylcyclohexyl)-1-propyloxy]benzoesäure-trans-4-hexylcyclohexylester;

4-[3-(trans-4-Pentylcyclohexyl)-1-propyloxy]benzoesäure-trans-4-heptylcyclohexylester;

4-[3-(trans-4-Pentylcyclohexyl)-1-propyloxy]benzoesäure-trans-4-octylcyclohexylester;
4-[3-(trans-4-Propylcyclohexyl)-1-propyloxy]benzoesäure-methylester;
4-[3-(trans-4-Propylcyclohexyl)-1-propyloxy]benzoesäure-äthylester;
4-[3-(trans-4-Propylcyclohexyl)-1-propyloxy]benzoesäure-propylester;
4-[3-(trans-4-Propylcyclohexyl)-1-propyloxy]benzoesäure-butylester;
4-[3-(trans-4-Propylcyclohexyl)-1-propyloxy]benzoesäure-pentylester;
4-[3-(trans-4-Propylcyclohexyl)-1-propyloxy]benzoesäure-hexylester;
4-[3-(trans-4-Propylcyclohexyl)-1-propyloxy]benzoesäure-heptylester;
4-[3-(trans-4-Propylcyclohexyl)-1-propyloxy]benzoesäure-octylester;
4-[3-(trans-4-Pentylcyclohexyl)-1-propyloxy]benzoesäure-methylester, Smp. (C-N) 57 °C, Klp. (N-I) 62 °C;
4-[3-(trans-4-Pentylcyclohexyl)-1-propyloxy]benzoesäure-äthylester;
4-[3-(trans-4-Pentylcyclohexyl)-1-propyloxy]benzoesäure-propylester;
4-[3-(trans-4-Pentylcyclohexyl)-1-propyloxy]benzoesäure-butylester;
4-[3-(trans-4-Pentylcyclohexyl)-1-propyloxy]benzoesäure-pentylester;
4-[3-(trans-4-Pentylcyclohexyl)-1-propyloxy]benzoesäure-hexylester;
4-[3-(trans-4-Pentylcyclohexyl)-1-propyloxy]benzoesäure-heptylester;
4-[3-(trans-4-Pentylcyclohexyl)-1-propyloxy]benzoesäure-octylester;
4-[3-(trans-4-Heptylcyclohexyl)-1-propyloxy]benzoesäure-methylester;
4-[3-(trans-4-Heptylcyclohexyl)-1-propyloxy]benzoesäure-äthylester;
4-[3-(trans-4-Heptylcyclohexyl)-1-propyloxy]benzoesäure-propylester;
4-[3-(trans-4-Heptylcyclohexyl)-1-propyloxy]benzoesäure-butylester;
4-[3-(trans-4-Heptylcyclohexyl)-1-propyloxy]benzoesäure-pentylester;
4-[3-(trans-4-Heptylcyclohexyl)-1-propyloxy]benzoesäure-hexylester;
4-[3-(trans-4-Heptylcyclohexyl)-1-propyloxy]benzoesäure-heptylester;
4-[3-(trans-4-Methylcyclohexyl)-1-propyloxy]benzoesäure-[(S)-1-methylheptyl]ester;
4-[3-(trans-4-Aethylcyclohexyl)-1-propyloxy]benzoesäure-[(S)-1-methylheptyl]ester;
4-[3-(trans-4-Propylcyclohexyl)-1-propyloxy]benzoesäure-[(S)-1-methylheptyl]ester;
4-[3-(trans-4-Butylcyclohexyl)-1-propyloxy]benzoesäure-[(S)-1-methylheptyl]ester;
4-[3-(trans-4-Pentylcyclohexyl)-1-propyloxy]benzoesäure-[(S)-1-methylheptyl]ester;
4-[3-(trans-4-Hexylcyclohexyl)-1-propyloxy]benzoesäure-[(S)-1-methylheptyl]ester;
4-[3-(trans-4-Heptylcyclohexyl)-1-propyloxy]benzoesäure-[(S)-1-methylheptyl]ester;
4-[3-(trans-4-Octylcyclohexyl)-1-propyloxy]benzoesäure-[(S)-1-methylheptyl]ester;
4-[3-(trans-4-Nonylcyclohexyl)-1-propyloxy]benzoesäure-[(S)-1-methylheptyl]ester;
4-[3-(trans-4-Decylcyclohexyl)-1-propyloxy]benzoesäure-[(S)-1-methylheptyl]ester.

Beispiel 5

Ein Gemisch von 0,3 g trans-4-Pentylcyclohexanol, 0,5 g 3-(trans-4-Pentylcyclohexyl)-1-propylbromid, 1,0 g Kaliumcarbonat und 50 ml absolutem Cyclohexanon wird während 7 Tagen unter Rückfluss erhitzt. Anschliessend wird der Ansatz in analoger Weise zu Beispiel 1 aufgearbeitet und gereinigt. Dies ergibt 0,5 g trans-4-Pentylcyclohexyl-3-(trans-4-pentylcyclohexyl)-1-propyläther; Smp. (C-$S_B$) 36 °C, Klp. ($S_B$-I) 42 °C.

In analoger Weise können folgende Verbindungen hergestellt werden:
trans-4-Propylcyclohexyl-3-(trans-4-methylcyclohexyl)-1-propyläther;
trans-4-Propylcyclohexyl-3-(trans-4-äthylcyclohexyl)-1-propyläther;
trans-4-Propylcyclohexyl-3-(trans-4-propylcyclohexyl)-1-propyläther;
trans-4-Propylcyclohexyl-3-(trans-4-butylcyclohexyl)-1-propyläther;
trans-4-Propylcyclohexyl-3-(trans-4-pentylcyclohexyl)-1-propyläther;
trans-4-Propylcyclohexyl-3-(trans-4-hexylcyclohexyl)-1-propyläther;
trans-4-Propylcyclohexyl-3-(trans-4-heptylcyclohexyl)-1-propyläther;
trans-4-Pentylcyclohexyl-3-(trans-4-methylcyclohexyl)-1-propyläther;
trans-4-Pentylcyclohexyl-3-(trans-4-äthylcyclohexyl)-1-propyläther;
trans-4-Pentylcyclohexyl-3-(trans-4-propylcyclohexyl)-1-propyläther;
trans-4-Pentylcyclohexyl-3-(trans-4-butylcyclohexyl)-1-propyläther;
trans-4-Pentylcyclohexyl-3-(trans-4-hexylcyclohexyl)-1-propyläther;
trans-4-Pentylcyclohexyl-3-(trans-4-heptylcyclohexyl)-1-propyläther;
trans-4-Heptylcyclohexyl-3-(trans-4-methylcyclohexyl)-1-propyläther;
trans-4-Heptylcyclohexyl-3-(trans-4-äthylcyclohexyl)-1-propyläther;

trans-4-Heptylcyclohexyl-3-(trans-4-propylcyclohexyl)-1-propyläther;
trans-4-Heptylcyclohexyl-3-(trans-4-butylcyclohexyl)-1-propyläther;
trans-4-Heptylcyclohexyl-3-(trans-4-pentylcyclohexyl)-1-propyläther;
trans-4-Heptylcyclohexyl-3-(trans-4-hexylcyclohexyl)-1-propyläther;
trans-4-Heptylcyclohexyl-3-(trans-4-heptylcyclohexyl)-1-propyläther;
trans-4-Cyanocyclohexyl-3-(trans-4-methylcyclohexyl)-1-propyläther;
trans-4-Cyanocyclohexyl-3-(trans-4-äthylcyclohexyl)-1-propyläther;
trans-4-Cyanocyclohexyl-3-(trans-4-propylcyclohexyl)-1-propyläther;
trans-4-Cyanocyclohexyl-3-(trans-4-butylcyclohexyl)-1-propyläther;
trans-4-Cyanocyclohexyl-3-(trans-4-pentylcyclohexyl)-1-propyläther;
trans-4-Cyanocyclohexyl-3-(trans-4-hexylcyclohexyl)-1-propyläther;
trans-4-Cyanocyclohexyl-3-(trans-4-heptylcyclohexyl)-1-propyläther;
trans-4-Cyanocyclohexyl-3-(trans-4-octylcyclohexyl)-1-propyläther;
trans-4-Cyanocyclohexyl-3-(trans-4-nonylcyclohexyl)-1-propyläther;
trans-4-Cyanocyclohexyl-3-(trans-4-decylcyclohexyl)-1-propyläther;
trans-4-(trans-4-Propylcyclohexyl)cyclohexyl-3-(trans-4-propylcyclohexyl)-1-propyläther;
trans-4-(trans-4-Pentylcyclohexyl)cyclohexyl-3-(trans-4-propylcyclohexyl)-1-propyläther;
trans-4-(trans-4-Heptylcyclohexyl)cyclohexyl-3-(trans-4-propylcyclohexyl)-1-propyläther;
trans-4-(trans-4-Propylcyclohexyl)cyclohexyl-3-(trans-4-pentylcyclohexyl)-1-propyläther;
trans-4-(trans-4-Pentylcyclohexyl)cyclohexyl-3-(trans-4-pentylcyclohexyl)-1-propyläther;
trans-4-(trans-4-Heptylcyclohexyl)cyclohexyl-3-(trans-4-pentylcyclohexyl)-1-propyläther;
trans-4-(trans-4-Propylcyclohexyl)cyclohexyl-3-(trans-4-heptylcyclohexyl)-1-propyläther;
trans-4-(trans-4-Pentylcyclohexyl)cyclohexyl-3-(trans-4-heptylcyclohexyl)-1-propyläther;
trans-4-(trans-4-Heptylcyclohexyl)cyclohexyl-3-(trans-4-heptylcyclohexyl)-1-propyläther;
3-(trans-4-Methylcyclohexyl)-1-propyl-trans-4-(4-cyanophenyl)cyclohexyläther;
3-(trans-4-Aethylcyclohexyl)-1-propyl-trans-4-(4-cyanophenyl)cyclohexyläther;
3-(trans-4-Propylcyclohexyl)-1-propyl-trans-4-(4-cyanophenyl)cyclohexyläther;
3-(trans-4-Butylcyclohexyl)-1-propyl-trans-4-(4-cyanophenyl)cyclohexyläther;
3-(trans-4-Pentylcyclohexyl)-1-propyl-trans-4-(4-cyanophenyl)cyclohexyläther;
3-(trans-4-Hexylcyclohexyl)-1-propyl-trans-4-(4-cyanophenyl)cyclohexyläther;
3-(trans-4-Heptylcyclohexyl)-1-propyl-trans-4-(4-cyanophenyl)cyclohexyläther;
3-(trans-4-Octylcyclohexyl)-1-propyl-trans-4-(4-cyanophenyl)cyclohexyläther;
3-(trans-4-Nonylcyclohexyl)-1-propyl-trans-4-(4-cyanophenyl)cyclohexyläther;
3-(trans-4-Decylcyclohexyl)-1-propyl-trans-4-(4-cyanophenyl)cyclohexyläther;
3-(trans-4-Methylcyclohexyl)-1-propyl-trans-4-(4-propylphenyl)cyclohexyläther;
3-(trans-4-Aethylcyclohexyl)-1-propyl-trans-4-(4-propylphenyl)cyclohexyläther;
3-(trans-4-Propylcyclohexyl)-1-propyl-trans-4-(4-propylphenyl)cyclohexyläther;
3-(trans-4-Butylcyclohexyl)-1-propyl-trans-4-(4-propylphenyl)cyclohexyläther;
3-(trans-4-Pentylcyclohexyl)-1-propyl-trans-4-(4-propylphenyl)cyclohexyläther;
3-(trans-4-Hexylcyclohexyl)-1-propyl-trans-4-(4-propylphenyl)cyclohexyläther;
3-(trans-4-Heptylcyclohexyl)-1-propyl-trans-4-(4-propylphenyl)cyclohexyläther;
3-(trans-4-Octylcyclohexyl)-1-propyl-trans-4-(4-propylphenyl)cyclohexyläther;
3-(trans-4-Nonylcyclohexyl)-1-propyl-trans-4-(4-propylphenyl)cyclohexyläther;
3-(trans-4-Decylcyclohexyl)-1-propyl-trans-4-(4-propylphenyl)cyclohexyläther;
3-(trans-4-Methylcyclohexyl)-1-propyl-trans-4-(4-pentylphenyl)cyclohexyläther;
3-(trans-4-Aethylcyclohexyl)-1-propyl-trans-4-(4-pentylphenyl)cyclohexyläther;
3-(trans-4-Propylcyclohexyl)-1-propyl-trans-4-(4-pentylphenyl)cyclohexyläther;
3-(trans-4-Butylcyclohexyl)-1-propyl-trans-4-(4-pentylphenyl)cyclohexyläther;
3-(trans-4-Pentylcyclohexyl)-1-propyl-trans-4-(4-pentylphenyl)cyclohexyläther;
3-(trans-4-Hexylcyclohexyl)-1-propyl-trans-4-(4-pentylphenyl)cyclohexyläther;
3-(trans-4-Heptylcyclohexyl)-1-propyl-trans-4-(4-pentylphenyl)cyclohexyläther;
3-(trans-4-Octylcyclohexyl)-1-propyl-trans-4-(4-pentylphenyl)cyclohexyläther;
3-(trans-4-Nonylcyclohexyl)-1-propyl-trans-4-(4-pentylphenyl)cyclohexyläther;
3-(trans-4-Decylcyclohexyl)-1-propyl-trans-4-(4-pentylphenyl)cyclohexyläther;
3-(trans-4-Methylcyclohexyl)-1-propyl-trans-4-(4-heptylphenyl)cyclohexyläther;
3-(trans-4-Aethylcyclohexyl)-1-propyl-trans-4-(4-heptylphenyl)cyclohexyläther;
3-(trans-4-Propylcyclohexyl)-1-propyl-trans-4-(4-heptylphenyl)cyclohexyläther;
3-(trans-4-Butylcyclohexyl)-1-propyl-trans-4-(4-heptylphenyl)cyclohexyläther;

3-(trans-4-Pentylcyclohexyl)-1-propyl-trans-4-(4-heptylphenyl)cyclohexyläther;
3-(trans-4-Hexylcyclohexyl)-1-propyl-trans-4-(4-heptylphenyl)cyclohexyläther;
3-(trans-4-Heptylcyclohexyl)-1-propyl-trans-4-(4-heptylphenyl)cyclohexyläther;
3-(trans-4-Octylcyclohexyl)-1-propyl-trans-4-(4-heptylphenyl)cyclohexyläther;
3-(trans-4-Nonylcyclohexyl)-1-propyl-trans-4-(4-heptylphenyl)cyclohexyläther;
3-(trans-4-Decylcyclohexyl)-1-propyl-trans-4-(4-heptylphenyl)cyclohexyläther.

Beispiel 6

Ein Gemisch von 0,1 g Natriumhydrid und 25 ml Tetrahydrofuran wurde unter Stickstoffbegasung mit 0,5 g trans-4-Pentylcyclohexanol versetzt, 2 Stunden gerührt, dann mit 1,0 g 3-(trans-4-Pentylcyclohexyl)-1-propylbromid versetzt und anschliessend über Nacht bei 70 °C erwärmt. Anschliessend wurde der Ansatz in analoger Weise zu Beispiel 1 aufgearbeitet und gereinigt. Dies ergab 0,7 g trans-4-Pentylcyclohexyl-3-(trans-4-pentylcyclohexyl)-1-propyläther. Schmelzpunkt (C-$S_B$) 36 °C, Klp. ($S_B$-I) 42 °C.

In analoger Weise wurde folgende Verbindung hergestellt:

trans-4-[2-(trans-4-Propylcyclohexyl)äthyl]cyclohexyl-3-(trans-4-pentylcyclohexyl)-1-propyläther, Smp. (C-$S_B$) 58 °C, Klp. ($S_B$-I) 129 °C.

In analoger Weise können auch die weiteren, in Beispiel 5 genannten Verbindungen hergestellt werden.

## Patentansprüche

**1.** Verbindungen der allgemeinen Formel

$$R^1\text{-}A^1\text{-}Z^1\text{-}A^2\text{-}(Z^2\text{-}A^3)_n\text{-}R^2 \qquad \text{I}$$

worin $Z^1$ die Gruppe $-CH_2CH_2CH_2O-$ oder $-OCH_2CH_2CH_2-$ bezeichnet: n für die Zahl 0 oder 1 steht; $R^1$ eine Gruppe $R^3$ oder $R^3$-$A^4$-$Z^3$- darstellt; $R^2$ eine Gruppe $R^4$ oder $R^4$-$A^5$-$Z^4$- darstellt; $Z^2$, $Z^3$ und $Z^4$ unabhängig voneinander eine einfache Kovalenzbindung, $-CH_2CH_2-$, $-CH_2O-$, $-OCH_2-$, -COO-, -OOC-, $-CH_2CH_2CH_2O-$ oder $-OCH_2CH_2CH_2$ bedeuten; $A^1$, $A^2$, $A^3$, $A^4$ und $A^5$ unabhängig voneinander unsubstituiertes oder mit Methyl, Halogen und/oder Cyano substituiertes 1,4-Phenylen, in welchem gegebenenfalls 1-4 CH-Gruppen durch Stickstoff ersetzt sind, unsubstituiertes oder mit Methyl und/oder Cyano substituiertes trans-1,4-Cyclohexylen, in welchem gegebenenfalls 2 $CH_2$-Gruppen durch Sauerstoff und/oder Schwefel ersetzt sind, Bicyclo[2,2,2]octan-1,4-diyl, 1,3,4-Thiadiazol-2,5-diyl, Naphthalin-2,6-diyl, Tetralin-2,6-diyl oder Decalin-2,6-diyl bezeichnen; $R^3$ und $R^4$ unabhängig voneinander eine unsubstituierte oder mit Halogen und/oder Cyano substituierte Alkyl- oder Alkenylgruppe mit 1 bzw. 2 bis 18 Kohlenstoffatomen darstellen, in welcher gegebenenfalls 1 oder 2 nicht benachbarte $CH_2$-Gruppen durch -O-, -COO-und/oder -OOC- ersetzt sind, oder einer der Reste $R^3$ und $R^4$ auch Wasserstoff, Halogen, Cyano oder -NCS darstellt.

**2.** Verbindungen nach Anspruch 1, dadurch gekennzeichnet, dass $A^1$, $A^2$, $A^3$, $A^4$ und $A^5$ unabhängig voneinander 1,4-Phenylen oder trans-1,4-Cyclohexylen bezeichnen, oder eine der Gruppen $A^1$, $A^2$, $A^3$, $A^4$ und $A^5$ auch mit Methyl, Halogen und/oder Cyano substituiertes 1,4-Phenylen oder mit Methyl und/oder Cyano substituiertes trans-1,4-Cyclohexylen bezeichnet, und/oder eine der Gruppen $A^1$, $A^2$, $A^3$, $A^4$ und $A^5$ auch 1,4-Phenylen, in welchem 1-4 CH-Gruppen durch Stickstoff ersetzt sind, trans-1,4-Cyclohexylen, in welchem 2 $CH_2$-Gruppen durch Sauerstoff und/oder Schwefel ersetzt sind, Bicyclo-[2,2,2]octan-1,4-diyl, 1,3,4-Thiadiazol-2,5-diyl, Naphthalin-2,6-diyl, Tetralin-2,6-diyl oder Decalin-2,6-diyl bezeichnet.

**3.** Verbindungen nach Anspruch 1 oder 2, dadurch gekennzeichnet, dass eine der Gruppen $Z^2$, $Z^3$ und $Z^4$ eine einfache Kovalenzbindung, $-CH_2CH_2-$, $-CH_2O-$, $-OCH_2-$, -COO-, -OOC-, $-CH_2CH_2CH_2CH_2O-$ oder $-OCH_2CH_2CH_2-$ bedeutet und die beiden andern der Gruppen $Z^2$, $Z^3$ und $Z^4$ je eine einfache Kovalenzbindung, -COO- und/oder -OOC- bedeuten.

**4.** Verbindungen nach einem der Ansprüche 1 bis 3, dadurch gekennzeichnet, dass $Z^1$ die Gruppe $-CH_2CH_2CH_2O-$ und $A^1$ einen gesättigten Ring bedeuten oder $Z^1$ die Gruppe $-OCH_2CH_2CH_2-$ und $A^2$ einen gesättigten Ring bedeuten.

**5.** Verbindungen nach einem der Ansprüche 1 bis 4, gekennzeichnet durch die allgemeinen Formel

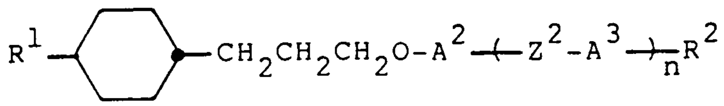

IA

oder

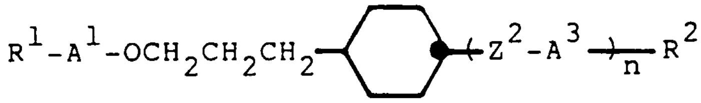

IF

worin $A^1$, $A^2$, $A^3$, $R^1$, $R^2$, $Z^2$ und n die in Anspruch 1 gegebenen Bedeutungen haben.

6. Verbindungen nach einem der Ansprüche 1 bis 5, gekennzeichnet durch die allgemeinen Formeln

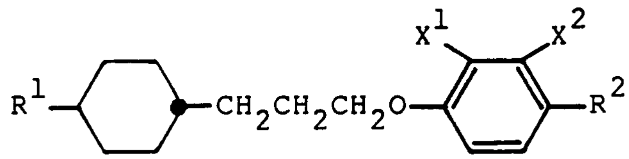

I-1

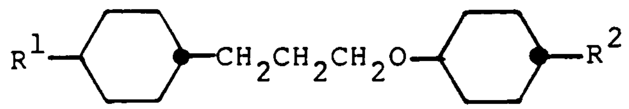

I-2

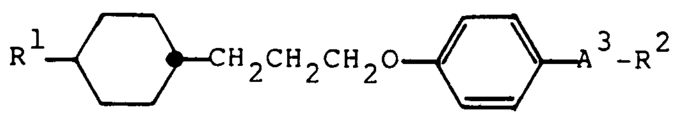

I-3

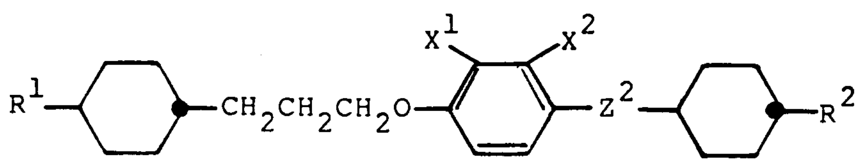

I-4

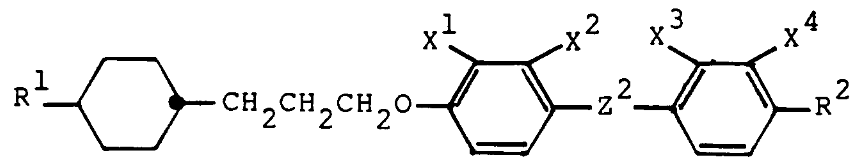

I-5

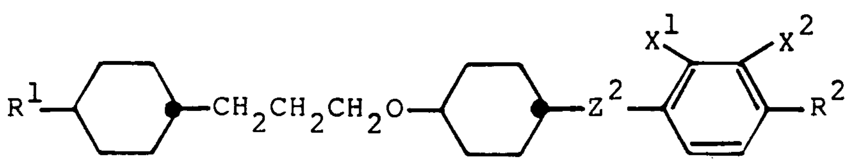

I-6

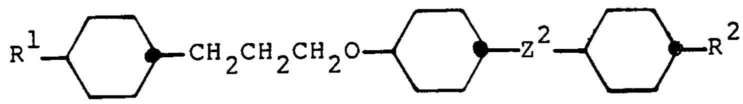

I-7

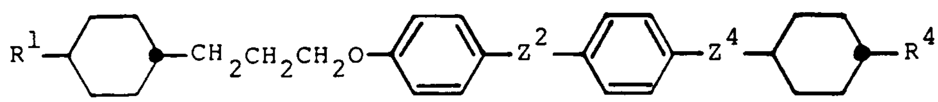

I-8

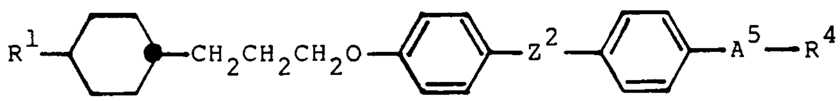

I-9

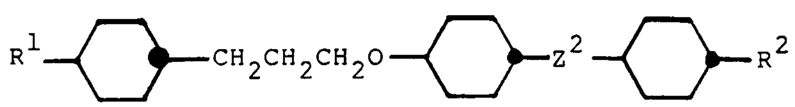

I-10

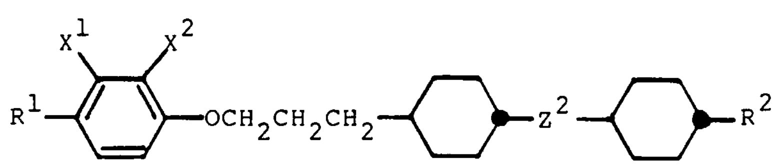

I-11

worin $A^3$, $A^5$, $R^1$, $R^2$, $R^4$ und $Z^2$ die in Anspruch 1 gegebenen Bedeutungen haben und $X^1$, $X^2$, $X^3$ und $X^4$ unabhängig voneinander Wasserstoff, Methyl, Halogen oder Cyano bezeichnen.

**7.** Verbindungen nach Anspruch 6, dadurch gekennzeichnet, dass $X^1$, $X^2$, $X^3$ und $X^4$ unabhängig voneinander Wasserstoff und/oder Fluor bedeuten.

**8.** Verbindungen nach einem der Ansprüche 1 bis 7, dadurch gekennzeichnet, dass $R^1$ eine Gruppe $R^3$ und $R^2$ eine Gruppe $R^4$ bedeuten.

**9.** Verbindungen nach einem der Ansprüche 1 bis 8, dadurch gekennzeichnet, dass $R^3$ und $R^4$ höchstens je 18 Kohlenstoffatome aufweisen.

**10.** Verbindungen nach einem der Ansprüche 1 bis 9, dadurch gekennzeichnet, dass $R^3$ und $R^4$ unabhängig voneinander eine unsubstituierte oder mit Halogen und/oder Cyano substituierte Alkyl- oder Alkenylgruppe, in welcher gegebenenfalls 1 oder 2 nicht benachbarte $CH_2$-Gruppen durch -O-, -COO- und/oder -COO- ersetzt sind, bedeuten und höchstens je 12, vorzugsweise höchstens je 7 Kohlenstoffatome aufweisen, oder einer der Reste $R^3$ und $R^4$ auch Wasserstoff, Halogen, Cyano oder -NCS bedeutet.

**11.** Verbindungen nach Anspruch 10, dadurch gekennzeichnet, dass $R^3$ Alkyl, Alkenyl, Alkoxy, Alkenyloxy, Alkoxycarbonyl, Alkenyloxycarbonyl, Alkanoyloxy oder Alkenoyloxy bezeichnet und $R^4$ Alkyl, Alkenyl, Alkoxy, Alkenyloxy, Alkoxycarbonyl, Alkenyloxycarbonyl, Alkanoyloxy, Alkenoyloxy, Halogen, Cyano oder -NCS bedeutet.

**12.** Verbindungen nach einem der Ansprüche 1 bis 9, dadurch gekennzeichnet, dass $R^3$ und $R^4$ unabhängig voneinander eine unsubstituierte oder mit Halogen und/oder Cyano substituierte $C_1$-$C_{18}$-Alkyl- oder $C_2$-$C_{18}$-Alkenylgruppe bedeuten, in welcher gegebenenfalls 1 oder 2 nicht benachbarte $CH_2$-Gruppen durch -O-, -COO- und/oder -OOC- ersetzt sind, und dass die Summe der Kohlenstoffatome in $R^3$ und $R^4$ zusammen mindestens 10, vorzugsweise mindestens 12 beträgt.

**13.** Verbindungen nach Anspruch 12, dadurch gekennzeichnet, dass $R^3$ und $R^4$ unabhängig voneinander Alkyl, Alkenyl, Alkoxy, Alkenyloxy, Alkoxycarbonyl, Alkenyloxycarbonyl, Alkanoyloxy oder Alkenoyloxy bedeuten.

**14.** Flüssigkristallines Gemisch mit mindestens 2 Komponenten, dadurch gekennzeichnet, dass mindestens eine Komponente eine Verbindung der in Anspruch 1 definierten Formel I ist.

**15.** Flüssigkristallines Gemisch nach Anspruch 14, dadurch gekennzeichnet, dass der Anteil an Verbindungen der Formel I 1-60 Gew.-% beträgt.

**16.** Verwendung der Verbindungen der in Anspruch 1 definierten Formel I für elektro-optische Zwecke.

**Claims**

**1.** Compounds of the general formula

$$R^1\text{-}A^1\text{-}Z^1\text{-}A^2\text{-}(Z^2\text{-}A^3)_n\text{-}R^2$$

wherein $Z^1$ denotes the group $-CH_2CH_2CH_2O-$ or $-OCH_2CH_2CH_2-$; n stands for the number 0 or 1; $R^1$ represents a group $R^3$ or $R^3$-$A^4$-$Z^3$-; $R^2$ represents a group $R^4$ or $R^4$-$A^5$-$Z^4$-; $Z^2$, $Z^3$ and $Z^4$ each independently signify a single covalent bond, $-CH_2-CH_2-$, $-CH_2O-$, $-OCH_2-$, -COO-, -OOC-, $-CH_2CH_2CH_2O-$ or $-OCH_2CH_2CH_2-$; $A^1$, $A^2$, $A^3$, $A^4$ and $A^5$ each independently denote unsubstituted or methyl-, halogen- and/or cyano-substituted 1,4-phenylene, in which optionally 1-4 CH groups are replaced by nitrogen, unsubstituted or methyl- and/or cyano-substituted trans-1,4-cyclohexylene, in which optionally 2 $CH_2$ groups are replaced by oxygen and/or sulphur, bicyclo[2.2.2]octane-1,4-diyl, 1,3,4-thiadiazole-2,5-diyl, naphthalene-2,6-diyl, tetralin-2,6-diyl or decalin-2,6-diyl; $R^3$ and $R^4$ each independently represent an unsubstituted or halogen- and/or cyano-substituted alkyl or alkenyl group with 1 and, respectively, 2 to 18 carbon atoms, in which optionally 1 $CH_2$ group or 2 non-adjacent $CH_2$ groups is/are replaced by -O-, -COO- and/or -OOC-, or one of the residues $R^3$ and $R^4$ also represents hydrogen, halogen, cyano or -NCS.

**2.** Compounds according to claim 1, characterized in that $A^1$, $A^2$, $A^3$, $A^4$ and $A^5$ each independently denote 1,4-phenylene or trans-1,4-cyclohexylene or one of the groups $A^1$, $A^2$, $A^3$, $A^4$ and $A^5$ also denotes methyl-, halogen- and/or cyano-substituted 1,4-phenylene or methyl- and/or cyano-substituted trans-1,4-cyclohexylene and/or one of the groups $A^1$, $A^2$, $A^3$, $A^4$ and $A^5$ also denotes 1,4-phenylene, in which 1-4 CH groups are replaced by nitrogen, trans-1,4-cyclohexylene, in which 2 $CH_2$ groups are replaced by oxygen and/or sulphur, bicyclo[2,2,2]octane-1,4-diyl, 1,3,4-thiadiazole-2,5-diyl, naphthalene-2,6-diyl, tetralin-2,6diyl or decalin-2,6-diyl.

**3.** Compounds according to claim 1 or claim 2, characterized in that one of the groups $Z^2$, $Z^3$ and $Z^4$ signifies a single covalent bond, $-CH_2CH_2-$, $-CH_2O-$, $-OCH_2-$, -COO-, -OOC-, $-CH_2CH_2CH_2O-$ or $-OCH_2CH_2CH_2-$ and the other two of the groups $Z^2$, $Z^3$ and $Z^4$ each signify a single covalent bond, -COO- and/or -OOC-.

**4.** Compounds according to any one of claims 1 to 3, characterized in that $Z^1$ signifies the group $-CH_2CH_2CH_2O-$ and $A^1$ signifies a saturated ring or $Z^1$ signifies the group $-OCH_2CH_2CH_2-$ and $A^2$ signifies a saturated ring.

**5.** Compounds according to any one of claims 1 to 4, characterized by the general formula

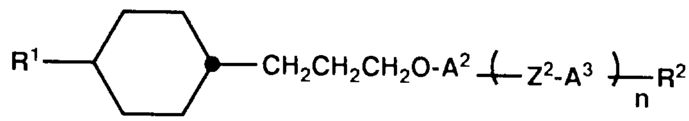

IA

or

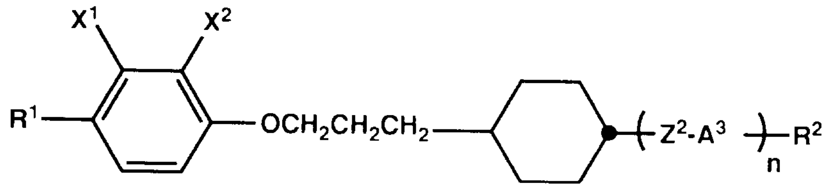

IG

wherein $A^1$, $A^2$, $A^3$, $R^1$, $R^2$, $Z^2$ and n have the significances given in claim 1.

**6.** Compounds according to any one of claims 1 to 5, characterized by the general formulae

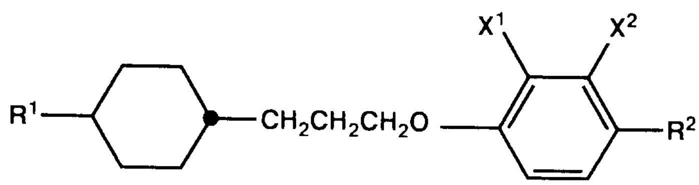

I-1

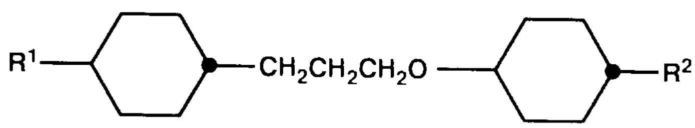

I-2

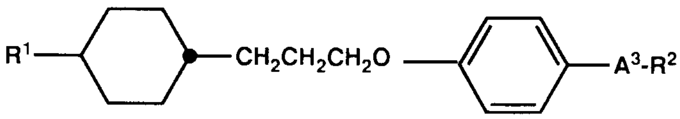

I-3

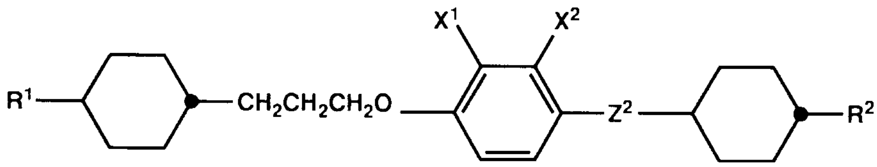

I-4

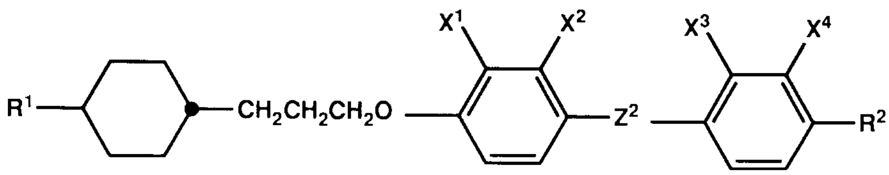

I-5

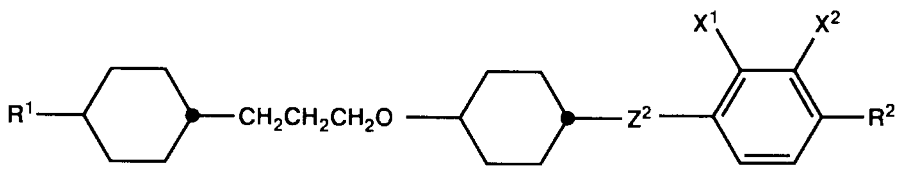

I-6

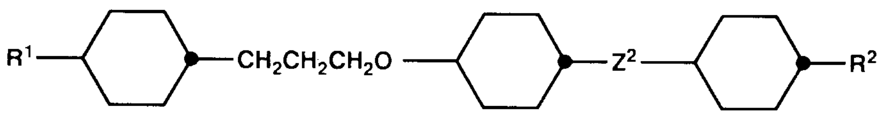

I-7

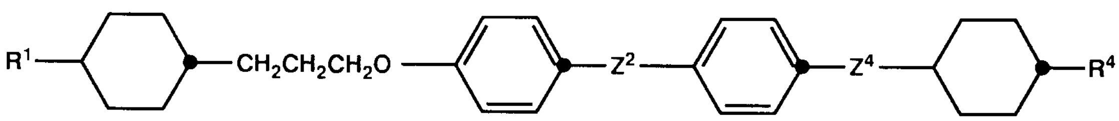

I - 8

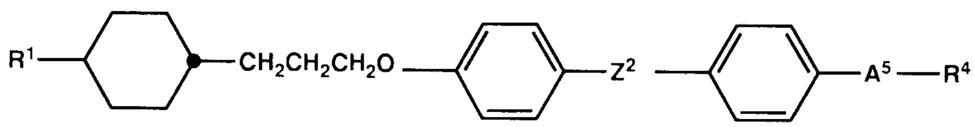

I-9

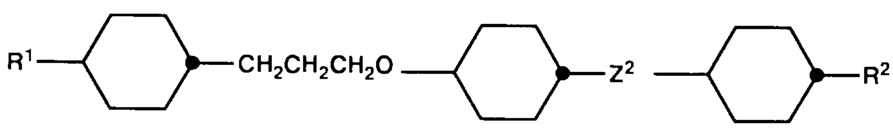

I-10

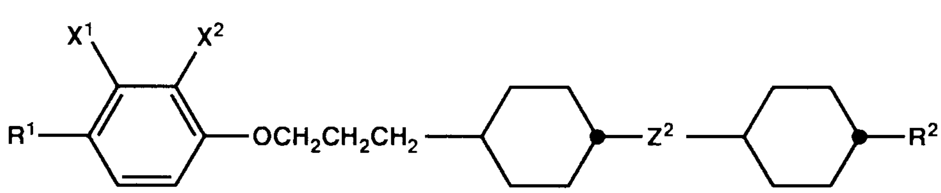

I-11

wherein $A^3$, $A^5$, $R^1$, $R^2$, $R^4$ and $Z^2$ have the significances given in claim 1 and $X^1$, $X^2$, $X^3$ and $X^4$

each independently denote hydrogen, methyl, halogen or cyano.

**7.** Compounds according to claim 6, characterized in that $X^1$, $X^2$, $X^3$ and $X^4$ each independently signify hydrogen and/or fluorine.

**8.** Compounds according to any one of claims 1 to 7, characterized in that $R^1$ signifies a group $R^3$ and $R^2$ signifies a group $R^4$.

**9.** Compounds according to any one of claims 1 to 8, characterized in that $R^3$ and $R^4$ have a maximum of in each case 18 carbon atoms.

**10.** Compounds according to any one of claims 1 to 9, characterized in that $R^3$ and $R^4$ each independently signify an unsubstituted or halogen- and/or cyano-substituted alkyl or alkenyl group, in which optionally 1 $CH_2$ group or 2 non-adjacent $CH_2$ groups is/are replaced by -O-, -COO- and/or -OOC-, and have a maximum of in each case 12 carbon atoms, preferably a maximum of in each case 7 carbon atoms, or one of the residues $R^3$ and $R^4$ also signifies hydrogen, halogen, cyano or -NCS.

**11.** Compounds according to claim 10, characterized in that $R^3$ denotes alkyl, alkenyl, alkoxy, alkenyloxy, alkoxycarbonyl, alkenyloxycabonyl, alkanoyloxy or alkenoyloxy and $R^4$ signifies alkyl, alkenyl, alkoxy, alkenyloxy, alkoxycarbonyl, alkenyloxycarbonyl, alkanoyloxy, alkenoyloxy, halogen, cyano or -NCS.

**12.** Compounds according to any one of claims 1 to 9, characterized in that $R^3$ and $R^4$ each independently signify an unsubstituted or halogen- and/or cyano-substituted $C_1$-$C_{18}$-alkyl or $C_2$-$C_{18}$-alkenyl group, in which optionally 1 $CH_2$ group or 2 non-adjacent $CH_2$ groups is/are replaced by -O-, -COO- and/or -OOC-, and the sum of the carbon atoms in $R^3$ and $R^4$ together is at least 10, preferably at least 12.

**13.** Compounds according to claim 12, characterized in that $R^3$ and $R^4$ each independently signify alkyl, alkenyl, alkoxy, alkenyloxy, alkoxycarbonyl, alkenyloxycarbonyl, alkanoyloxy or alkenoyloxy.

**14.** A liquid crystalline mixture having at least 2 components, characterized in that at least one component is a compound of formula I defined in claim 1.

**15.** A liquid crystalline mixture according to claim 14, characterized in that the content of compounds of formula I is 1-60 wt.%.

**16.** The use of the compounds of formula I defined in claim 1 for electro-optical purposes.

**Revendications**

**1.** Composés de formule générale

$$R^1\text{-}A^1\text{-}Z^1\text{-}(Z^2\text{-}A^3)_n\text{-}R^2$$

dans laquelle $Z^1$ désigne le groupe $-CH_2CH_2CH_2O-$ ou $-OCH_2CH_2CH_2-$; n représente le nombre 0 ou 1; $R^1$ représente un groupe $R^3$ ou $R^3$-$A^4$-$Z^3$; $R^2$ représente un groupe $R^4$ ou $R^4$-$A^5$-$Z^4$-; $Z^2$, $Z^3$ et $Z^4$ représentent, indépendamment les uns des autres, une simple liaison de covalence ou un groupe $-CH_2CH_2-$, $-CH_2O-$, $-OCH_2-$, -COO-, -OOC-, $-CH_2CH_2CH_2O-$ ou $-OCH_2CH_2CH_2-$; $A^1$, $A^2$, $A^3$, $A^4$ et $A^5$ désignent, indépendamment les uns des autres, un radical 1,4-phénylène non substitué ou substitué par un atome d'halogène ou par le groupe méthyle et/ou cyano, et dans lequel éventuellement 1-4 groupes CH sont remplacés par un atome d'azote, un radical trans-1,4-cyclohexylène non substitué ou substitué par le groupe méthyle et/ou cyano, et dans lequel éventuellement 2 groupes $CH_2$ sont remplacés par des atomes d'oxygène et/ou de soufre, un radical bicyclo[2,2,2]octane-1,4-diyle, 1,3,4-thiadiazol-2,5-diyle, naphtalène-2,6-diyle, tétrahydronaphtalène-2,6-diyle ou décahydronaphtalène-2,6-diyle; $R^3$ et $R^4$ représentent, indépendamment l'un de l'autre, un groupe alkyle ou alcényle non substitué ou substitué par un atome d'halogène et/ou par le groupe cyano, ayant respectivement de 1 à 18 ou de 2 à 18 atomes de carbone et dans lequel éventuellement 1 ou 2 groupes $CH_2$ non contigus sont remplacés par -O-, -COO-et/ou -OOC-, ou l'un des radicaux $R^3$ et $R^4$ représente également un atome d'hydrogène ou d'halogène ou le groupe cyano ou -NCS.

**2.** Composés selon la revendication 1, caractérisés en ce que $A^1$, $A^2$, $A^3$, $A^4$ et $A^5$ désignent, indépendamment les uns des autres, le radical 1,4-phénylène ou trans-1,4-cyclohexylène, ou l'un des groupes $A^1$, $A^2$, $A^3$, $A^4$ et $A^5$ désigne également un radical 1,4-phénylène substitué par un atome d'halogène ou par le groupe méthyle et/ou cyano, ou un radical trans-1,4-cyclohexylène substitué par le groupe méthyle et/ou cyano, et/ou l'un des groupes $A^1$, $A^2$, $A^3$, $A^4$ et $A^5$ désigne également un radical 1,4-phénylène dans lequel 1-4 groupes CH sont remplacés par des atomes d'azote, un radical trans-1,4-cyclohexylène dans lequel 2 groupes $CH_2$ sont remplacés par des atomes d'oxygène et/ou de soufre, le radical bicyclo[2.2.2]octane-1,4-diyle, 1,3,4-thiadiazol-2,5-diyle, naphtalène-2,6-diyle, tétrahydronaphtalène-2,6-diyle ou décahydronaphtalène-2,6-diyle.

**3.** Composés selon la revendication 1 ou 2, caractérisés en ce que l'un des groupes $Z^2$, $Z^3$ et $Z^4$ représente une simple liaison de covalence, $-CH_2CH_2-$, $-CH_2O-$, $-OCH_2-$, -COO-, -OOC-, $-CH_2CH_2CH_2O-$ ou $-OCH_2CH_2CH_2-$, et les deux autres groupes $Z^2$, $Z^3$ et $Z^4$ représentent chacun une simple liaison de covalence, -COO- et/ou -OOC-.

**4.** Composés selon l'une des revendications 1 à 3, caractérisés en ce que $Z^1$ représente le groupe $-CH_2CH_2CH_2O-$et $A^1$ représente un cycle saturé ou $Z^1$ représente le groupe $-OCH_2CH_2CH_2-$ et $A^2$ représente un cycle saturé.

**5.** Composés selon l'une des revendications 1 à 4, caractérisés par la formule générale

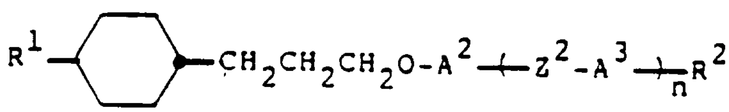

IA

ou

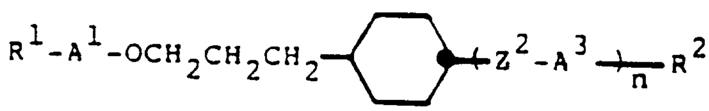

IF

formules dans lesquelles $A^1$, $A^2$, $A^3$, $R^1$, $R^2$, $Z^2$ et n ont les significations données dans la revendication 1.

**6.** Composés selon l'une des revendications 1 à 5, caractérisés par les formules générales

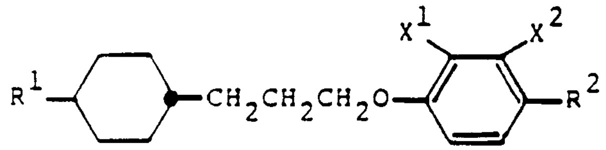

I-1

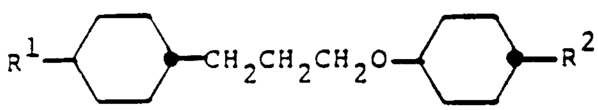

I-2

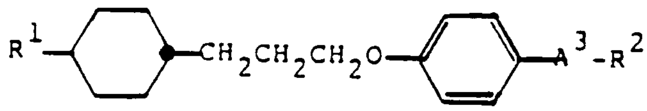

I-3

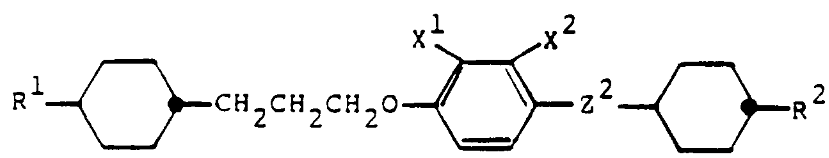

I-4

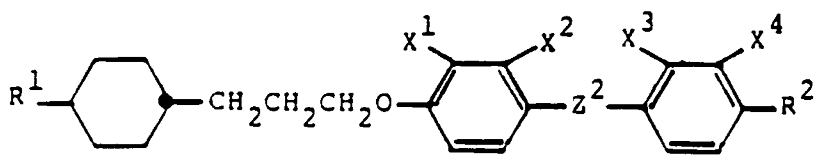

I-5

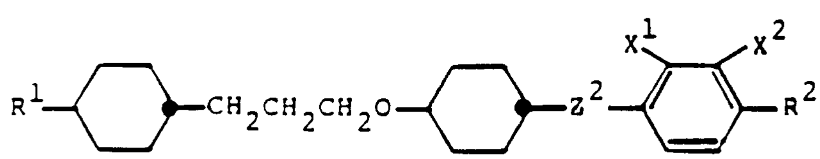

I-6

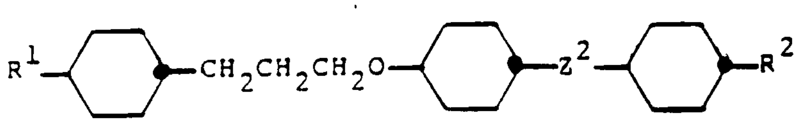

I-7

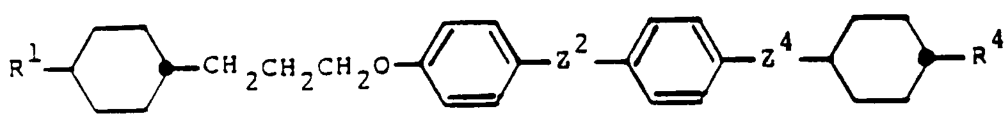

I-8

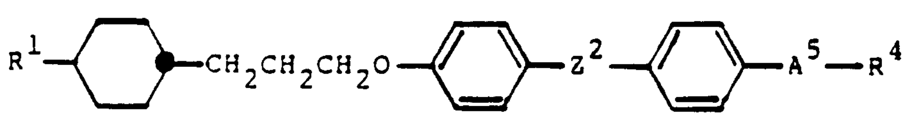

I-9

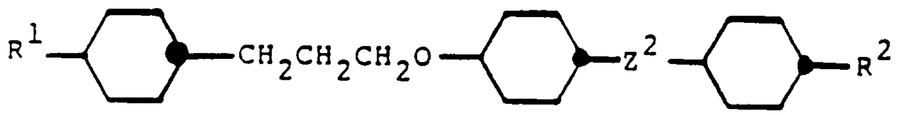

I-10

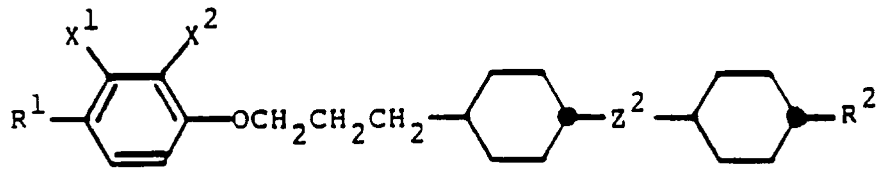

I-11

dans lesquelles $A^3$, $A^5$, $R^1$, $R^2$, $R^4$ et $Z^2$ ont les significations données dans la revendication 1, et $X^1$, $X^2$, $X^3$ et $X^4$ désignent, indépendamment les uns des autres, un atome d'hydrogène ou d'halogène ou le groupe méthyle ou cyano.

**7.** Composés selon la revendications 6, caractérisés en ce que $X^1$, $X^2$, $X^3$ et $X^4$ représentent, indépendamment les uns des autres, un atome d'hydrogène et/ou un atome de fluor.

**8.** Composés selon l'une des revendications 1 à 7, caractérisés en ce que $R^1$ représente un groupe $R^3$ et $R^2$ représente un groupe $R^4$.

**9.** Composés selon l'une des revendications 1 à 8, caractérisés en ce que $R^3$ et $R^4$ comportent chacun 18 atomes de carbone au maximum.

**10.** Composés selon l'une des revendications 1 à 9, caractérisés en ce que $R^3$ et $R^4$ représentent, indépendamment l'un de l'autre, un groupe alkyle ou alcényle non substitué ou substitué par un atome d'halogène et/ou par le groupe cyano, et dans lequel éventuellement 1 ou 2 groupes $CH_2$ non contigus sont remplacés par -O-, -COO- et/ou -COO-, et comportent au maximum chacun 12, de préférence au maximum chacun 7 atomes de carbone, ou l'un des radicaux $R^3$ et $R^4$ représente également un atome d'hydrogène ou d'halogène ou le groupe cyano ou -NCS.

**11.** Composés selon la revendication 10, caractérisés en ce que $R^3$ représente un atome d'halogène ou un groupe alkyle, alcényle, alcoxy, alcényloxy, alcoxycarbonyle, alcényloxycarbonyle, alcanoyloxy, alcénoyloxy, cyano ou -NCS.

**12.** Composés selon l'une des revendications 1 à 9, caractérisés en ce que $R^3$ et $R^4$ représentent, indépendamment l'un de l'autre, un groupe alkyle en $C_1$-$C_{18}$ ou alcényle en $C_2$-$C_{18}$ non substitué ou substitué par un atome d'halogène et/ou par le groupe cyano, et dans lequel éventuellement 1 ou 2 groupes $CH_2$ non contigus sont remplacés par -O-, -COO-et/ou -OOC-, et en ce que la somme des atomes de carbone dans $R^3$ et $R^4$ ensemble s'élève du moins à 10, de préférence au moins à 12.

**13.** Composés selon la revendication 12, caractérisés en ce que $R^3$ et $R^4$ représentent, indépendamment l'un de l'autre, un groupe alkyle, alcényle, alcoxy, alcényloxy, alcoxycarbonyle, alcényloxycarbonyle, alcanoyloxy ou alcénoyloxy.

**14.** Composition de cristaux liquides à au moins 2 composants, caractérisée en ce qu'au moins un composant est un composé de formule I définie dans la revendication 1.

**15.** Composition de cristaux liquides selon la revendication 14, caractérisée en ce que la proportion des composés de formule I va de 1 à 60 % en poids.

**16.** Utilisation des composés de formule I définie dans la revendication 1, à des fins électro-optiques.